# EUROPÄISCHE PATENTSCHRIFT

(11) **EP 3 408 257 B1**
(45) Veröffentlichungstag und Bekanntmachung des Hinweises auf die Patenterteilung: **22.12.2021**
(21) Anmeldenummer: 17703355.2
(22) Anmeldetag: 30.01.2017
(51) Int. Cl.: C07C 243/38, C07C 317/18, C07C 317/28, C07D 207/46, C07K 1/00, C07K 1/107, C07K 1/14, C07K 7/08

(54) **LINKERMOLEKÜL UND VERWENDUNG DESSELBEN IN VERFAHREN ZUR REINIGUNG VON PEPTIDEN**
LINKER MOLECULE AND USE OF IT IN PROCESS FOR PURIFICATION OF PEPTIDES
MOLÉCULE DE LIAISON ET SON UTILISATION DANS DES PROCÉDÉS DE PURIFICATION DE PEPTIDES

(30) Priorität: 29.01.2016 DE 102016101606
(43) Veröffentlichungstag der Anmeldung: 05.12.2018
(73) Patentinhaber: Belyntic GmbH, 12489 Berlin (DE)
(72) Erfinder: ZITTERBART, Robert, 10405 Berlin (DE); SEITZ, Oliver, 13467 Berlin (DE)
(74) Vertreter: Schulz Junghans Patentanwälte PartGmbB
(86) Internationale Anmeldenummer: PCT/EP2017/051932
(87) Internationale Veröffentlichungsnummer: WO 2017/129818

(56) Entgegenhaltungen:
- EP-A1- 0 552 368
- EP-B1- 2 501 711
- US-A1- 2012 270 937

### SEQUENCE LISTING

<110> Humboldt-Universitaet zu Berlin
<120> Linkermolekuel und Verwendung desselben in Verfahren zur Reinigung von Peptiden
<130> U30701WO
<140> 10 2016 101 606.3
   <141> 2016-01-29
<160> 10
<170> PatentIn version 3.5
<210> 1
   <211> 8
   <212> PRT
   <213> Artificial Sequence
<220>
   <223> Peptid zur Demonstration der Reversibilität der Hydrazonbindung mit einem Reinigungsharz
<400> 1
   Gly Leu Tyr Ala Glu Gly Gly Gly
   1               5
<210> 2
   <211> 6
   <212> PRT
   <213> Artificial Sequence
<220>
   <223> Peptid für eine native chemische Ligation
<400> 2
   Tyr Glu Asn Arg Ile Lys
   1               5
<210> 3
   <211> 12
   <212> PRT
   <213> Homo sapiens
<400> 3
   Thr Leu Ala Asp Glu Val Ser Ala Ser Leu Ala Lys
   1               5                   10
<210> 4
   <211> 13
   <212> PRT
   <213> Homo sapiens
<400> 4
   Ala Thr Leu Ala Asp Glu Val Ser Ala Ser Leu Ala Lys
   1               5                   10
<210> 5
   <211> 32
   <212> PRT
   <213> Homo sapiens
<400> 5
   Cys Gln Trp Ser Leu His Arg Lys Arg His Leu Ala Arg Thr Leu Leu
   1               5                   10                  15
   Thr Ala Ala Arg Glu Pro Arg Pro Ala Pro Pro Ser Ser Asn Lys Val
               20                  25                  30
<210> 6
   <211> 23
   <212> PRT
   <213> Homo sapiens
<400> 6
   Gly Ile Gly Lys Phe Leu His Ser Ala Lys Lys Phe Gly Lys Ala Phe
   1               5                   10                  15
   Val Gly Glu Ile Met Asn Ser
               20
<210> 7
   <211> 9
   <212> PRT
   <213> Homo sapiens
<400> 7
   Tyr Leu Phe Phe Tyr Arg Lys Ser Val
   1               5
<210> 8
   <211> 20
   <212> PRT
   <213> Homo sapiens
<400> 8
   Phe Pro Arg Pro Gly Gly Gly Gly Asn Gly Asp Phe Glu Glu Ile Pro
   1               5                   10                  15
   Glu Glu Tyr Leu
               20
<210> 9
   <211> 12
   <212> PRT
   <213> Homo sapiens
<400> 9
   Gly Arg Lys Lys Arg Arg Gln Arg Arg Arg Pro Gln
   1 5 10
<210> 10
   <211> 13
   <212> PRT
   <213> Artificial Sequence
<220>
   <223> Peptid zur Demonstration der Regeneration des Alehyd-Harzes und Reinigungsexperimente
<400> 10
   Ala Lys Ala Asp Glu Val Ser Leu His Lys Trp Tyr Gly
   1 5 10

## Beschreibung

### Gebiet der Erfindung

Die vorliegende Erfindung betrifft ein Verfahren zur Reinigung von Peptiden, die durch Festphasenpeptidsynthese (SPPS) hergestellt werden und entsprechende Linkermoleküle zur Verwendung in diesem Verfahren.

### Hintergrund und Stand der Technik

Die Festphasenpeptidsynthese ist eine bekannte Methode zur Herstellung von Peptiden. Neben der Synthese der Peptide ist auch deren Reinigung ein wesentlicher Verfahrensschritt.

Eine weit verbreitete Methode zur Reinigung von Peptiden ist die präparative High Performance Liquid Chromatography (HPLC). Nachteilig an dieser Methode ist die schlechte Skalierbarkeit hinsichtlich der gewünschten Produktionsmengen, so dass unterschiedliche Mengen nicht mit ein und derselben Anlage produziert werden können; dieses verursacht relativ hohe Anschaffungskosten der entsprechenden komplexen Geräte. Ein weiterer Nachteil liegt darin, dass die korrekte analytische Bewertung der einzelnen Fraktionen relativ umfangreiche Kenntnisse notwendig macht; hinzu kommt der Verbrauch an Lösungsmitteln und Säulenmaterial (feste Phase) während des Betriebs.

Zur Senkung der Kosten bei der Peptidherstellung wären daher kostengünstigere und weniger störanfällige Methoden von Vorteil.

Die EP 0 552 368 A1 beschreibt ein Verfahren zur Reinigung von Peptiden, bei dem ein sogenannter Linker zum einen an das N-terminale Ende des synthetisierten Volllängenpeptids und zum anderen durch Reaktion einer Thiolgruppe mit funktionalisiertem Kieselgur zu Thioether kovalent gebunden wird. Das Volllängenpeptid wird somit immobilisiert und kann gereinigt werden. Anschließend erfolgt die Freisetzung des Volllängenpeptids unter basischen Bedingungen. Das Verfahren ist jedoch nicht für Thiol-enthaltende Peptide geeignet, wie z.B. solche, die die Aminosäure Cystein oder Penicillamin enthalten. Des Weiteren besteht der Nachteil, dass die zur Reinigung verwendete feste Phase (in diesem Fall Kieselgur) nicht zur Wiederverwendung vorgesehen oder geeignet ist.

EP 2 501 711 B1 schlägt ein analoges Verfahren vor, bei dem der Linker zum einen über das N-terminale Ende des synthetisierten Volllängenpeptids und über eine 1,3-dipolare Cycloaddition zwischen einem Azid (-N₃) und einem Alkin (Huisgen-Reaktion) an eine feste Phase (synthetisches, hydrophiles Polymer, z.B. PEGA) gebunden wird. Nachteilig an diesem Verfahren ist hier die Notwendigkeit der Zugabe von Kupfer oder Kupfer-haltigen Verbindungen zur Durchführung der 1,3-dipolaren Cycloaddition. Viele Peptide komplexieren Kupfer, insbesondere solche, die Schwefel aufweisen, also Methionin und /oder Cystein enthalten; ebenfalls können Arginine und Lysine Kupfer binden. Das Kupfer lässt sich daher nur schwer wieder entfernen, und aufgrund der Toxizität des verbleibenden Kupfers ist die Methode nicht in allen Fällen anwendbar, insbesondere nicht für die Reinigung von Peptidtherapeutika. Zudem besteht auch hier der Nachteil, dass die zur Reinigung verwendete feste Phase nicht zur Wiederverwendung vorgesehen oder geeignet ist.

Die Druckschrift US 2012/270937 A1 beschreibt Linkerverbindungen, die für die Synthese von sich selbst-eliminierenden Oligomeren zur Wirkstoffabgabe verwendet werden. Die offenbarten Linkerverbindungen eignen sich nicht zur Spaltung unter basischen Bedingungen.

Es ist daher Aufgabe der Erfindung, ein Verfahren zur Reinigung von Peptiden bereitzustellen, das keine komplette HPLC-Anlage erfordert und auch für schwefelhaltige oder Kupfer-bindende Peptide geeignet ist. Darüber hinaus ist es Aufgabe der Erfindung, ein zur Reinigung von Peptiden geeignetes Verfahren zur Verfügung zu stellen, das eine Regeneration und Wiederverwendung der zur Reinigung eingesetzten festen Phase ermöglicht. Des Weiteren ist es Aufgabe der Erfindung, eine Verbindung bereitzustellen, die eine Verbindung zwischen der N-terminalen Aminogruppe eines Volllängenpeptids und einer festen Phase ermöglicht.

In einem ersten Aspekt der vorliegenden Erfindung wird diese Aufgabe durch eine Verbindung gelöst, die die allgemeine Formel

X₁-L-X₂ (1)

aufweist, wobei
X₁ ausgewählt ist aus 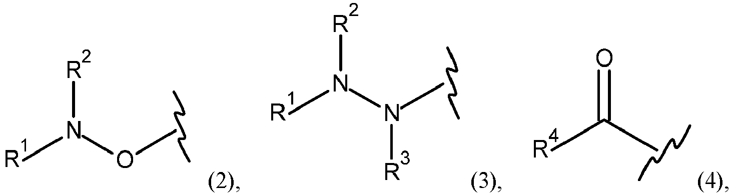
   - wobei jedes R¹ und R² unabhängig voneinander ausgewählt ist aus H oder B, wobei wenigstens R¹ oder R² B ist, wobei
   - R³ aus H oder B ausgewählt ist,
      - wobei B eine säurelabile Amin-Schutzgruppe ist, wobei
   - R⁴ ausgewählt ist aus H, C₁-C₁₂-Alkyl oder Aryl, wobei die Aldehyd- oder Ketogruppe durch eine säurelabile Schutzgruppe geschützt vorliegen kann,
L ausgewählt ist aus funktionellen Linkerverbindungen, die unter basischen Bedingungen von X₂ nukleophil abspaltbar sind, insbesondere weist L die Form -T-U- auf, wobei
   - T ein Abstandshalter zwischen X₁ und U ist, wobei T insbesondere ausgewählt ist aus substituierte oder unsubstituierte -C₁-C₁₂-Alkyl-, insbesondere C₁-C₆-Alkyl, insbesondere C₁-C₃-Alkyl, -R⁵-C(=O)-NH-R⁶-, -R⁵-C(=O)-O-R⁶-, -C(=O)-O-R⁶-, -C(=O)-NH-R⁶-, -C(=O), -R⁵-phenyl-R⁶-, -R⁵-phenyl-, -phenyl-R⁶-, -phenyl-,
      - wobei R⁵ und R⁶ unabhängig voneinander ausgewählte substituierte oder unsubstituierte C₁-C₁₂-Alkyle, insbesondere C₁-C₆ Alkyle, besonders C₁-C₃ Alkyle sind, sind, und wobei
   - U der die Spaltung aktivierende Teil der funktionellen Linkerverbindung ist, wobei der aktivierende Teil ausgebildet ist ein bei einer basischen Abspaltung von X₂ entstehendes Anion zu stabilisieren,
X₂ die Form -Y-Z aufweist, wobei
   - Y aus -O-C(=O)- oder -S(=O)₂- ausgewählt ist, und
   - Z eine elektronenziehende Fluchtgruppe ist.

Im Kontext der vorliegenden Erfindung bezieht sich der Begriff "Spaltung aktivierender Teil" eines Moleküls auf ein strukturelles Element einer reaktiven Funktion.

"Reaktive Funktion" bezieht sich auf eine Verbindung, die zur Generierung einer reaktiven Spezies, angeregt (aktiviert) werden kann. Dies kann zum Beispiel einen Katalysator oder eine pH-Wert-Änderung geschehen. Die reaktive Spezies ist in der Lage, in kurzer Zeit mit einem geeigneten Reaktionspartner eine kovalente Bindung zu bilden, zum Beispiel eine Carbamat-Bindung. Die reaktive Funktion umfasst also Gruppen, die nach ihrer Aktivierung spezifisch mit anderen funktionellen Gruppen, zum Beispiel Amin- oder Amid-, reagieren.

Der Begriff "Abstandshalter" bezieht sich auf eine Verbindung von mehreren Atomen innerhalb eines Moleküls, die selbst frei von reaktiven Funktionen ist und zwei funktionelle Gruppen des Moleküls räumlich trennt. Der Abstandshalter ist eine kovalent verbundene Ketten- oder Ringstruktur bestehend aus Kohlenstoff-, Phosphor-, Schwefel-, Silizium-, Stickstoff- und/oder Sauerstoffatomen. Der Abstandshalter kann substituierende Gruppen enthalten, die nicht zur Distanz zwischen den zu trennenden funktionellen Gruppen beiträgt.

Der Begriff "Gruppierung" bezieht sich auf eine Verbindung von mehreren Atomen innerhalb eines Moleküls. Typischerweise bilden diese Atome funktionelle Einheiten, wie zum Beispiel einen Abstandshalter, eine reaktive Funktion oder eine molekulare Struktur, die einen mesomeren oder induktiven Effekt ausübt.

Die Begriffe "funktionelle Linkerverbindung" oder "Linkerverbindung" bezieht sich auf eine funktionelle Gruppe, das zwei funktionelle Einheiten innerhalb eines Moleküls verbindet. Die Linkerverbindung ist kovalent mit den funktionellen Gruppen verbunden.

Die Begriffe "Linker", "Linkermolekül", "Linkersystem" und "Fängerverbindung" beziehen sich auf ein Molekül, das zwei andere Moleküle durch Ausbildung einer kovalenten Bindung zu dem jeweils anderen Molekül verbindet. Die kovalenten Bindungen zu den funktionellen Gruppen der zwei anderen Moleküle geschehen nur unter bestimmten Reaktionsbedingungen. Insbesondere beziehen sich die Begriffe "Linker", "Linkermolekül", "Linkersystem" und "Fängerverbindung" auf Verbindungen, die unter Formel (1) fallen und eine Verbindung zwischen einem N-Terminus eines Peptids und einem festen Träger herstellen können.

Der Begriff "substituiert" bezieht sich auf die Addition eines Atoms oder einer molekularen Gruppe oder Verbindung an eine Stammverbindung. Die substituierende Gruppe oder Verbindung kann geschützt oder ungeschützt addiert werden an eine oder mehrere verfügbare Stellen im Stammmolekül. Die substituierende Gruppe oder Verbindung selbst kann substituiert oder nicht substituiert sein und direkt oder über eine verbindende Gruppe oder Verbindung wie zum Beispiel eine Alkyl-, Amid- oder Hydrocarbonylgruppe mit dem Stammmolekül verbunden sein. Substituierende Gruppen oder Verbindungen umfassen zum Beispiel Halogene, Sauerstoff, Stickstoff, Schwefel, Hydroxyl-, Alkyl-, Alkenyl-, Alkynyl- Carboxyl- (-C(O)OR^{a}), Acyl-(-C(O)R^{a})-Gruppen, aliphatische, alicyclische Gruppen, Alkoxy-, Amino ((-N(R^{b})(R^{c})), Imino-(=NR^{b}), Amido-(-C(O)N(R^{b})(R^{c}) oder -N(R^{b})C(O)R^{a}) Gruppen, Hydrazinderivate (-C(NH)NR^{a}R^{b}), Triazole, Tetrazole (CN₄H₂), Azido- (-N₃), Nitro (-NO₂), Cyano (-CN), Isocyano (-NC), Cyanato (-OCN), Isocyanato- (-NCO), Thiocyanato- (-SCN); Isothio-cyanato- (-NCS); Carbamido- (-OC(O)N(R^{b})(R^{c}) oder -(R^{b})C(O)OR^{a}) Gruppen, Thiole (-SR^{b}), Sulfinyl- (-S(O)R^{b}), Sulfonyl- (-S(O)₂R^{b}), Sulfonamidyl-(-S(O)₂N(R^{b})(R^{c}) oder -N(R^{b})S(O)₂R^{b}) Gruppen und fluorinierte Verbindungen wie zum Beispiel-CF₃, -OCF₃, -SCF₃, -SOCF₃ or -SO₂CF₃. R^{a}, R^{b} and R^{c} ist unabhängig voneinander H oder eine weitere substituierende Gruppe.

Der Begriff "Alkyl" bezieht sich auf eine gesättigte gerade oder verzweigte Kohlenwasserstoffkette mit bis zu 12 Kohlenstoffatomen. Beispiele für bevorzugte Alkylgruppen sind Methyl-, Ethyl-, Propyl-, Butyl-, Isopropyl-, n-Hexyl-, Oktyl-, Decyl-Gruppen.

Der Begriff "Aryl" bezieht sich auf eine Kohlenwasserstoffverbindung mit alternierenden Doppel- und Einfachbindungen zwischen den Kohlenstoffatomen, wobei eine Ringstruktur gebildet wird.

Der Begriff "Fluchtgruppe" bezieht sich auf eine funktionelle Gruppe innerhalb eines Moleküls, die einen -M und/oder -I-Effekt ausübt und sich dadurch leicht abspalten lässt, wobei das bindende Elektronenpaar nach der Spaltung bei der Fluchtgruppe verbleibt.

Der Begriff "oberflächenmodifizierter fester Träger" bezieht sich auf eine feste Struktur, wie zum Beispiel Sepharose-, Agarose- oder Zelluloseeinheiten, Kieselgel oder Polydextrane, die durch synthetische oder natürliche Polymere wie zum Beispiel Polysaccharide, Polylysin, Polyarylamid, Polyethylenglykol (PEG) oder Acrylamid-PEG-Kopolymere modifiziert sind. Die Oberfläche des festen Trägers ist durch Aldehyd-, Keton-, Hydroxylamin- oder Hydrazingruppen charakterisiert.

In einigen Ausführungsformen ist Z eine elektronenziehende Fluchtgruppe, die einen -M und/oder -I-Effekt ausübt, und bei einem heterolytischen Bindungsbruch das bindende Elektronenpaar mitnimmt.

In einigen Ausführungsformen ist Z eine elektronenziehende Fluchtgruppe, wobei die zum Anion der Fluchtgruppe korrespondierende Säure durch einen pks-Wert von kleiner als fünf charakterisiert ist.

In einigen Ausführungsformen ist Z eine elektronenziehende Fluchtgruppe, wobei die zum Anion der Fluchtgruppe korrespondierende Säure durch einen pks-Wert von kleiner als fünf charakterisiert ist, und wobei die Fluchtgruppe insbesondere einen -M und/oder -I-Effekt ausübt, und bei einem heterolytischen Bindungsbruch das bindende Elektronenpaar mitnimmt.

In einigen Ausführungsformen ist U der die Spaltung aktivierende Teil der funktionellen Linkerverbindung, wobei der aktivierende Teil eine Gruppierung darstellt, die durch -M und -I Effekte eine Anionenbildung ermöglicht, sowie die entstandene anionische Verbindung durch eine Elektronenpaarverschiebung stabilisiert, wobei diese Stabilisierung zu einem heterolytischen Bindungsbruch zwischen U und X₂ führt.

In einigen Ausführungsformen ist B ausgewählt aus Boc (-C=OOtBu), Trityl (-C(Ph)₃), Mmt (-C(Ph)₂C₆H₄OMe), DMT (-C(Ph)(C₆H₄OMe)₂), Cbz (-C=OOCH₂Ph), Benzylideneamin (=CPh), Phtalimide (=(CO)₂C₆H₄), p-Toluenesulfonamide (-SO₂C₆H₄Me), Benzylamin (-CH₂Ph), Acetamide (-COMe), Trifluoracetamid (-COCF₃), Dde (1-(4,4-Dimethyl-2,6-dioxocyclohex-1-ylidene)-3-ethyl) und 1-(4,4-Dimethyl-2,6-dioxocyclohex-1-ylidene)-3-methylbutyl (ivDde), wobei B insbesondere Boc ist.

In einigen Ausführungsformen sind die Acetal- oder Ketalschutzgruppen ausgewählt aus 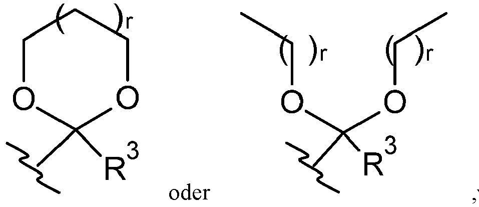 wobei r 0 bis 12, insbesondere 0, 1 oder 2, ist.

Dem Fachmann ist bekannt, dass die erfindungsgemäße Verbindung auch mit anderen säurelabilen Schutzgruppen gebildet werden kann.

In einigen Ausführungsformen ist T ausgewählt aus substituiertem oder unsubstituiertem C₁-C₁₂-Alkyl-,-, insbesondere C₁-C₆-Alkyl, insbesondere C₁-C₃-Alkyl, -R⁵-C(=O)-NH-R⁶-, -R⁵-C(=O)-O-R⁶-, -R⁵-C(=O)-O-, -C(=O)-O-R⁶-, -C(=O)-NH-R⁶-, -C(=O)-, -C(=O)-O-, wobei R⁵ und R⁶ unabhängig voneinander ausgewählte substituierte oder unsubstituierte C₁-C₁₂-Alkyle, insbesondere C₁-C₆-Alkyle, insbesondere C₁-C₃-Alkyle sind.

Wenn T ein substituiertes Alkyl ist, sind die Substituenten insbesondere solche, die die Wasserlöslichkeit erhöhen, zum Beispiel -SO₃H, -CO₂H oder -NO₂.

In einigen Ausführungsformen ist T ausgewählt aus -CH₂-, -CH₂-C(=O)-NH-(CH₂)₂-, -(CH₂)-C(=O)-O-(CH₂)₂-, -CH₂-C(=O)-O-, -C(=O)-O-, -C(=O)-O-(CH₂)₂-, und -C(=O)-.

In einigen Ausführungsformen ist T ausgewählt aus -CH₂-, -CH₂-C(=O)-NH-(CH₂)₂-, -C(=O)-O-(CH₂)₂-, -CH₂-C(=O)-O-, und -C(=O)-.

In einigen Ausführungsformen ist U der Verbindung T-U-Y ausgewählt aus den Verbindungen nach den Formeln (5), (6), (7), (8), (9), (10) und (11), insbesondere aus (5), (6), (8), (9) und (10), 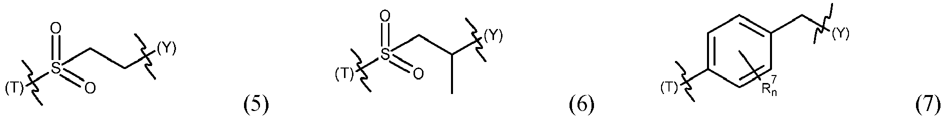 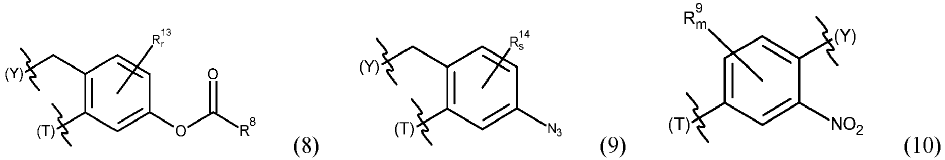 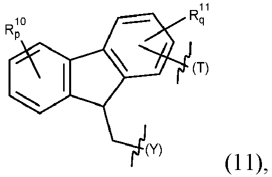 wobei R⁸ ausgewählt ist aus C₁-C₆-Alkyl, CF₃, CH₂CF₃, 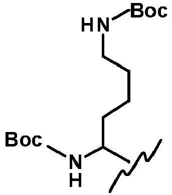 (Boc-Lys(Boc)-) und 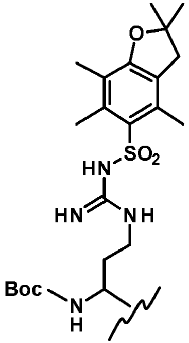 (Boc-Arg(pbf)), insbesondere aus Boc-Lys(Boc)-,
wobei R⁷ₙ, R⁹ₘ, R¹⁰ₚ, R¹¹_{q}, R¹³ᵣ und R¹⁴ₛ ausgewählt ist aus C₁-C₆-Alkyl oder -I und/oder -M-Effekt erzeugenden Substituenten, insbesondere C₁-C₃-Alkyle, -F, -Cl, -Br, -I, -CN -NO₂, -N₃, -CF₃, -SO₃H,-und CO₂H,
- wobei n gleich 0, 1, 2, 3 oder 4, insbesondere n ist 0 oder 1, insbesondere 0, ist
- wobei m gleich 0, 1, 2 oder 3, insbesondere m ist 0 oder 1, insbesondere 0, ist
- wobei p gleich 0, 1, 2, 3 oder 4, insbesondere p ist 0 oder 1, insbesondere 0, ist
- wobei q gleich 0, 1, 2 oder 3, insbesondere q ist 0 oder 1, insbesondere 0, ist
- wobei r gleich 0, 1, 2 oder 3, insbesondere r ist 0 oder 1, insbesondere 0, ist
- wobei s gleich 0, 1, 2 oder 3, insbesondere s ist 0 oder 1, insbesondere 0, ist.

In einigen Ausführungsformen ist R⁷ₙ, R⁹ₘ, R¹⁰ₚ, R¹¹_{q}, R¹³ᵣ und R¹⁴ₛ ausgewählt aus C₁-C₆-Alkyl oder-I und/oder -M-Effekte erzeugenden Substituenten, insbesondere C₁-C₃-Alkyle, -F, -Cl, -Br, -I, -CN-NO₂, -N₃, -CF₃, -SO₃H, und -CO₂H, insbesondere -F, -Cl, -Br, -I, -NO₂, und -N₃
- wobei n gleich 0, 1, 2, 3 oder 4, insbesondere n ist 0 oder 1, insbesondere 0, ist
- wobei m gleich 0, 1, 2 oder 3, insbesondere m ist 0 oder 1, insbesondere 0, ist
- wobei p gleich 0, 1, 2, 3 oder 4, insbesondere p ist 0 oder 1, insbesondere 0, ist
- wobei q gleich 0, 1, 2 oder 3, insbesondere q ist 0 oder 1, insbesondere 0, ist
- wobei r gleich 0, 1, 2 oder 3, insbesondere r ist 0 oder 1, insbesondere 0, ist
- wobei s gleich 0, 1, 2 oder 3, insbesondere s ist 0 oder 1, insbesondere 0, ist.

In einigen Ausführungsformen ist R⁷ₙ, R⁹ₘ, R¹⁰ₚ, R¹¹_{q}, R¹³ᵣ und R¹⁴ₛ ausgewählt aus die Wasserlöslichkeit erhöhende Substituenten, insbesondere aus -NO₂, -SO₃H und -CO₂H.

In einigen Ausführungsformen ist Z ausgewählt aus der Gruppe -F, -Cl, -Br, -I, -N₃, -SR¹², -OCF₃,-OCH₂CF₃, -OSO₂CF₃, -SO₂C₆H₄CH₃, -SO₂CF₃, -SO₂CH₃ 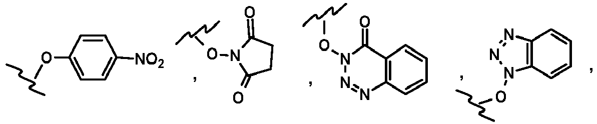 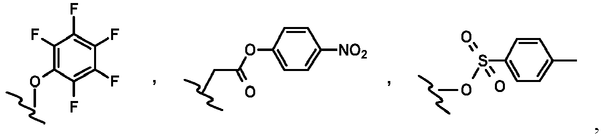 wobei R¹² ein C₁-C₆-Alkyl-, ein Aryl- oder ein Benzylrest ist.

In einigen Ausführungsformen ist Z ausgewählt aus -Cl, 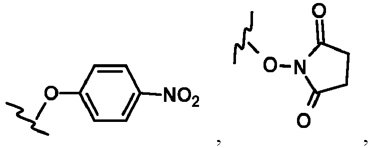 insbesondere aus

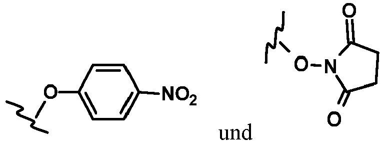

In einigen Ausführungsformen ist X₁ eine Verbindung nach Formel (2) oder (3), wobei R³ H ist, R¹ und R² eine Boc-Schutzgruppe aufweisen oder R¹ H ist und R² eine Boc-Schutzgruppe ist.

In einigen Ausführungsformen ist X₁ eine Verbindung nach Formel (3), wobei R¹ und R³ H ist und R² eine Boc-Schutzgruppe ist.

In einigen Ausführungsformen weist Y die Form -O-C(=O)- auf.

In einigen Ausführungsformen weist T die Form -CH₂-C(=O)-NH-(CH₂)₂-, -CH₂-C(=O)-O-(CH₂)₂-,-C(=O)-O-(CH₂)₂- auf und U ist eine Verbindung nach Formel (5) oder (6),

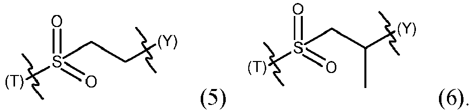

In einigen Ausführungsformen weist T die Form -C(=O)-O-(CH₂)₂- oder -CH₂-C(=O)-NH-(CH₂)₂- auf und U ist eine Verbindung nach Formel (6).

In einigen Ausführungsformen weist T die Form -C(=O)-O-(CH₂)₂- oder -CH₂-C(=O)-NH-(CH₂)₂- auf und U ist eine Verbindung nach Formel (6).

In einigen Ausführungsformen weist T die Form -CH₂-C(=O)-NH-(CH₂)₂- auf und U ist eine Verbindung nach Formel (6).

In einigen Ausführungsformen weist T die Form -CH₂-C(=O)-O- oder -C(=O)-O- auf und U ist eine Verbindung nach Formel (7), 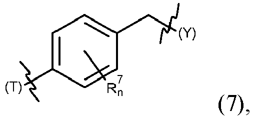 wobei R⁷ ausgewählt ist aus C₁-C₆-Alkyl oder -I und/oder -M-Effekte erzeugenden Substituenten, insbesondere C₁-C₃-Alkyl, -F, -Cl, -Br, -I, -CN -NO₂, -N₃, -CF₃, -SO₃H, und -CO₂H, wobei n gleich 0, 1, 2, 3 oder 4, insbesondere 0 oder 1, insbesondere 0, ist.

In einigen Ausführungsformen weist T die Form -CH₂-C(=O)-O- auf, und U ist eine Verbindung nach Formel (7), wobei n gleich 0 ist.

In einigen Ausführungsformen weist T die Form -CH₂- auf, und U ist eine Verbindung nach Formel (8), 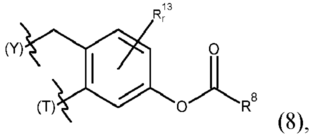 wobei R⁸ Boc-Lys(Boc)- und und r gleich 0 ist.

In einigen Ausführungsformen weist T die Form -CH₂- oder -(C=O)- auf, und U ist eine Verbindung nach Formel (9), 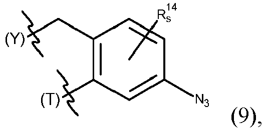 wobei s gleich 0 ist.

In einigen Ausführungsformen weist T die Form -CH₂- auf, und U ist eine Verbindung nach Formel (9).

In einigen Ausführungsformen weist T die Form -C(=O)- auf, und U ist eine Verbindung nach Formel (10), 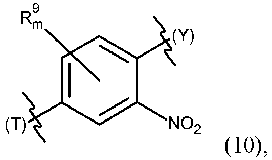 wobei m gleich 0 ist, Y die Form -SO₂- aufweist und Z Cl ist.

In einigen Ausführungsformen ist die Verbindung nach Formel (1) ausgewählt aus 2,2-Dimethylpropanoyloxy-[2-[2-[2-(4-nitrophenoxy)carbonyloxypropylsulfonyl]ethylamino]-2-oxo-ethoxy]amino] 2,2-dimethylpropanoat (Formel (14)), [[2-[2-[2-(4-Nitrophenoxy) carbonyloxypropylsulfonyl]ethylamino]-2-oxoethoxy]amino] 2,2-dimethylpropanoat (Formel (15)), [2-(4-Chlorsulfonyl-3-nitrobenzoyl)hydrazino] 2,2-dimethylpropanoat (Formel (16)), [2,2-Dimethylpropanoyloxy-[2-[4-[(2,5-dioxopyrrolidin-1-yl)oxycarbonyloxymethyl]phenoxy]-2-oxo-ethoxy]amino] 2,2-dimethylpropanoat (Formel (17)), [2-(2,2-Dimethylpropanoyloxy)-2-[2-[2-[2-(2,5-dioxopyrrolidin-1-yl)oxycarbonyloxypropylsulfonyl]ethylamino]-2-oxo-ethyl]hydrazino] 2,2-dimethylpropanoat (Formel (18)), [2-(2,2-Dimethylpropanoyloxy)-2-[2-[2-[2-(2,5-dioxopyrrolidin-1-yl)oxycarbonyloxyethylsulfonyl]ethylamino]-2-oxo-ethyl]hydrazino] 2,2-dimethylpropanoat (Formel (19)), [2-[5-Azido-2-[(2,5-dioxopyrrolidin-1-yl)oxycarbonyloxymethyl]benzoyl]hydrazino] 2,2-dimethylpropanoat (Formel (20)), [3-[(2,2-Dimethylpropanoyloxyamino)carbamoyl]-4-[(2,5-dioxopyrrolidin-1-yl)oxycarbonyloxymethyl]phenyl] 2,6-bis(2,2-dimethylpropanoyloxyamino) hexanoat (Formel (21)), [2-[2-[2-[2-(4-Nitrophenoxy)carbonyloxypropylsulfonyl]ethylamino]-2-oxo-ethyl]hydrazino] 2,2-dimethylpropanoat (Formel (22)), [2-[2-[2-[2-(2,5-Dioxopyrrolidin-1-yl) oxycarbonyloxy-propylsulfonyl]ethylamino]-2-oxo-ethyl]hydrazino] 2,2-dimethylpropanoat (Formel (23)), [2-[2-[4-[(2,5-Dioxopyrrolidin-1-yl)oxycarbonyloxymethyl]phenoxy]-2-oxo-ethyl]hydrazino] 2,2-dimethyl-propanoat (Formel (24)), [2-[2-[2-(4-Nitrophenoxy)carbonyloxyethylsulfonyl] ethoxycarbonyl] hydrazino] 2,2-dimethylpropanoat (Formel (25)), [3-[[2-(2,2-Dimethylpropanoyloxy)hydrazino]methyl]-4-[(2,5-dioxopyrrolidin-1-yl)oxycarbonyloxymethyl] phenyl] 2,6-bis(2,2-dimethylpropanoyloxyamino)hexanoat (Formel (26)), [2-[[5-Azido-2-[(2,5-dioxopyrrolidin-1-yl)oxycarbonyloxymethyl]phenyl]methyl]hydrazino] 2,2-dimethylpropanoat (Formel (27)), 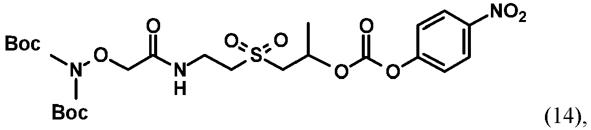 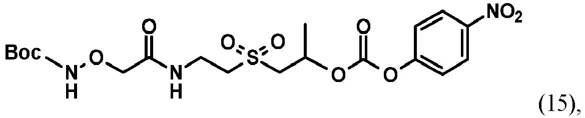 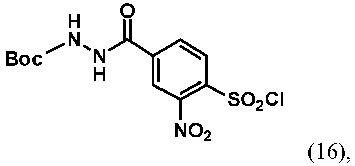 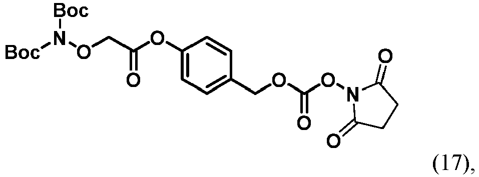 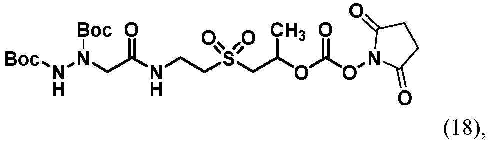 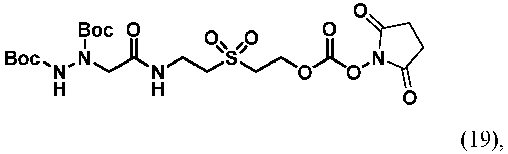 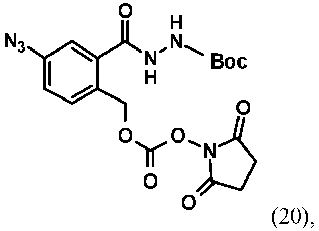 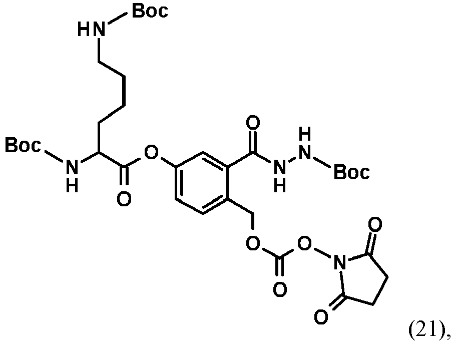 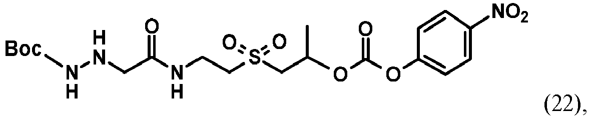 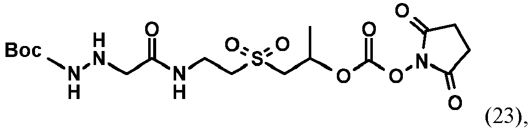 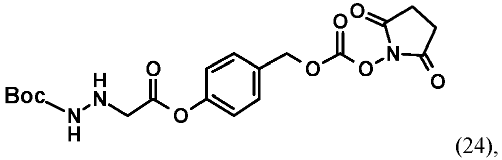 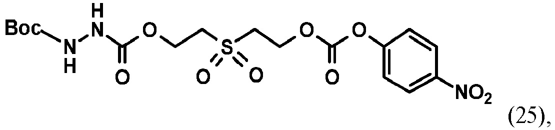 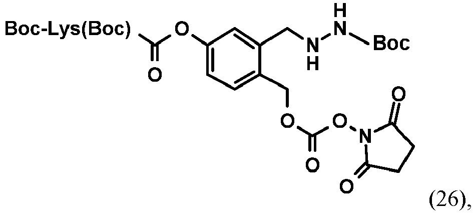 und

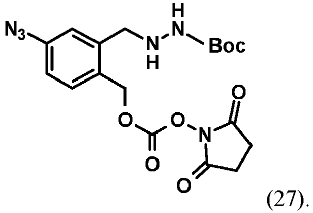

In einigen Ausführungsformen ist die Verbindung nach Formel (1) ausgewählt aus einer Verbindung nach Formel (14), (15), (16), (17), (18), (19), (20) und (21).

In einigen Ausführungsformen ist die Verbindung nach Formel (1) ausgewählt aus einer Verbindung nach Formel (16), (19), (20) und (21).

In einem weiteren Aspekt wird die Aufgabe der Erfindung durch die Verwendung einer Verbindung nach dem ersten Aspekt gelöst, die eine Verbindung zwischen der N-terminalen Aminogruppe eines Volllängenpeptids und einer festen Phase bildet.

In einem weiteren Aspekt wird die Aufgabe der Erfindung durch eine Verbindung der Formel (12), X₁-L-Y-PEP (12), gelöst, wobei X₁, L und Y durch den ersten Aspekt und seine Ausführungsformen definiert sind, und wobei PEP ein Volllängenpeptid umfasst, das über seinen N-Terminus mit X₂' verbunden ist.

In einem weiteren Aspekt wird die Aufgabe der Erfindung durch eine Verbindung der Formel (13), D-X₁'-L-Y-PEP (13), gelöst, wobei D ein oberflächenmodifizierter fester Träger ist, der dadurch gekennzeichnet ist, dass die Oberfläche durch synthetische oder natürliche Polymere modifiziert ist, wobei X₁' die Form -NH-O-, -NH-NH- oder -C(=O)- aufweist und wobei L, Y und PEP durch den ersten Aspekt und seine Ausführungsformen definiert sind.

In einigen Ausführungsformen ist der oberflächenmodifizierte feste Träger D durch modifizierte Polysaccharide charakterisiert.

In einigen Ausführungsformen ist der oberflächenmodifizierte feste Träger D durch Aldehyd- oder Hydrazin-modifizierte Sepharose/Agarose oder Zellulose charakterisiert.

In einem weiteren Aspekt wird die Aufgabe der Erfindung durch ein Verfahren zur Reinigung von Peptiden gelöst, insbesondere von mittels Festphasenpeptidsynthese (SPPS) hergestellten Peptiden, das die folgenden Schritte umfasst:
i. In-Kontakt-Bringen einer Zusammensetzung eines zu reinigenden Volllängenpeptids und mindestens einer Verunreinigung, insbesondere mindestens einer acetylierten Abbruchsequenz, mit einer Fängerverbindung, die durch den ersten Aspekt und seine Ausführungsformen definiert ist, und anschließender Reaktion zu einer Verbindung der Formel (12),
ii. Abspaltung der säurelabilen Schutzgruppen durch Zugabe einer Säure,
iii. In-Kontakt-Bringen der Zusammensetzung aus ii. mit einem oberflächenmodifizierten festen Träger, wobei zwischen der Fängerverbindung und dem festen Träger eine kovalente Hydrazon- oder Oxim-Bindung ausgebildet wird, und eine Verbindung der Formel (13) bereitgestellt wird,
iv. Abspaltung des Volllängenpeptids vom festen Träger.

In einigen Ausführungsformen umfasst Schritt i. das In-Kontakt-Bringen einer Mischung von Volllängenpeptid und Abbruchsequenzen, die sich noch an der festen Phase (dem Syntheseharz) befinden, mit einer Verbindung (Fängermolekül) der allgemeinen Formel X₁-L-X₂ wobei X₁, X₂ und L wie oben definiert sind und wobei der Schritt des in-Kontakt-Bringens zu einer Reaktion der Verbindung X₁-L-X₂ an X₂ mit der freien N-terminalen Aminogruppe des Volllängenpeptids unter Ausbildung einer kovalenten Bindung führt. Die Abspaltung der Peptide von der festen Phase (Syntheseharz) erfolgt mittels Säuren, wodurch eine Mischung von Volllängenpeptid, das mit dem Fängermolekül kovalent verbunden ist und acetylierten Abbruchsequenzen von Peptiden aus einer Festphasen-Peptidsynthese (SPPS) erhalten wird. Die Trennung der festen und flüssigen Phase erfolgt z.B. durch Filtration. Die nicht-peptidischen Verunreinigungen werden durch Fällung in Ether bei einer Temperatur von -78 °C bis 0 °C entfernt. Vorzugsweise wird die Säuremischung dabei in den vorgelegten Ether gegeben, wobei alles peptidische Material ausfällt und organische Verunreinigungen im Ether verbleiben. Anschließend erfolgt eine Trennung der etherischen Lösung von der Peptidmischung z.B. durch Zentrifugation. Die Peptidmischung wird als amorpher Feststoff erhalten.

In einigen Ausführungsformen umfasst Schritt ii. die Aufnahme des amorphen Feststoffs aus Schritt i) in einer zumindest teilweise wässrigen Pufferlösung bei einem pH-Wert zwischen 2 und 4, bevorzugt zwischen 2,5 und 3,5 besonders bevorzugt bei 3. Die Einstellung des pH-Wertes erfolgt dabei geeignet durch Zugabe von Säuren oder Basen.

In einigen Ausführungsformen umfasst Schritt iii. das In-Kontakt-Bringen der Mischung aus ii) mit einem Oberflächen-modifizierten festen Träger (Reinigungsharz), zwecks kovalenter Anbindung der mit dem Fängermolekül (Schritt i) modifizierten Volllängenpeptide über die Bildung einer Hydrazon oder Oxim-Bindung. Besonders vorteilhaft ist hier die Zugabe von Aminen und/oder Essigsäure als Katalysator zur Verbesserung der Kinetik der Anbindungsreaktion.

Die nicht über die Hydrazon/Oxim-Bindung an den festen Träger gebundenen peptidischen Abbruchsequenzen werden durch Waschen mit organischen Lösungsmitteln und/oder mit Wasser und wässriger Pufferlösung, vorzugsweise unter Zusatz von chaotropen Stoffen, um eventuell nicht kovalent gebundene Peptide zu lösen, entfernt.

In einigen Ausführungsformen umfasst Schritt iv. die Abtrennung der Volllängenpeptide von der festen Phase durch Spaltung des Linkers L von Y unter basischen (nukleophilen) Bedingungen, wobei Y in Form von CO₂ oder SO₂ freigesetzt wird.

Bei der Abspaltung des Volllängenpeptids ausgehend von der Formel (13) (D-X₁'-L-Y-PEP), entstehen das Volllängenpeptid (PEP), CO₂ oder SO₂ (Y') und D-X₁'-L oder D' und X₁'-L'.

In einigen Ausführungsformen weist der feste Träger an seiner Oberfläche die funktionalen Gruppen Aldehyd, Keton, Hydroxylamin und Hydrazin auf.

In einigen Ausführungsformen weist der feste Träger an seiner Oberfläche die funktionale Gruppe -O-CH₂-CHO auf.

In einigen Ausführungsformen weist der feste Träger an seiner Oberfläche die funktionalen Gruppen - ONH₂ oder -N₂H₃ auf.

In einigen Ausführungsformen wird die feste Phase von den gewünschten Volllängenpeptiden durch Filtration getrennt; die feste Phase (Reinigungsharz, D) wird durch Behandlung mit Hydrazin (H₄N₂) und/oder Ammoniumhydroxid H₄NOH und/oder Aldehyden und/oder Ketonen und/oder Waschen mit Wasser regeneriert.

Zur Abspaltung der Peptide vom Syntheseharz (Schritt i) werden Säuren verwendet, bevorzugt sind hier organische und anorganische Säuren, die einen pks Wert unter 4 besitzen. Besonders geeignet sind Säuren ausgewählt aus der Gruppe der fluorhaltigen Säuren: Trifluoressigsäure (TFA), Flussäure (HF) und Triflourmethansulfonsäure. Außerdem geeignet sind Bromwasserstoffsäure (HBr), Chlorwasserstoffsäure (HCl), Schweflige Säure (H₂SO₃), Schwefelsäure (H₂SO₄), Phosphorsäure (H₃PO₄), Salpetersäure (HNO₃) oder Methansulfonsäure.

Zur Fällung in Schritt i) werden organische Lösungsmittel verwendet die bei den Fällungstemperaturen in flüssiger Form vorliegen, solche Lösungsmittel sind dem Fachmann allgemein bekannt. Bevorzugt werden organische Lösungsmittel aus der Gruppe der Ether, besonders bevorzugt sind Diethylether und/oder Methyl-tert-butylether. Auch Alkane, die bei den Fällungstemperaturen in flüssiger Form vorliegen, können verwendet werden, wobei n-Hexan und oder n-Pentan besonders bevorzugt sind. Geeignete, zumindest teilweise wässrige, Pufferlösungen, die in Schritt ii) Anwendung finden sind dem Fachmann bekannt, nämlich Puffer, die im pH Bereich 2-5 eine Pufferkapazität aufweisen, somit Puffer mit den Anionen: Citrat, Malat, Format, Lactat, Succinat, Acetat, Pivalat, und Phosphat in Kombination mit den Kationen: Natrium, Kalium, Ammonium (NH₄, NMe₄, NEt₄, NPr₄, NBu₄, HNC₅H₅).

Zur Einstellung des pH-Wertes können organische oder anorganische Säuren, bevorzugt HCl und als Basen bevorzugt Alkali- und/oder Erdalkali-Hydroxide, besonders bevorzugt NaOH und/oder KOH, verwendet werden.

Zur besseren Löslichkeit des Peptids kann es in Schritt ii) vorteilhaft sein, dem System mit Wasser mischbare organische Lösungsmittel zuzugeben, solche Lösungsmittel sind dem Fachmann allgemein bekannt und können ausgewählt sein aus der Gruppe: Dimethylformamid (DMF), Acetonitril. Tetrahydrofuran (THF), Dioxan, Pyridin, Aceton, Dimethylsulfoxid (DMSO), Methanol, Ethanol, 1-Propanol, 2-Propanol, 1-Butanol, Formamid, N -Methylpyrolidon (NMP).

Zur Beschleunigung der Immobilisierung in Schritt iii) können der wässrigen Lösung Amine und/oder Essigsäure zugegeben werden. Amine können dabei ausgewählt sein aus der Gruppe bestehend aus: Pyridin, Piperidin, Methylamin, Ethylamin, Propylamin, Butylamin, Anilin und Dimethylamin.

Als oberflächenmodifizierter fester Träger (Reinigungsharz) in Schritt iii) kommen synthetische und natürliche Polymere in Frage. Die Oberflächenmodifizierung ist dabei derart, dass sie mit X₁ zu Hydrazonen oder Oximen reagiert. Ist X₁ eine Verbindung gemäß Formel (2) oder (3), so besteht die Oberflächenmodifizierung in aldehydischen oder ketonischen Gruppen, welche dann entsprechend zu Hydrazonen oder Oximen reagieren. Ist X₁ eine Aldehyd- oder Ketonfunktion der allgemeinen Formel (4), so sollte die Oberflächenmodifizierung -ONH₂, oder -N₂H₃, aufweisen. Bevorzugt als feste Träger sind oberflächenmodifizierte natürliche oder auch Biopolymere, besonders bevorzugt oberflächenmodifizierte Polysacharide. Ganz besonders bevorzugt ist die Verwendung von aldehydmodifizierter Sepharose/Agarose, sowie Cellulose, wobei X₁ eine Verbindung nach Formel (3) ist, wobei R¹ und R³ H sind.

Im Schritt iii. (Waschen der an das Reinigungsharz gebundenen Volllängenpeptide) kann mit Wasser, wässrigen Waschlösungen oder organischen Lösungsmitteln gewaschen werden. Als chaotrope Stoffe zu der wässrigen Waschlösung in Schritt iii. sind geeignet: Bariumsalze, Guadiniumhydrochlorid, Guadiniumthiocyanate, Thiocyanate, Perchlorate, Jodide, Butanol, Phenol, Thioharnstoff, Harnstoff, oder Ammoniumsulfat. Lösungsmittel können ausgewählt sein aus der Gruppe von: Dichlormethan (DCM), Trichlormethan, Tetrachlorkohlenstoff, Ethylacetat, Diethylether, Methyl-tert-butylether, Essigsäure, 2,2,2-Trifluorethanol, Hexafluorisopropanol, Dimethylformamid (DMF), Acetonitril. Tetrahydrofuran (THF), Dioxan, Pyridin, Aceton, Dimethylsulfoxid (DMSO), Methanol, Ethanol, 1-Propanol, 2-Propanol, 1-Butanol, Phenol, Formamid und N-Methylpyrolidon (NMP).

Die Spaltung in Schritt iv. erfolgt durch Basen in wässrigen Lösungen oder organischen Lösungsmitteln, die Peptide lösen. Basen können ausgewählt sein aus der Gruppe von: LiOH, NaOH, KOH, Ammonium (NH₄, NMe₄, NEt₄, NPr₄, NBu₄, HNEt(iPr)₂, HNMe₃, HNEt₃, HNPr₃, HNBu₃, HNC₅H₅) hydroxide, Piperidin, Methylamin, Ethylamin, Propylamin, Butylamin, Hydrazin, Hydroxylamin, Methylhydrazin und O-Methylhydroxylamin. Organischen Lösungsmitteln, die Peptide lösen, können ausgewählt sein aus der Gruppe von: Dimethylformamid (DMF), Acetonitril. Tetrahydrofuran (THF), Dioxan, Pyridin, Aceton, Dimethylsulfoxid (DMSO), Methanol, Ethanol, 1-Propanol, 2-Propanol und 1-Butanol.

Die Filtration in Schritt iv) erfolgt bevorzugt mittels handelsüblichen Spritzenreaktoren oder Filtersystemen. Die Filterlochgrößen sollten zwischen 10 bis 100 µm liegen.

In einigen Ausführungen wird nach oder bei der Abspaltung des Volllängenpeptids vom festen Träger der feste Träger D von dem Rest X¹-L der Fängerverbindung abgespalten und der feste Träger regeneriert.

Ein besonderer Vorteil der erfindungsgemäßen Methode liegt in der Reversibilität der Hydrazonsowie Oximbindung.

Das hier beschriebene Verfahren kann durch die Gleichgewichtsnatur der Hydrazon/Oximbindung wie eine Affinitätschromatographie benutzt werden. Nach dem Aus- oder Wegwaschen der Verunreinigungen und der Spaltung des basenlabilen Linkers und somit Erhalt des Zielpeptides kann das Reinigungsharz wieder regeneriert werden und ist somit für eine weitere Reinigung zugänglich. Befindet sich an der Oberfläche des Reinigungsharzes ursprünglich Aldehyd- oder Ketogruppen, stellt Waschen mit saurer wässriger Lösung in der Aldehyde oder Ketone gelöst sind die Aldehyd oder Keton Funktion wieder her. Befindet sich an der Oberfläche des Reinigungsharzes ursprünglich Hydrazin oder Hydroxylaminderivate, stellt Waschen mit saurer wässriger Lösung mit Hydrazin oder Hydroxylamin die Hydrazin oder Hydroxylaminfunktion wieder her. Es wird dasselbe Material wie bei der Protein-Aufreinigung mittels Affinitätschromatographie verwendet, Sepharose/ Agarose.

Des Weiteren lässt sich das Verfahren auf Cellulose übertragen, welches das häufigste Biomaterial auf der Erde darstellt und somit kostengünstig verfügbar ist.

Proteine werden standardmäßig über Affinitätschromatographie aufgereinigt, und diese Methode ist auch im Vergleich zur HPLC Reinigung sehr kostengünstig und effizient, sowie skalierbar. Durch die niedrigen Drücke und höheren Beladungsdichten ist die Affinitätschromatographie auch viel besser für große Synthesemengen geeignet als die HPLC.

In einem Unteraspekt des 1. Aspekts der vorliegenden Erfindung wird die Aufgabe durch eine Verbindung gelöst, die als Linker eine Verbindung zwischen der N-terminalen Aminogruppe eines Volllängenpeptids und einer festen Phase herstellt. Die erfindungsgemäße Verbindung weist die allgemeine Formel

X₁-L-X₂

auf, wobei
X₁ ausgewählt ist aus 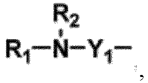 worin Y₁ = O, N und wobei R₁ und R₂ gleich oder verschieden sein können und R₁ und R₂ H oder B ist, wobei B eine nicht basenlabile Schutzgruppe für eine Aminogruppe ist, die unter sauren Bedingungen Amine liefert.

In einer bevorzugten Ausführungsform ist B ausgewählt aus der Gruppe: Boc (-C=OOtBu), Trityl (-C(Ph)₃), Mmt (-C(Ph)₂C₆H₄OMe), DMT (-C(Ph)(C₆H₄OMe)₂), Cbz (-C=OOCH₂Ph), Benzylidenamin (=CPh), Phtalimid (=(CO)₂C₆H₄), p-Toluolsulfonamid (-SO₂ C₆H₄Me), Benzylamin (-CH₂Ph), Acetamid (-COMe), Trifluoracetamid (-COCF₃), Dde (1-(4,4-Dimethyl-2,6-dioxocyclohex -1-yliden )-3-ethyl) und 1-(4,4-Dimethyl-2,6-dioxocyclohex-lyliden)-3-methylbutyl (ivDde).

Dem Fachmann sind weiter Schutzgruppen aus P. G. M. Wuts, T. W. Greene, Greene's Protective Groups in Organic Synthesis, 4th ed., Wiley, 2007, Seite 696-926 bekannt.

X₁ kann alternativ 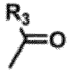 sein, wobei R₃ = H ist, oder eine gesättigte oder ungesättigte, verzweigte oder nicht verzweigte, substituierte oder nicht substituierte aliphatische oder aromatische Kette einer Länge von bis zu 12 Kohlenstoffatomen ist und wobei die Aldehyd-, oder Keto-Gruppe in einer dem Fachmann bekannten Art und Weise geschützt vorliegen kann 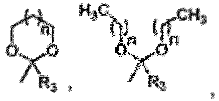 wobei n zwischen 0 und 12 sein kann, besonders bevorzugt 0, 1 oder 2.

Als geschütztes Aldehyd oder Keton können alle Acetal-Ketalschutzgruppen dienen, die sich unter sauren Bedingungen spalten lassen. Eine Übersicht findet sich insbesondere in: P. G. M. Wuts, T. W. Greene, Greene's Protective Groups in Organic Synthesis, 4th ed., Wiley, 2007, Seite 435-477.

X₂ kann 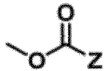 sein, worin Z eine elektronenziehende Fluchtgruppe ist, die bei einem heterolytischen Bindungsbruch das bindende Elektronenpaar mitnimmt. In einer besonderen Ausführungsform ist Z ausgewählt aus der Gruppe F, Cl, Br, J, N₃, SR₈ (worin R₈ wie R₃ definiert ist), OCF₃, OCH₂CF₃,

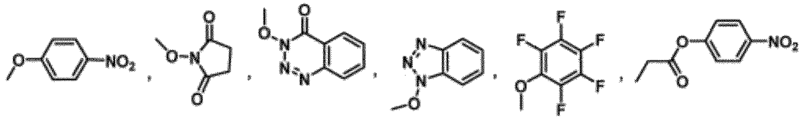

X₂ stellt somit eine Carbamatvorstufe dar, die mit der Aminogruppe des Volllängenpeptids ein Carbamat bilden soll.

L ist ein funktioneller Linker, der X₁ und X₂ trennt und unter basischen Bedingungen (nukleophil) spaltbar ist. Eine Auswahl der nukleophil spaltbaren Linker findet sich bei F. Guillier, D. Drain, M. Bradley, Linkers and Cleavage Strategies in Solid-Phase Organic Synthesis and Combinatorial Chemistry, Chem. Rev. 2000, 100, 2091-2157.

In einigen Ausführungsformen sind die Verbindungen nach der allgemeinen Formel X₁-L-X₂ pharmazeutisch akzeptable Salze davon.

In einigen Ausführungsformen wird die Verbindung nach der allgemeinen Formel X₁-L-X₂ als Linker verwendet, der eine Verbindung zwischen der N-terminalen Aminogruppe eines Volllängenpeptids und einer festen Phase bildet.

In einer bevorzugten Ausführungsform ist der Linker L ausgewählt aus den allgemeinen Strukturen a, b oder c 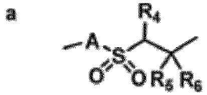
A: C₀-C₁₂, aromatisch, aliphatisch, ungesättigt, gesättigt
R₄: H, Alkyl oder elektronenziehende Gruppe
R₅, R₆: gleich oder verschieden H oder Alkyl C₁-C₁₂ 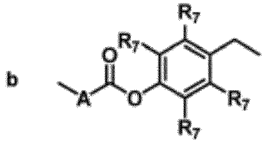
R₇: gleich oder verschieden H, elektronenziehende Gruppe oder Alkyl C₁-C₁₂ 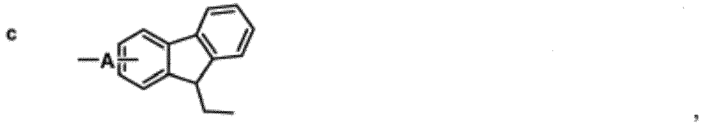
worin A eine gesättigte oder ungesättigte, verzweigte oder nicht verzweigte, substituierte oder nicht substituierte aliphatische oder aromatische Kette mit einer Länge von 0 bis zu 12 Kohlenstoffatomen ist und wobei R₄ = H, eine Alkylkette von 0 bis zu 12 Kohlenstoffatomen oder eine Gruppe ist, die fähig ist, Elektronen durch einen induktiven oder mesomeren Effekt an sich zu ziehen.

Wobei R₅ und R₆ gleich oder verschieden sein können und R₅, R₆ = H, eine Alkylkette mit einer Länge von 1 bis 12 Kohlenstoffatomen oder eine Gruppe ist, die fähig ist Elektronen durch einen induktiven oder mesomeren Effekt an sich zu ziehen. R₇ ist H, eine Alkylkette mit einer Länge von 1 bis 12 Kohlenstoffatomen oder eine Gruppe die fähig ist Elektronen durch einen induktiven oder mesomeren Effekt an sich zu ziehen.

R₈ ist wie R₃.

In einem weiteren Aspekt wird die Aufgabe der Erfindung durch ein Verfahren gelöst, das die folgenden Schritte umfasst.
i) In-Kontakt-Bringen einer Mischung von Volllängenpeptid und Abbruchsequenzen, die sich noch an der festen Phase (dem Syntheseharz) befinden, mit einer Verbindung (Fängermolekül) der allgemeinen Formel X₁-L-X₂ wobei X₁, X₂ und L wie oben definiert sind und wobei der Schritt des in-Kontakt-Bringens zu einer Reaktion der Verbindung X₁-L-X₂ an X₂ mit der freien N-terminalen Aminogruppe des Volllängenpeptids unter Ausbildung einer kovalenten Bindung führt.
ii) Abspaltung der Peptide von der festen Phase (Syntheseharz) mittels Säuren und Erhalten einer Mischung von Volllängenpeptid, das mit dem Fängermolekül kovalent verbunden ist und acetylierten Abbruchsequenzen von Peptiden aus einer Festphasen-Peptidsynthese (SPPS);
iii) Trennung der festen und flüssigen Phase, z.B. durch Filtration;
iv) Entfernen der nicht-peptidischen Verunreinigungen durch Fällung in Ether bei einer Temperatur von -78 °C bis 0 °C. Vorzugsweise wird die Säuremischung dabei in den vorgelegten Ether gegeben, wobei alles peptidische Material ausfällt und organische Verunreinigungen im Ether verbleiben. Anschließend erfolgt eine Trennung der etherischen Lösung von der Peptidmischung z.B. durch Zentrifugation. Die Peptidmischung wird als amorpher Feststoff erhalten;
v) Aufnahme des amorphen Feststoffs aus Schritt iv) in einer zumindest teilweise wässrigen Pufferlösung bei einem pH-Wert zwischen 2 und 4, bevorzugt zwischen 2,5 und 3,5 besonders bevorzugt bei 3. Die Einstellung des pH-Wertes erfolgt dabei geeignet durch Zugabe von Säuren oder Basen;
vi) In Kontakt bringen der Mischung aus v) mit einem Oberflächen-modifizierten festen Träger (Reinigungsharz), zwecks kovalenter Anbindung der mit dem Fängermolekül (Schritt i) modifizierten Volllängenpeptide über die Bildung einer Hydrazon oder Oxim-Bindung. Besonders vorteilhaft ist hier die Zugabe von Aminen und/oder Essigsäure als Katalysator zur Verbesserung der Kinetik der Anbindungsreaktion;
vii) Entfernung der nicht über die Hydrazon/Oxim-Bindung an den festen Träger gebundenen peptidischen Abbruchsequenzen durch Waschen mit organischen Lösungsmitteln und/oder mit Wasser und wässriger Pufferlösung, vorzugsweise unter Zusatz von chaotropen Stoffen, um eventuell nicht kovalent gebundene Peptide zu lösen;
viii) Abtrennung der Volllängenpeptide von der festen Phase durch Spaltung des Linkers L unter basischen (nukleophilen) Bedingungen;
ix) Filtration zur Abtrennung der festen Phase von den gewünschten Volllängenpeptiden; und
x) Regeneration der festen Phase (Reinigungsharz) durch Behandlung mit Hydrazin (H₄N₂) und/oder Ammoniumhydroxid H₄NOH und/oder Aldehyden und/oder Ketonen und/oder Waschen mit Wasser.

Zur Abspaltung der Peptide vom Syntheseharz (Schritt ii)) werden Säuren verwendet, bevorzugt sind hier organische und anorganische Säuren die einen pks Wert unter 4 besitzen. Besonders geeignet sind Säure ausgewählt aus der Gruppe der fluorhaltigen Säuren: Trifluoressigsäure (TFA), Flussäure (HF) und Triflourmethansulfonsäure. Außerdem geeignet sind Bromwasserstoffsäure (HBr), Chlorwasserstoffsäure (HCl), Schweflige Säure (H₂SO₃), Schwefelsäure (H₂SO₄), Phosphorsäure (H₃PO₄), Salpetersäure (HNO₃), Methansulfonsäure.

Zur Fällung in Schritt iii) werden organische Lösungsmittel verwendet die bei den Fällungstemperaturen in flüssiger Form vorliegen, solche Lösungsmittel sind dem Fachmann allgemein bekannt. Bevorzugt werden organische Lösungsmittel aus der Gruppe der Ether, besonders bevorzugt sind Diethylether und/oder Methyl-tert-butylether. Auch Alkane die bei den Fällungstemperaturen in flüssiger Form vorliegen können verwendet werden besonders bevorzugt sind n-Hexan und oder n-Pentan.

Geeignete zumindest teilweise wässrige Pufferlösungen die in Schritt v) Anwendung finden sind dem Fachmann bekannt, nämlich Puffer, die im pH Bereich 2-5 eine Pufferkapazität aufweisen, somit Puffer mit den Anionen: Citrat, Malat, Format, Lactat, Succinat, Acetat, Pivalat, und Phosphat in Kombination mit den Kationen: Natrium, Kalium, Ammonium (NH₄, NMe₄, NEt₄, NPr₄, NBu₄, HNC₅H₅).

Zur Einstellung des pH-Wertes können organische oder anorganische Säuren, bevorzugt HCl und als Basen bevorzugt Alkali- und/oder Erdalkali-Hydroxide, besonders bevorzugt NaOH und/oder KOH, verwendet werden.

Zur besseren Löslichkeit des Peptids kann es in Schritt v) vorteilhaft sein, dem System mit Wasser mischbare organische Lösungsmittel zuzugeben, solche Lösungsmittel sind dem Fachmann allgemein bekannt und können ausgewählt sein aus der Gruppe: Dimethylformamid (DMF), Acetonitril. Tetrahydrofuran (THF), Dioxan, Pyridin, Aceton, Dimethylsulfoxid (DMSO), Methanol, Ethanol, 1-Propanol, 2-Propanol, 1-Butanol, Formamid und N -Methylpyrolidon (NMP).

Zur Beschleunigung der Immobilisierung in Schritt vi) können der wässrigen Lösung Amine und/oder Essigsäure zugegeben werden. Amine können dabei ausgewählt sein aus der Gruppe bestehend aus: Pyridin, Piperidin, Methylamin, Ethylamin, Propylamin, Butylamin, Anilin und Dimethylamin.

Als oberflächenmodifizierter fester Träger (Reinigungsharz) in Schritt vi) kommen synthetische und natürliche Polymere in Frage. Die Oberflächenmodifizierung ist dabei derart, dass sie mit X₁ zu Hydrazonen oder Oximen reagiert. Ist X₁ = 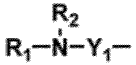 (Y₁ =NH, O) so besteht die Oberflächenmodifizierung in aldehydischen oder ketonischen Gruppen, welche dann entsprechend zu Hydrazonen oder Oximen reagieren. Ist X₁ eine Aldehyd- oder Ketonfunktion der allgemeinen Formel-R₃C=O, so sollte die Oberflächenmodifizierung eine NH₂-Y₁-R₇ (R₇ = fester Träger) Gruppe aufweisen. Bevorzugt als feste Träger sind oberflächenmodifizierte natürliche oder auch Biopolymere, besonders bevorzugt oberflächenmodifizierte Polysacharide. Ganz besonders bevorzugt ist die Verwendung von Aldehyd-modifizierter Sepharose/Agarose, sowie Cellulose und X₁ = NH₂-NH-C=OO-.

Im Schritt vi. (Waschen der an das Reinigungsharz gebundenen Volllängenpeptide) kann mit Wasser, wässrigen Waschlösungen oder organischen Lösungsmitteln gewaschen werden. Als chaotrope Stoffe zu der wässrigen Waschlösung in Schritt vi. sind geeignet: Bariumsalze, Guadiniumhydrochlorid, Guadiniumthiocyanate, Thiocyanate, Perchlorate, Iodide, Butanol, Phenol, Thioharnstoff, Harnstoff, Ammoniumsulfat. Lösungsmittel können ausgewählt sein aus der Gruppe: Dichlormethan (DCM), Trichlormethan, Tetrachlorkohlenstoff, Ethylacetat, Diethylether, Methyl-tert-butylether, Essigsäure, 2,2,2-Trifluorethanol, Hexafluorisopropanol, Dimethylformamid (DMF), Acetonitril. Tetrahydrofuran (THF), Dioxan, Pyridin, Aceton, Dimethylsulfoxid (DMSO), Methanol, Ethanol, 1-Propanol, 2-Propanol, 1-Butanol, Phenol, Formamid und N-Methylpyrolidon (NMP).

Die Spaltung in Schritt vii. erfolgt durch Basen in wässrige Lösungen oder organischen Lösungsmitteln, die Peptide lösen. Basen können ausgewählt sein aus der Gruppe: LiOH, NaOH, KOH, Ammonium (NH₄, NMe₄, NEt₄, NPr₄, NBu₄, HNEt(iPr)₂, HNMe₃, HNEt₃, HNPr₃, HNBu₃, HNC₅H₅) hydroxide, Piperidin, Methylamin, Ethylamin, Propylamin, Butylamin, Hydrazin, Hydroxylamin, Methylhydrazin und O-Methylhydroxylamin. Organischen Lösungsmitteln, die Peptide lösen, können ausgewählt sein aus der Gruppe: Dimethylformamid (DMF), Acetonitril. Tetrahydrofuran (THF), Dioxan, Pyridin, Aceton, Dimethylsulfoxid (DMSO), Methanol, Ethanol, 1-Propanol, 2-Propanol und 1-Butanol. Die Filtration in Schritt viii) erfolgt bevorzugt mittels handelsüblichen Spritzenreaktoren oder Filtersystemen. Die Filterlochgrößen sollten zwischen 10 bis 100 µm liegen.

In einigen Ausführungsformen ist das erfindungsgemäße Verfahren dadurch gekennzeichnet, dass die Trennung der festen und flüssigen Phasen durch Filtration erfolgt.

In einigen Ausführungsformen ist das erfindungsgemäße Verfahren dadurch gekennzeichnet, dass in Schritt vi. Amine und/oder Essigsäure als Katalysator zugegeben werden.

In einigen Ausführungsformen ist das erfindungsgemäße Verfahren dadurch gekennzeichnet, dass die Pufferlösung in Schritt v. einen pH-Wert zwischen 2.5 und 3.5 und bevorzugt bei 3 aufweist.

In einigen Ausführungsformen ist das erfindungsgemäße Verfahren dadurch gekennzeichnet, dass die Entfernung in Schritt vii. durch Waschen mit organischen Lösungsmitteln und/oder mit Wasser und wässriger Pufferlösung, vorzugsweise unter Zusatz von chaotropen Stoffen, um eventuell nicht kovalent gebundene Peptide zu lösen, erfolgt.

In einigen Ausführungsformen ist das erfindungsgemäße Verfahren dadurch gekennzeichnet, dass die Abtrennung der Volllängenpeptide von der festen Phase in Schritt viii. durch Spaltung des Linkers L unter basischen (nukleophilen) Bedingungen erfolgt.

In einigen Ausführungsformen ist das erfindungsgemäße Verfahren dadurch gekennzeichnet, dass zur Abtrennung der Peptide vom Syntheseharz in Schritt ii. Säuren verwendet werden, die einen pks Wert von unter 4 aufweisen.

In einigen Ausführungsformen ist das erfindungsgemäße Verfahren dadurch gekennzeichnet, dass zur Fällung in Schritt iii. organische Lösungsmittel aus der Gruppe der Ether, besonders bevorzugt Diethylether und/oder Methyl-tert-butylether oder n-hexan und oder n-Pentan verwendet werden.

In einigen Ausführungsformen ist das erfindungsgemäße Verfahren dadurch gekennzeichnet, dass als oberflächenmodifizierter fester Träger (Reinigungsharz) in Schritt vi. synthetische und natürliche Polymere verwendet werden, z.B. oberflächenmodifizierte Polysaccharide, besonders bevorzugt Aldehyd-modifizierte Sepharose/Agarose, oder Cellulose und X₁= NH₂-NH-C=OO-.

Ein besonderer Vorteil der erfindungsgemäßen Methode liegt in der Reversibilität der Hydrazonsowie Oximbindung.

Das hier beschriebene Verfahren kann durch die Gleichgewichtsnatur der Hydrazon/Oximbindung wie eine Affinitätschromatographie benutzt werden. Nach dem Aus- oder Wegwaschen der Verunreinigungen und der Spaltung des basenlabilen Linkers und somit Erhalt des Zielpeptides kann das Reinigungsharz wieder regeneriert werden und ist somit für eine weitere Reinigung zugänglich. Befindet sich an der Oberfläche des Reinigungsharzes ursprünglich Aldehyd- oder Ketogruppen, stellt Waschen mit saurer wässriger Lösung in der Aldehyde oder Ketone gelöst sind die Aldehyd oder Keton Funktion wieder her. Befindet sich an der Oberfläche des Reinigungsharzes ursprünglich Hydrazin oder Hydroxylaminderivate, stellt Waschen mit saurer wässriger Lösung mit Hydrazin oder Hydroxylamin die Hydrazin oder Hydroxylaminfunktion wieder her. Es wird dasselbe Material wie bei der Protein-Aufreinigung mittels Affinitätschromatographie verwendet, Sepharose/ Agarose.

Des Weiteren lässt sich das Verfahren auf Cellulose übertragen, welches das häufigste Biomaterial auf der Erde darstellt und somit kostengünstig verfügbar ist.

Proteine werden standardmäßig über Affinitätschromatographie aufgereinigt, und diese Methode ist auch im Vergleich zur HPLC Reinigung sehr kostengünstig und effizient, sowie skalierbar. Durch die niedrigen Drücke und höheren Beladungsdichten auch vielmehr für große Synthesemengen geeignet als die HPLC.

Im Folgenden soll, die Allgemeinheit der Lehre nicht einschränkend, die Erfindung anhand einiger Beispiele mit Bezug auf die Figuren erläutert werden.

### Allgemeines Syntheseschema

Die erfindungsgemäßen Fängermoleküle können nach dem allgemeinen Syntheseschema (1) hergestellt werden. Nach diesem Schema kann der nukleophile linke Teil des Linkermoleküls zwischen Hydroxylamin und Hydrazin variiert werden. Der spaltbare Teil des Linkers L kann ebenfalls variiert werden. Es kann zwischen Sulfonlinkern und Phenolester-Linkersystemen unterschieden werden. Darüber hinaus kann der rechte Carbonatteil des Linkersystems unterschiedliche Fluchtgruppen aufweisen. Somit ergibt sich eine Fülle an Kombinationsmöglichkeiten Die Bausteine L1 und L3 sind kommerziell erhältlich L2 kann nach Schema 2 hergestellt werden.

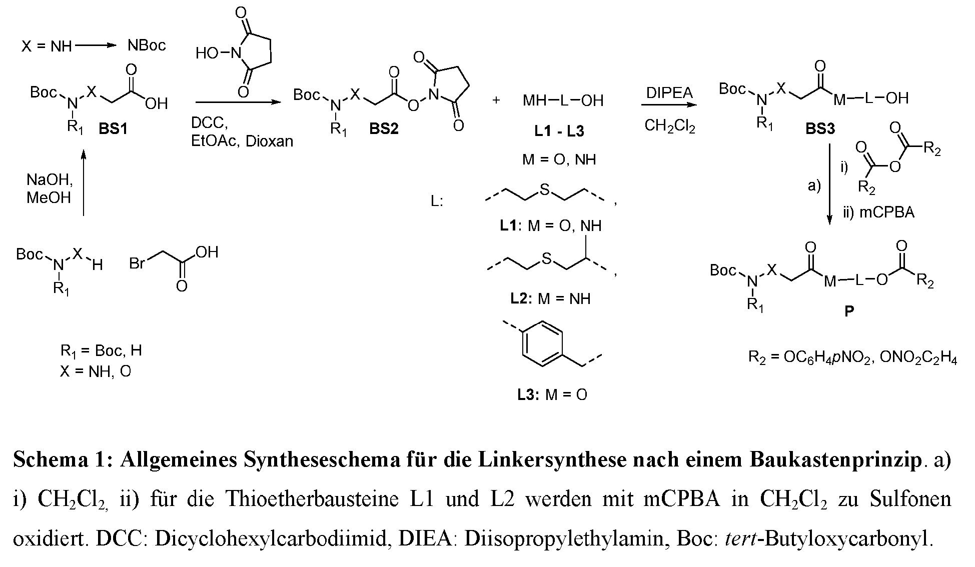

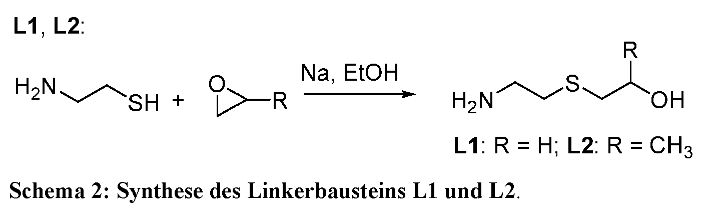

### Beschreibung der Abbildungen

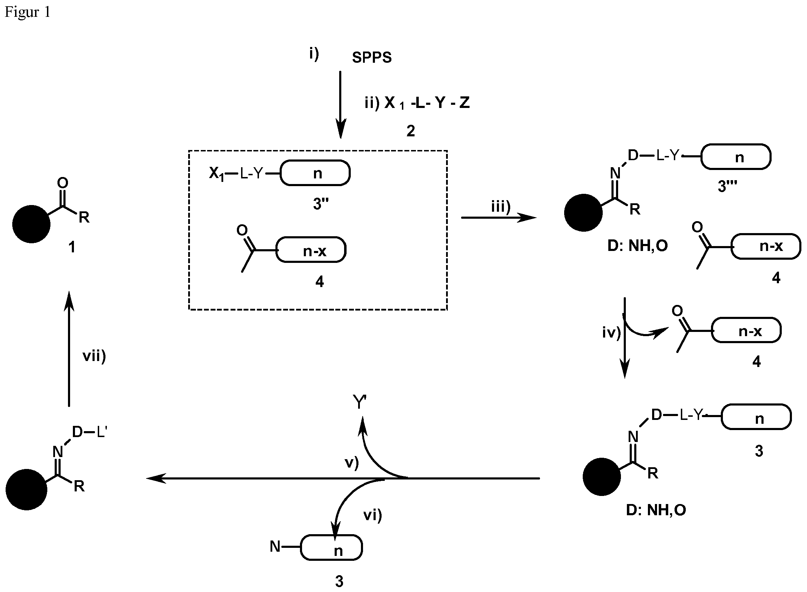

Figur 1 zeigt eine schematische Darstellung des erfindungsgemäßen Verfahrens zur Verdeutlichung desselben. i) SPPS mit Acetylierung nach jeder Kupplung, n-fache Wiederholung; ii) Zugabe von Molekül X1-L-X2, iii) Immobilisierung; iv) Waschen; v) basische Spaltung; vi) Filtration; vii) Regeneration. 1: Reinigungsharz, 2: Fängermolekül, 3: natives Peptid (gewünschtes Produkt), 4: Abbruchsequenzen, x = 1 bis n, n: Volllängenpeptid, n-x: Abbruchsequenzen.

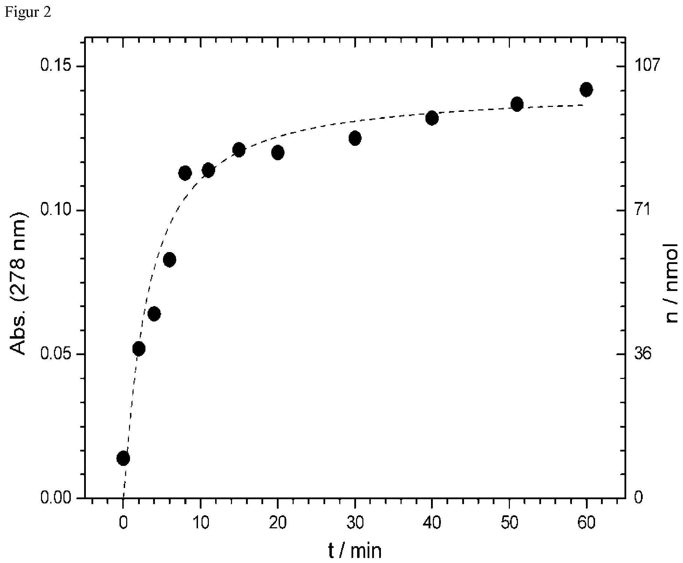

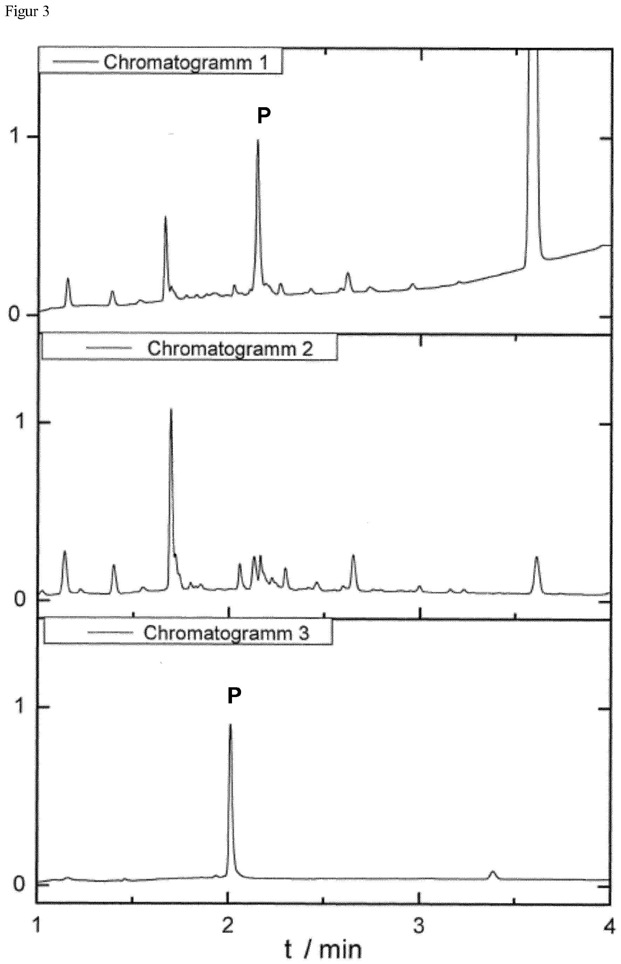

Figur 2 zeigt die Absorption bei 278 nm bei Vermessung des Überstandes nach Abspaltung des Peptids (Verbindung 3 in Schema 3) mit 0,5 vol. % N₂H₄ zur Demonstration der Reversibilität der Hydrazonbindung aus Beispiel 1. Abs.: Absorption des Überstandes von mit Peptid beladenen Aldehyd-modifizierten Agarose-Trägers bei 278 nm. t: Zeit, die nach der Zugabe von einer 0.5% Hydrazinlösung vergangen ist in Minuten (min). n: Aufgrund der Absorption berechnete Menge an Peptid im Überstand in nmol. Gestrichelte-Linie: Nicht-lineare Regression nach Hill mit der Formel: y = 0.142^{∗}x/(3.16+x), R² = 92, t_{1/2} = 3.16 min. Figur 3 zeigt Chromatogramme der einzelnen Phasen bei erfindungsgemäßer Reinigung eines Peptides nach Nativer Chemischer Ligation (NCL) aus Beispiel 2.

Chromatogramm 1 zeigt das gewünschte Produkt (P) im Reaktionsgemisch der NCL nach 24 Stunden. Chromatogramm 2 zeigt den Überstand nach 30 Minuten Immobilisierung. Chromatogramm 3 zeigt das gewünschte Produkt (P) nach 30-minütigem Waschen und Abspalten. P: Peak des gewünschten Produkts.

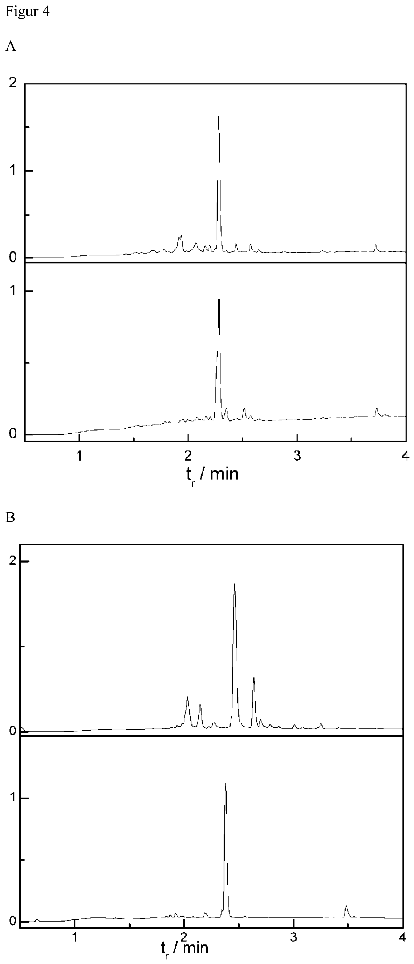

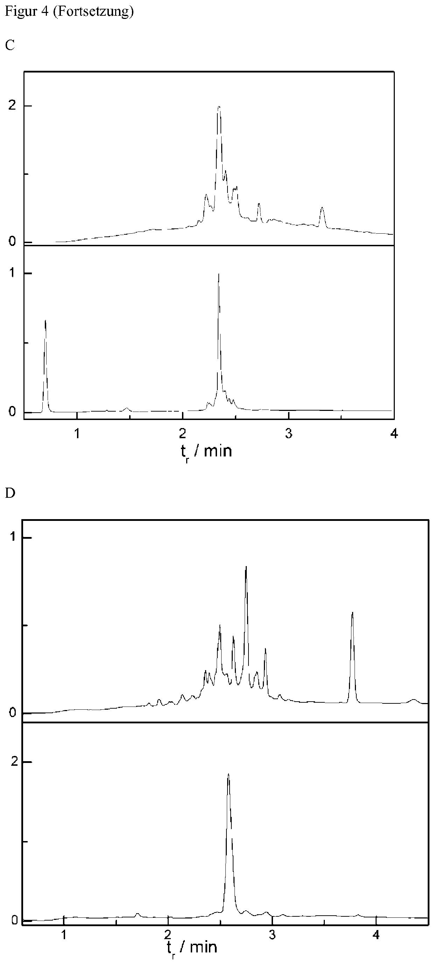

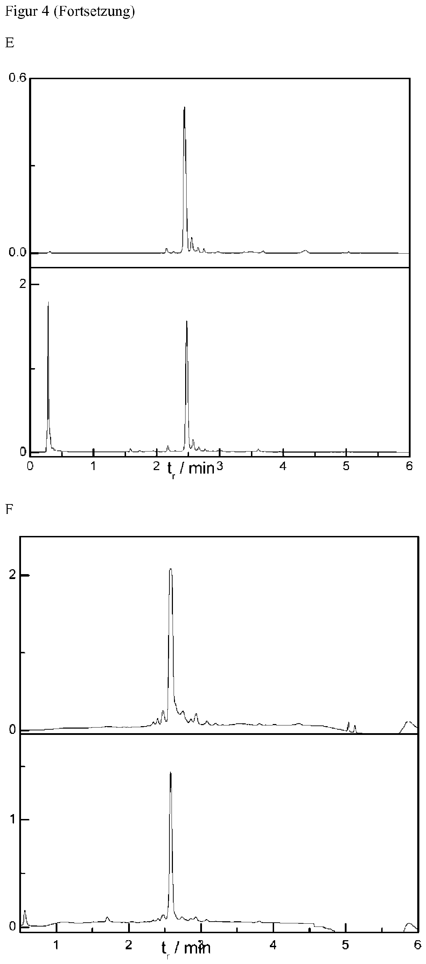

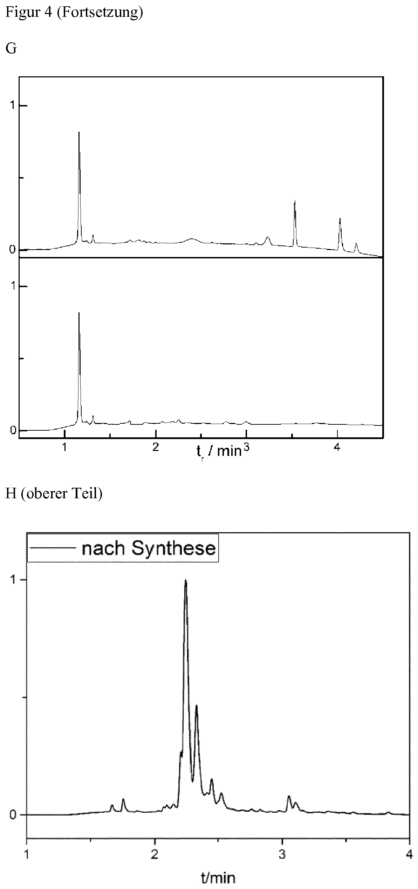

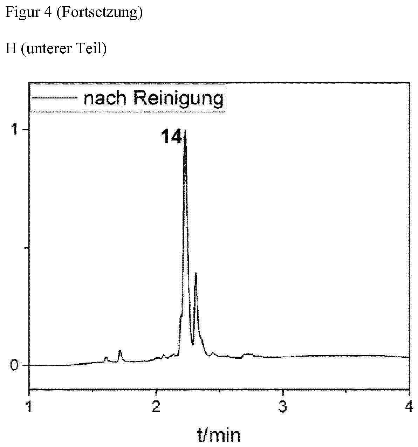

Figur 4 zeigt Chromatogramme bei erfindungsgemäßer Reinigung der Peptide 7-13 nach der Festphasenpeptidsynthese aus Beispiel 4. Die Chromatogramme, die mit Überstand beschriften sind, zeigen die Verunreinigungen, welche mit der Methode abgetrennt werden konnten. FM = erfindungsgemäßes Fängermolekül; (a) Peptid 7 (Taul); (b) Peptid **8** (Tau2); (c) Peptid 9 (GNRH), (d) Peptid 10 (Magainin); (e) Peptid 11 (Terts72Y); (f) Peptid 12 (Bivalrudin); (g) Peptid 13 (TAT), (h) Peptid 14 (Forschungspeptid). Das jeweils obere Diagramm zeigt das Peptid nach der Synthese und das jeweils untere Diagramm zeigt das Peptid nach der Reinigung.

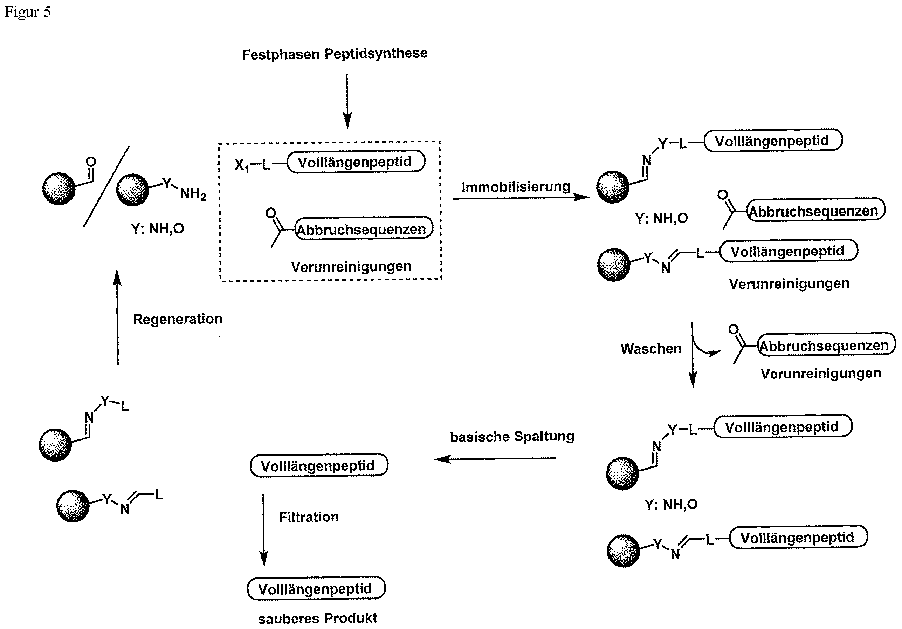

Figur 5 zeigt eine schematische Darstellung des erfindungsgemäßen Verfahrens zur Verdeutlichung desselben. Figur 5 zeigt eine alternative Darstellung zu Figur 1.

SEQ ID Nos: 1 bis 10 zeigen Peptide aus den Beispielen 1, 2 und 4.

### Beispiele

### Beispiel 1. Demonstration der Reversibilität der Hydrazonbindung/Oximbindung

Die reversible Anbindung des Peptids an Aldehyd-modifizierte Agarosebeads, wird im Folgenden am Beispiel der Hydrazonbindung demonstriert, durch die elektronische Ähnlichkeit (siehe auch A. Dirksen, P. Dawson, Bioconjugate Chem. 2008, 19, 2543-2548.) sind die Ergebnisse auf die Oxim-Bindung übertragbar. Es zeigt sich, dass das Gleichgewicht durch die Zugabe von Hydrazin (N₂H₄) gesteuert werden kann.

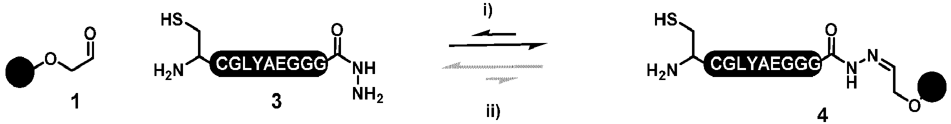

Das Peptid 3 wurde in dem Konjugationspuffer (0,1 M NH₄OAc, 0,1 M PhNH₂, pH= 3) in 30 min auf den Träger 1 gebracht (Schema 3). Anschließend wurde mit Wasser gewaschen und der Überstand entfernt. Dann wurde auf die Beads eine Lösung von 0,5 Volumenprozent Hydrazin-hydrat gegeben und die Absorption bei 278 nm des Überstandes (200 µL) im zeitlichen Abstand gemessen (mit dem Gerät ND-I000 Spectrophotometer der Firma NanoDrop Technologie). Dabei zeigte sich, dass nach 10 min 80% des Peptides im Überstand zu messen ist. Nach weiteren 50 Minuten konnten 86 % des Peptides rückgewonnen werden. Durch nicht-lineare Regression wurde eine Halbwertszeit von drei Minuten ermittelt, Figur 2 zeigt die Adsorption bei 278 nm bei Vermessung des Überstandes nach Zugabe von N₂H₄.

### Beispiel 2: Reinigung eines Peptides nach der Nativen Chemischen Ligation (NCL)

Es wurde die Reinigung eines Peptids aus einem komplexen System, das ein Gemisch peptidischen Materials, sowie organische und anorganische Verunreinigungen enthielt durchgeführt.

Die zu reinigende Mischung wurde nach einer NCL erhalten (Schema 4). Diese Reaktion findet im wässrigen Puffersystem statt und wird verwendet, um größere Peptide und Proteindomänen zu synthetisieren. Nach 24 h Reaktionszeit der NCL liegt die rohe Mischung vor, die im Wesentlichen das gewünschte Ligationsprodukt, sowie den Thioester (H-YENRIK-MESA), der im Überschuss eingesetzt worden ist, enthielt. Dieses ist im Chromatogramm (UPLC-MS der Firma Waters, Acquity H-Class PDA/QDa, auf Polaris C18 A 5 µm 250/4 Säule) in Figur 3 zu sehen.

Dem Ligationspuffer (0,1 M Na₂HPO₄, 20 mM TCEP, 50 mM MesNa, pH = 7) wurde das doppelte Volumen Konjugationspuffer zugesetzt. Anschließend wurden modifizierte Sepharosebeads hinzugegeben und das Zweiphasensystem wurde 30 Minuten geschüttelt. Es wurde der Überstand des Sepharosegels mittels UPLC-MS analysiert (Fig. 3, Chromatogramm 2), in dem angeschlossenen Massenspektrometer konnte keine Masse gefunden werden, die sich dem Ligationsprodukt zuordnen ließ. Da aber ein Absorptionssignal bei der Retentionszeit des Produktes zu sehen ist, wird hierbei auch deutlich, dass Verunreinigungen entfernt werden können, die nicht mit Hilfe der HPLC getrennt werden können. Nach Waschen mit Wasser sowie etwas Ethanol und Acetonitril wurde 0.5 vol.% Hydrazinhydrat in Wasser zu den peptidbeladenen Sepharosebeads gegeben und nach 30 Minuten der Überstand analysiert, wobei das Chromatogramm 3 (Fig. 3) erhalten wurde. Dieses zeigt eine hohe Reinheit des Peptids (gewünschtes Produkt) von über 90%.

### Beispiel 3: Exemplarische Reinigung einer Peptidmischung nach der Festphasenpeptidsynthese

Das Reaktionsschema (Schema 5) der erfindungsgemäßen Reinigung ist im Folgenden dargestellt.

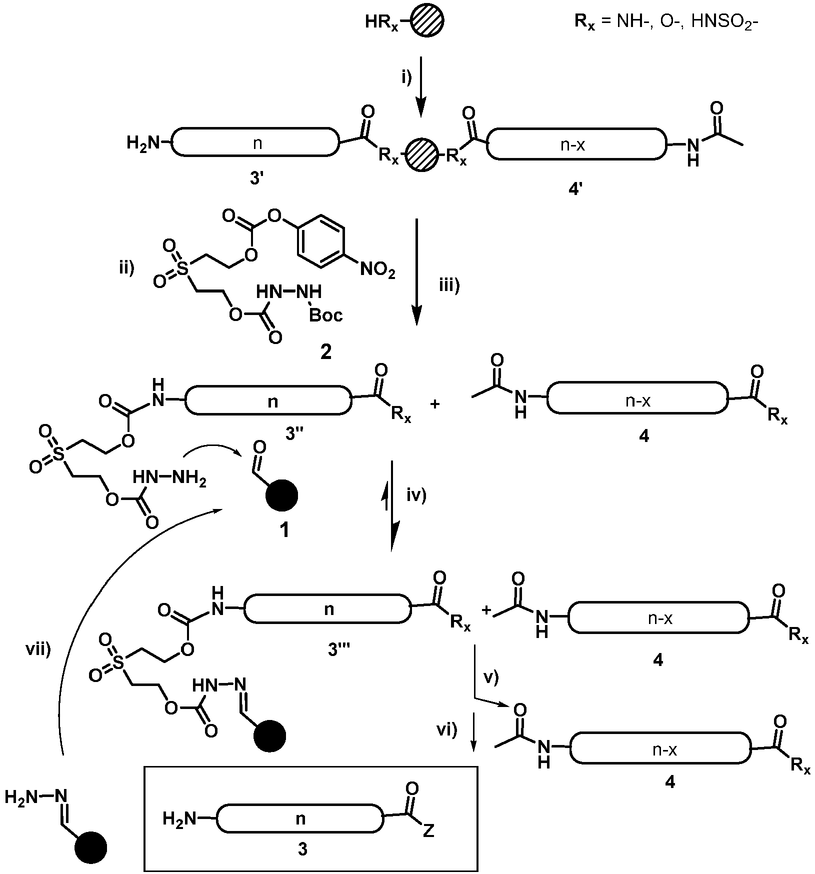

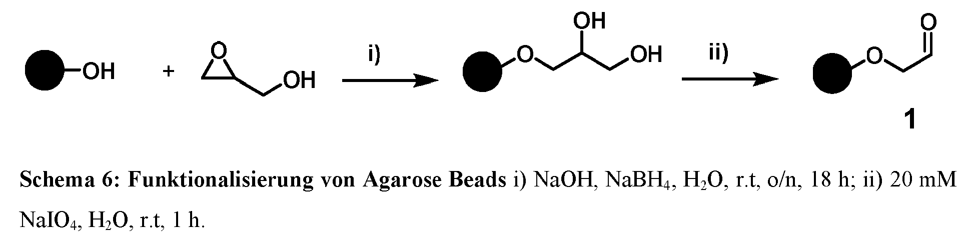

Nach der Festphasensynthese (SPPS) wird ein Fängermolekül 2 (Schema 5) auf die letzte gekuppelte Aminosäure aufgebracht. Wichtige Voraussetzung hierbei ist, dass die Acetylierung in den vorherigen Schritten vollständig verlief. Danach werden alle Abbruchsequenzen und das Fängermolekülmodifizierte Volllängenpeptid vom Harz gespalten. Anschließend werden nicht-peptidische Verunreinigungen durch Etherfällung entfernt und die rohe Peptidmischung wird in einem Acetat Puffer gelöst, der einen pH-Wert von 3-4 hat und dem 0.1 M Anilin als Katalysator beigefügt ist. Diese Lösung wird nun auf funktionalisierte Sepharose-Beads 1 gegeben (Schema 5 und 6). Dieses Material findet Anwendung in der Proteinreinigung als Material für die Affinitätschromatographie. Die Sepharose hat den Vorteil, leicht für die Peptide penetrierbar zu sein. Die Sepharose wird vorher Aldehyd-modifiziert (siehe Schema oben), die Aldehydmodifikation der Sepharose ist bekannt aus: J. Guisan, Enzyme Microb. Technol. 1988, 10, S.375.

Hierbei werden nur Peptide auf dem festen Träger verankert, die das Fängermolekül mit der Hydrazidfunktion tragen. Amine, die theoretisch auch mit Aldehyden reagieren können, sind bei dem zu verwendenden pH-Wert protoniert und somit nicht nukleophil genug für einen Angriff am Aldehyd. Die Abbruchsequenzen, die sich noch in der Sepharose befinden, können mit Wasser herausgewaschen werden. Eine Behandlung der Sepharose mit einer basischen Lösung, z.B. Ammoniak in Wasser, lässt das Fängermolekül zerfallen und das Volllängenpeptid geht in Lösung über. Die Lösung kann danach lyophilisiert werden, wobei Ammoniak entfernt wird. Das Peptid wird dann in reiner Form als Feststoff erhalten. Ein Vorteil der Methode ist die schnelle Immobilisierung und die breite Anwendbarkeit bei unterschiedlichen Peptiden.

### Beispiel 4: Exemplarische Regenerierung des Reinigungsharzes

Nach der Peptidreinigung mit dem Fängermolekül 2 (Schema 5) bleibt die ursprüngliche Aldehydfunktion von 1 mit Hydrazin blockiert zurück, um das Reinigungsharz wieder für einen neuen Reinigungszyklus bereit zu stellen, muss das Harz regeneriert werden und somit die Aldehydfunktion wiederhergestellt werden. Dies gelingt bei Verschieben des Gleichgewichtes durch Zugabe von Aldehyden oder Ketonen. Die exemplarische Durchführbarkeit wurde wie folgt gezeigt. Es wurde das Reinigungsharz 1 in drei gleiche Aliquote geteilt (I, II, III) und zwei Aliquote (II, III) wurden mit Hydrazin im Konjugationspuffer für 30 min behandelt. Anschließend wurde mit Wasser gewaschen und Aliquot III wurde mit einer Mischung aus Wasser, Aceton und TFA (49.5:49.5:1) siebenmal gewaschen. Danach wurden alle Aliquote mit FmocN₂H₃ in Konjugationspuffer über 14 h behandelt und dann wurde mit jeweils fünfmal mit Wasser, DMF und Wasser gewaschen. Es folgte eine zweimalige Behandlung mit DMF/Piperidin(20%) je 4 min und dann wurde die UV Absorption bei 301 nm des entstandenen Fluoren Piperidin Addukts im Überstand gemessen (SmartSpec^{™}Plus Spectrophotometer der Firma BioRad in 1 mL Halbmikro-Quarzküvetten der Firma Hellma). Das unbehandelte Aliquot I zeigte eine Beladung von 27 µmol/g; das Aliquot II ohne Regeneration eine Beladung von 18 µmol/g (67% von I), und das regenerierte Harz zeigte eine Beladungsdichte von 25 µmol/g (93% von I). Dieses Experiment zeigt die erfolgreiche Regeneration des Reinigungsharzes.

**Tabelle 1: UV-Absorption und Beladungsdichte von Reinigungsharzen nach unterschiedlichen Regenerationsbehandlungen**

| **Aliquot** | **A(301nm)** | **S(µmol/g)** | **Anteil [%]** |
|---|---|---|---|
| I | 0,286 | 27 | 100 |
| II | 0,199 | 18 | 67 |
| III | 0,267 | 25 | 93 |

### Beispiel 5: Reinigung einer Peptidmischung

Das erfindungsgemäße Verfahren zur Reinigung von Peptiden wurde auf sieben Peptide unterschiedlicher Polarität angewendet, diese waren H-TLADEVSASLAK-OH (SEQ ID NO: 3) (7) Fragment 427-438 des Alzheimer relevanten Tau-Proteines, H-ATLADEVSASLAK-NH₂ (SEQ ID NO: 4) (8) Fragment 427-439 von Tau, das Cysteinylpeptid H-CQWSLHRKRHLARTLLTAAREPRPAPPSSNKV-NH₂ (SEQ ID NO: 5) (8) aus Protein Progonadoliberin-2 (9), H-GIGKFLHSAKKFGKAFVGEIMNS-NH₂ (SEQ ID NO: 6) Magainin (10), H-YLFFYRKSV-NH₂ Terts72Y (SEQ ID NO: 7) (11), H-FPRPGGGGNGDFEEIPEEYL-NH₂ (SEQ ID NO: 8) Bivalirudin (**12**) und H-GRKKRRQRRRPQ-NH₂ TAT (SEQ ID NO: 9) (**13**).

Es wurde die Rohpeptidmischung im Konjugationspuffer gelöst und auf die Sepharose-Beads **1** innerhalb von 30-60 min aufgebracht, anschließendes Waschen mit Wasser und wässrigen neutralen Lösungen (4M Harnstoff, 1M Kochsalz) konnten alle acetylierten Abbruchsequenzen und andere Verunreinigungen entfernt werden. Die Spaltung des Linkers **2** (Schema 7) erfolgte mit 5%iger Ammoniak Lösung in Wasser über 20min, anschließend wurde *in-situ* mit Essigsäure neutralisiert und die Löslichkeit erhöht.

Mit Hilfe der UPLC-MS wurde die Reinheit der einzelnen Phasen überprüft. Die Chromatogramme der nicht gereinigten (ohne Fängermolekül FM) und gereinigten Peptide, sowie des die Verunreinigungen enthaltenden Überstandes sind in Figur 4 zu sehen. Es konnte eine Peptidreinheit bei 7 von 85% (ursprünglich 39%), bei 8 von 93% (ursprünglich 39%), bei 9 von 80% (ursprünglich 24%), bei 10 von 90% (ursprünglich 23%), bei 11 von 87% (ursprünglich 60%), bei 12 von 90% (ursprünglich 40%), bei 13 von 95% (ursprünglich 37%) erreicht werden (vgl. Abb. 4).

**Tabelle 2: Reinheit und Ausbeute verschiedener Peptide nach Anwendung des erfindungsgemäßen Reinigungsverfahrens**

| **Name** | **Nr.** | **Anwendung** | **Status** | **Länge in AS** | **Hydrophobe AS** | **Reinheit Roh-produkt** | **Reinheit nach Verfahren** | **Ausbeute** |
|---|---|---|---|---|---|---|---|---|
| **Tau1** | 7 | Alzheimer | präklinisch | 13 | 62 % | 40 % | 85 % | 70 % |
| **Tau2** | 8 | Alzheimer | präklinisch | 12 | 58% | 39% | 93% | 69 % |
| **GNRH** | 9 | Fertilisation | zugelassen | 32 | 59 % | 35 % | 80% | 42% |
| **Magainine** | 10 | Antibiotikum | zugelassen | 23 | 65 % | 23 % | 90 % | 62 % |
| **Terts72Y** | 11 | Lungenkrebs | Phase II | 9 | 77 % | 60% | 87% | 92% |
| **Bivalrudin** | 12 | Anti-koagulation | zugelassen | 20 | 65 % | 40% | 90 % | 89% |
| **TAT** | 13 | HIV | Phase II | 12 | 17% | 37% | 95% | 83% |
| **Testpeptid** | 14 | Forschung | - | 13 | 46 % | 45 % | 60 % | - |

Die Synthese des basenlabilen Linkers 2 erfolgte nach folgendem Schema 7.

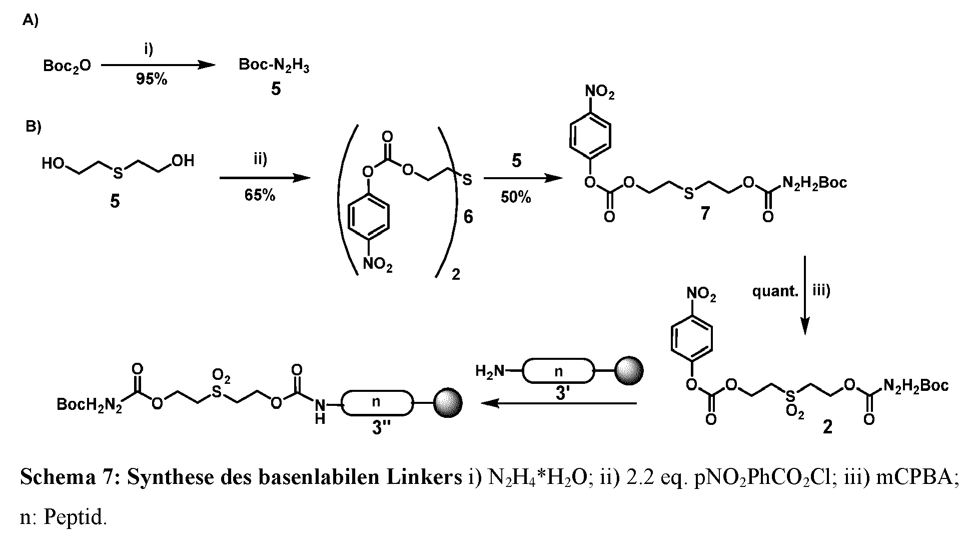

### Material und Methoden

### Festphasenpeptidsynthese der Peptide sowie Reinigung

Die automatisierte Festphasenpeptidsynthese erfolgte in 25 µmol Ansätzen, mit einem MultiPep RS-Peptidsyntheseautomaten der Firma Intavis AG. Die Synthesen der Peptidamide erfolgten am Tentagel R RAM Harz (0.2 mmol · g -1) der Firma Rapp Polymer. Vor Beginn der Synthese wurde das Harz in 3 mL Spritzenreaktoren (PE-Reaktor der Firma Multisyntech) überführt und in DMF gequollen. Soweit nicht anders beschrieben, bezieht sich die Angabe der Äquivalente auf die Erstbeladung des eingesetzten Harzes.

*Fmoc-Abspaltung:* Zur Abspaltung der temporären Fmoc-Schutzgruppen wurde das Harz einmal 4 min und ein weiteres Mal 6 min mit 400 µL Piperidin/DMF (4:1) behandelt und nachfolgend fünfmal mit 800 µL DMF gewaschen und es wurde mit der Kupplung der Fmoc-Aminosäurederivate fortgefahren.

*Kupplung der Fmoc-Aminosäurederivate:* Eine Lösung von 5 eq. Aminosäure in DMF (0.3 M) wurde 1 min bei Raumtemperatur mit Lösungen von 4.5 eq. HCTU in DMF (0.3 M) und 10 eq. NMM in DMF (0.6M) voraktiviert und anschließend zum Harz gegeben. Nach 30 min Reaktionszeit wurde das Harz dreimal mit 800 µL DMF gewaschen und es wurde mit dem Blockieren der Abbruchsequenzen fortgefahren.

*Blockieren der Abbruchsequenzen:* Das Harz wurde einmal 5 min mit 400 µl einer Lösung von AC₂O/2,6-Lutidin/DMF (5:6:89) behandelt und nachfolgend dreimal mit je 800 µL DMF gewaschen.

*Letzter Schritt Kopplung des Fängermoleküls:* Als letzter Schritt der Festphasenpeptidsynthese wurde das Fängermolekül 2 auf das gewünschte Zielpeptid gekuppelt. Es wurde das Syntheseharz mit einer Lösung aus 5 eq. Fängermolekül 2 in DMF (0.3 M) und 5 eq. Oxyma in DMF (0,3 M) und 12 eq. DIPEA in DMF (0,7 M) gemischt. Nach 60 min Reaktionszeit wurde zweimal mit je 800 µL DMF gewaschen.

*Freisetzung vom polymeren Träger:* Das Harz wurde mit 2 mL einer Lösung von 96% TFA, 2% Wasser, 2% Triisopropylsilan, im Falle von thiolhaltigen Aminosäuren (Cystein oder Methionin) in der Zielsequenz wurde der Lösung 0,5% Ethandithiol und 0,5% Thioanisol zugesetzt wobei die Menge an TFA um 1 % reduziert wurde. Das Syntheseharz wurde mit dieser Abspaltmischung versetzt und 2 h bei Raumtemperatur geschüttelt. Anschließend wurde die Abspaltlösung aufgefangen und das Harz zweimal mit je 1 mL TFA nachgewaschen. Die Abspaltlösung wurde mit den Waschlösungen vereinigt und in 50 ml kaltem Diethylether ausgefällt. Diese Suspension wurde anschließend zentrifugiert und der organische Überstand wurde verworfen.

*Immobilisierung auf Reinigungsharz:* Der krude Niederschlag wurde nach der Zentrifugation in 3 ml des Konjugationspuffer (0.1 M NH₄OAc, 0,1 M Anilin, pH = 3) aufgenommen, wenn sich die Mischung nicht vollständig löste wurde Acetonitril dazugegeben. Diese Lösung wurde in eine 6 ml Spritze der Firma bBraun mit einem Filtereinsatz PE 25 µm Porengröße der Firma Multisyntech gegeben, in dieser Spritze befand sich ein gram funktionalisierte Sepharose. Die Immobilisierung wurde 30 bis 60 min durchgeführt. Anschließend wurde 5-mal mit deionisiertem Wasser der Reinheit MilliQ gewaschen, 5-mal mit einer 4M Harnstoff Lösung und 5-mal mit Wasser. Anschließend wurde das gewünschte Peptid basisch mit 5 v% NH₄OH und 1 v% Mercaptoethanol in Wasser vom Harz gespalten. Lyophilisieren lieferte die gewünschten Peptide als weißer flockiger Feststoff.

### Synthese des Fängermoleküls 2 (Verbindung 2 in Schema 7 - entspricht Verbindung (25)):

### tert-Butylhydrazincarboxylat 5 (Verbindung 5 in Schema 7)

Hydrazinmonohydrat (80%, 32,5 g, 520 mmol) wurde mit Isopropanol (100 ml) bei 0 °C, mit einer Lösung aus Boc₂O (50,0 g, 230 mmol) in Isopropanol (50 ml) tropfenweise versetzt. Das Reaktionsgemisch wurde trüb nach der Zugabe und das Rühren wurde bei Raumtemperatur für 2 h fortgesetzt. Das Lösungsmittel wurde entfernt, der Rückstand in Dichlormethan gelöst und über Magnesiumsulfat getrocknet. Dann wurde das Lösungsmittel verdampft und der Rückstand wurde aus Hexan umkristallisiert, was die Titelverbindung 5 (22,8 g, 75%) als farblose Kristalle ergab. Smp 36-37°C Rf (EtOAc / Hexan 1:1) 0,20. ¹H- NMR (300 MHz, CDCl₃): δ 6,16 (s, 1H, NH), 3,67 (s, 2H, NH₂), 1,42 (s, 9H, C(CH₃)₃). ¹³C-NMR (75 MHz, CDCl₃, TMS): δ 158,3, 77,2,28,5. Die analytischen Daten stimmen mit den Literaturdaten überein (A. Bredihhin, U. Mäeorg, Tetrahedron 2008, 64, 6788-6793).

### Bis(4-Nitrophenyl)(thiobis(ethan-2,1-diyl))bis(carbonat) 6 (Verbindung 6 in Schema 7)

6,72 (33 mmol) 4-Nitrophenylchlorformiat wurde zu einer Lösung von 1,87 ml (2,12 g, 15 mmol) 2,2-Thiodiethanol in 40 ml wasserfreiem Dichlormethan gegeben. Dann wurde 2,68 ml (33 mmol) wasserfreies Pyridin langsam tropfenweise unter Eiskühlung und kräftigem Rühren zugegeben. Die Reaktionsmischung wurde für 1 h bei Raumtemperatur gerührt. Die Reaktionslösung wurde mit 100 ml gesättigter Ammoniumchloridlösung versetzt und dreimal mit 100 ml Chloroform extrahiert und über wasserfreiem Magnesiumsulfat getrocknet. Die organischen Phasen wurden vereinigt und im Vakuum eingeengt. Der Rückstand wurde in Ethylacetat aufgenommen und mit wenig Cyclohexan wurde das Produkt ausgefällt. Nach dem Filtrieren wurden 5,43g (12 mmol, 80%) als weißer Feststoff erhalten. Schmelzpunkt: 136,5 °C, Rf (EtOAc / Cyclohexan 1:1) 0,78. ¹H NMR (300 MHz, DMSO) δ 8.30 (d, *J=* 9,2 Hz, 2H, Ar-H), 7,55 (d, *J*= 9,3 Hz, 2H, Ar-H), 4,42 (t, *J*= 6,4 Hz, 2H, CH₂), 2,96 (t, *J*= 6,5 Hz, 2H, CH₂). ¹³C-NMR (75 MHz, CDCl₃, TMS): δ 155,22, 151,93, 145,17, 125,41, 122,56, 67,85, 29,63.

### 2-[2-(1-((tert-Butyl)oxy-carbonyl)oxy-carbonyl)-hydrazyl-ethylsulfanyl]-ethyl 4-nitrophenylcarbonat 7 (Verbindung 7 in Schema 7)

Es wurden 1,97 g (4,31 mmol) bis(4-nitrophenyl) (thiobis(Ethan-2,1-diyl))bis(carbonat) 6 in 20 ml trockenem Dichlormethan vorgelegt und bei 0 °C wurden 1 eq. (0,58 g, 4,31 mmol) tert-butyl hydrazincarboxylat 5 mit 3 eq. (1,13 ml, 6,66 mmol) DIPEA langsam über eine Stunde hinzugetropft. Die Reaktionslösung wurde 12 weitere Stunden gerührt und anschließend mit Wasser versetzt. Das Produkt wurde dreimal mit 100 ml Dichlormethan extrahiert und über wasserfreiem Magnesiumsulfat getrocknet. Die organischen Phasen wurden vereinigt und im Vakuum eingeengt. Der Rückstand wurde säulenchromatographisch gereinigt (EtOAc / Cyclohexan 2:1), wonach 1,03 g (2,31 mmol, 53%) eines durchsichtigen Öls erhalten wurde. Rf (EtOAc / CyclOhexan 1:1) 0,20. ¹H NMR (300 MHz, CDCh) δ 8,28 (d, *J=* 9,1 Hz, 2H, Ar-H), 7,39 (d, *J=* 9,1 Hz, 1H, Ar-H), 6,64 (s, 1H, NH), 6,33 (s, 1H, NH), 4,44 (t, *J=* 6,8 Hz, 2H, CH₂), 4,33 (t, *J=* 6,6 Hz, 2H, CH₂), 2,92 (t, *J=* 6,8 Hz, 2H, CH₂), 2,84 (t, *J =* 6,6 Hz, 2H, CH₂), 1,46 (s, 9H). ¹³C NMR (75 MHz, CDCl₃) δ 155,52, 152,57, 125,47, 121,96, 82,09, 68,06, 65,34, 64,18, 31,10 ,30,59, 28,27, 27,03.

### 2-[2-(1-((tert-Butyl)oxy-carbonyl)oxy-carbonyl)-hydrazyl-ethylsulfonyl] -ethyl4-nitrophenylcarbonat 2 (Verbindung 2 in Schema 7)

Zu einer Lösung des Thioethers 7 (0,52 g, 1,1 mmol) in 50 ml Dichlormethan wurde langsam bei Raumtemperatur m-CPBA 77%ig (489 g, 2,2 mmol) zugegeben. Nach Rühren für 12 h wurde die Reaktionsmischung mit 1M NaHCO₃ Lösung versetzt und die organische Phase dreimal mit 50 ml Dichlormethan extrahiert. Die vereinten organischen Phasen wurden über Magnesiumsulfat getrocknet und am Rotationsverdampfer wurde das Lösungsmittel entfernt worauf das Produkt als weißer amorpher Feststoff ausfiel 0,52mg (1,1 mmol, quantitativ). ¹H NMR (300 MHz, CDCl₃) δ 8,29 (d, *J*= 9,2 Hz, 2H, Ar-H), 7,41 (d, *J=* 9,2 Hz, 2H, Ar-H), 6,89 (s, 1H, NH), 6,36 (s, 1H, NH), 4,74 (t, *J* = 5,9 Hz, 2H, CH₂), 4,63 (t, *J* = 5,3 Hz, 2H, CH₂), 3,54 (t, *J* = 5,9 Hz, 2H, CH₂), 3,45 (t, *J* = 5,5 Hz, 2H, CH₂), 1,45 (s, 9H). ¹³C NMR (75 MHz, CDCl₃) δ 155,85, 155,18, 152.29, 145,79, 125,56, 122,00, 82,32, 62,25, 59,45, 54,01, 53,24, 28,23.

Die Synthese der Verbindungen nach Formel (14), (15), (17), (18), (19): erfolgte nach einem Baukastenprinzip, das im allgemeinen Syntheseschema (Schema 1) dargestellt ist.

### Synthesen zur Darstellung der Verbindung der Formel (14)

### N,N'-Bis-(tert-Butoxycarbonyl)-aminooxyacetyl-N-hydroxylsuccinimid Ester (BS2: X = O, R₁ = Boc)

Zu einer Lösung des kommerziell erhältlichen *N*,*N*'-bis-Boc-amino-oxyessigsäure (1.00 g, 3.20 mmol) in 11 ml Ethylacetat/Dioxan (1:1) wurde bei 0 °C N-Hydroxylsuccinimid (0.41 g, 3.20 mmol) und Dicyclohexylcarbodiimid (0.67 g, 0.32 mmol) hinzugegeben. Bei Raumtemperatur wurde die Lösung für 3 Stunden rühren gelassen und die Suspension wurde über Celite abfiltriert und mit Ethylacetat nachgewaschen. Das Filtrat wurde unter Vakuum zur Trockne konzentriert und wieder in 100 ml Ethylacetat gelöst. Es wurde mit 5%iger NaHCO₃-Lösung, gesättigter NaCl-Lösung und Wasser (je 100 ml) gewaschen. Die organische Phase wurde mit MgSO₄ getrocknet und unter Vakuum verdampft wobei 1.24 g (3.20 mmol) Produkt als weißer Feststoff erhalten wurde. Ausbeute: 1.24 g (quant.); Rf (Cyclohexan / Ethylacetat, 1:1) 0.50; ¹H NMR (300 MHz, CDCl₃) δ 4.86 (s, 2H), 2.85 (s, 4H), 1.53 (s, 18H).

### N,N'-Bis-(tert-Butoxycarbonyl)-aminooxyacetyl-1-((2-aminoethyl)thio)propan-2-ol Amid (BS3: X = O, R₁ = Boc)

BS2 (X = O, R₁ = Boc, 1.00 g, 2.55 mmol) wurde in 25 ml Dichlormethan mit 1-((2-aminoethyl)thio)propan-2-ol (0.38 g, 2.55 mmol) und Diisopropyl-ethylamin (DIPEA, 0.53 ml, 3.06 mmol) gemischt und über Nacht rühren gelassen. Es wurde mit 5%iger NaHCO₃-Lösung, gesättigter NaCl-Lösung und Wasser (je 100 ml) gewaschen. Die organische Phase wurde mit MgSO₄ getrocknet und unter Vakuum eingeengt wobei 1.04 g (2.55 mmol) Produkt als weißer Feststoff erhalten wurde. Ausbeute: 1.04 g (quant.); Rf (CH₂Cl₂ / MeOH, 98:2) 0.35; ¹H NMR (300 MHz, CDCl₃) δ 7.95 (s, 1H), 4.44 (s, 2H), 3.92 - 3.82 (m, 1H), 3.53 (qd, *J* = 6.7, 1.5 Hz, 2H), 2.83 - 2.65 (m, 4H), 2.49 (dd, *J* = 13.7, 8.7 Hz, 1H), 1.55 (s, 18H), 1.25 (d, *J* = 6.2 Hz, 3H); ¹³C NMR (75 MHz, CDCl₃) δ 167.94, 150.57, 85.41, 65.98, 41.86, 38.81, 32.21, 28.19, 22.23.

### 2,2-Dimethylpropanoyloxy-[2-[2-[2-(4-nitrophenoxy)carbonyloxypropylthionyl]ethylamino]-2-oxo-ethoxy]amino] 2,2-dimethylpropanoat P' (X = O, R₁ = Boc, R₂ = OC₆H₄pNO₂)

Bis(4-nitrophenyl)carbonat (1.01 g, 4.95 mmol) wurden zu einer Lösung von BS3 (X = O, R₁ = Boc) (1.52 g, 3.30 mmol) in 5 ml trockenem CH₂Cl₂ gegeben. Dann wurde trockenes Pyridin (0.40 ml, 4.95 mmol) unter Eiskühlung hinzugetropft. Die Reaktionslösung wurde 18 Stunden gerührt. Der Niederschlag wurde abfiltriert und es wurde mit 50 ml DCM nachgewaschen. Das Filtrat wurde mit gesättigter NH₄Cl-Lösung (50 ml) gewaschen und die wässrige Phase wurde mit 50 ml CHCl₃ extrahiert. Nach dem Trockenen mit MgSO4 wurden die vereinigten organischen Phasen am Rotationsverdampfer eingeengt und der Rückstand wurde mittels Säulenchromatographie (Cyclohexan/EtOAc, 2:1) gereinigt. Ausbeute: 1.27 g (67%); Rf (Cyclohexan/EtOAc, 1:1) 0.44; ¹H NMR (300 MHz, CDCl₃) δ 8.27 (d, *J* = 9.2 Hz, 2H), 7.89 (s, 1H), 7.39 (d, *J* = 9.2 Hz, 2H), 5.00 (dd, *J* = 12.5, 6.3 Hz, 1H), 4.43 (s, 2H), 3.53 (dd, *J* = 13.3, 6.5 Hz, 1H), 2.86 - 2.69 (m, 2H), 1.53 (s, 18H), 1.47 (d, *J* = 6.3 Hz, 3H).

### 2,2-Dimethylpropanoyloxy-[2-[2-[2-(4-nitrophenoxy)carbonyloxypropylsulfonyl]ethylamino]-2-oxo-ethoxy]amino] 2,2-dimethylpropanoate Formel (14)

Zu einer Lösung von *P' (X* = *O, R₁* = *Boc, R₂* = *OC₆H₄pNO₂)* (1.27 g, 2.15 mmol) in 21 ml Dichlormethan wurde langsam bei Raumtemperatur m-CPBA (0.96 g, 4.30 mmol) gegeben. Nach 12-stündigem Rühren wurde das Reaktionsgemisch zweimal mit einer gesättigten NaHCO₃-Lösung (15 ml) gewaschen und die organischen Phase nach dem Trocknen mit MgSO₄ im Vakuum konzentriert. Das Sulfon wurde als weißer Feststoff erhalten. Ausbeute: 1.26 g (97%); Rf (Cyclohexan/EtOAc, 1:1) 0.29; ¹H NMR (300 MHz, CDCl₃) δ 8.28 (d, *J* = 9.3 Hz, 1H), 8.15 (t, *J* = 5.8 Hz, 1H), 7.41 (d, *J* = 9.3 Hz, 1H), 5.48 - 5.39 (m, 1H), 4.44 (s, 1H), 3.83 (dd, *J* = 6.2, 4.2 Hz, 1H), 3.56 (dd, *J* = 14.9, 8.3 Hz, 1H), 3.37 (t, *J* = 6.5 Hz, 1H), 3.27 (dd, *J* = 14.9, 3.9 Hz, 1H), 1.57 (d, *J* = 6.4 Hz, 1H), 1.54 (s, 7H). ¹³C NMR (75 MHz, CDCl₃) δ 168.58, 155.42, 151.58, 150.52, 145.67, 125.46, 122.04, 85.60, 77.16, 76.62, 70.63, 58.19, 53.67, 33.07, 28.16, 20.21; ESI-MS: (berechnet MNa+: 628.16 g/mol, gefunden: 628.17 m/z).

### Synthesen zur Darstellung der Verbindung der Formel (18)

### ((tert-Butoxycarbonyl)amino)glycin (BS1 X = NH, R₁ = H)

Zu einer methanolischen Lösung (10 ml) von NaOH (0.70 g, 17.4 mmol) und Boc-Hydrazin (1.17 g, 8.7 mmol) wurde bei 0°C Bromessigsäure (1.48 g, 10.4 mmol) gegeben. Die Lösung wurde für 5 Stunden unter Rückfluss erhitzt. Anschließend wurde MeOH entfernt und 50 ml Wasser hinzugegeben. Die wässrige Phase wurde dreimal mit Ethylacetat (50 ml) extrahiert. Anschließend wurde die wässrige Phase mit Zitronensäure auf pH 2 gebracht und dreimal mit 50 ml Ethylacetat extrahiert. Die vereinigten organischen Phasen wurden mit MgSO₄ getrocknet und das Lösungsmittel wurde unter verminderten Druck entfernt. Ausbeute: 0.85 g (51%) weißer Feststoff; Rf (CH₂Cl₂ / MeOH, 8:2) 0.15. ¹H NMR (300 MHz, DMSO) δ 8.55 (s, 2H), 8.17 (s, 2H), 3.40 (s, 2H), 1.37 (s, 9H); ESI-MS: (berechnet MH+: 191.10 g/mol, gefunden: 191.33 m/z).

### N-(tert-Butoxycarbonyl)-N-((tert-butoxycarbonyl)amino)glycin (BS1 X = NBoc, R₁ = H)

Zu einer Lösung aus ((tert-Butoxycarbonyl)amino)glycin (5.00 g, 26.0 mmol) und NaOH (1.57 g, 39.04 mmol) in 104 ml Dioxan/H₂O (1:1) wurde Boc₂O (5.74 g, 26.03 mmol) als Feststoff hinzugegeben. Die Lösung wurde 18 h über Nacht bei Raumtemperatur gerührt und anschließend wurde das Dioxan unter verminderten Druck entfernt. Der wässrige Rückstand wurde mit 100 ml gesättigter NaHCO3 Lösung versetzt und es wurde zweimal mit 100 ml Et₂O gewaschen. Die wässrige Phase wurde mit Zitronensäure auf pH 2 gebracht. Die weiße Suspension wurde dreimal mit 150 ml Ethylacetat extrahiert. Nach dem Trocknen mit MgSO₄ wurde das Lösungsmittel entfernt wobei sich ein weißer Feststoff bildete. Ausbeute: 7.56 g (quant.); Rf (CH₂Cl₂ / MeOH, 9:1) 0.75; ¹H NMR (300 MHz, DMSO) δ 12.34 (s, 1H), 9.24 (s, 1H), 3.56 (s, 2H), 1.46 - 1.32 (m, 18H).

### N-(tert-Butoxycarbonyl)-N-((tert-butoxycarbonyl)amino)glycinyl-N-hydroxylsuccinimid (BS2 X = NBoc, R₁ = H)

Zu einer Lösung des *N-(tert-Butoxycarbonyl)-N-((tert-butoxycarbonyl)amino)glycins* (1.36 g, 4.45 mmol) in 15 ml Ethylacetat/Dioxan (1:1) wurde bei 0 °C N-Hydroxylsuccinimid (0.52 g, 4.45 mmol) und Dicyclohexylcarbodiimid (DCC, 0.93 g, 4.45 mmol) hinzugegeben. Bei Raumtemperatur wurde die Lösung für 15 Stunden rühren gelassen. Anschließend wurde die Suspension wurde über Celite abfiltriert und mit Ethylacetat nachgewaschen. Das Filtrat wurde unter Vakuum zur Trockne konzentriert und wieder in 100 ml Ethylacetat gelöst. Es wurde mit 5%iger NaHCO₃-Lösung, gesättigter NaCl-Lösung und Wasser (je 100 ml) gewaschen. Die Organische Phase wurde mit MgSO₄ getrocknet und unter Vakuum verdampft wobei 1.24 g (3.20 mmol) Produkt als weißer Schaum erhalten wurde. Ausbeute: 1.51 g (88%); Rf (Cyclohexan / Ethylacetat, 1:1) 0.45; ¹H NMR (300 MHz, CDCl₃) δ 4.67 (s, 1H), 4.19 (s, 2H), 2.87 (s, 4H), 1.49 (m, 18H).

### N-(tert-Butoxycarbonyl)-N-((tert-butoxycarbonyl)amino)glycinyl-1-((2-aminoethyl)thio)propan-2-ol Amid (BS3: X = NBoc, R₁ = H)

BS2 (*X* = *NBoc, R₁* = *H,* 0.36 g, 0.92 mmol) wurde in 10 ml Dichlormethan mit 1-((2-aminoethyl)thio)propan-2-ol (0.13 g, 0.92 mmol) und DIPEA (0.18 ml, 1.01 mmol) gemischt und über Nacht rühren gelassen. Es wurde mit 5%iger NaHCO₃-Lösung, gesättigter NaCl-Lösung und Wasser (je 100 ml) gewaschen. Die organische Phase wurde mit MgSO₄ getrocknet und unter Vakuum eingeengt wobei 0.27 g (0.65 mmol) Produkt als weißer Schaum erhalten wurde. Ausbeute: 0.27 g (quant.); Rf (CH₂Cl₂ / MeOH, 98:2) 0.36; ¹H NMR (300 MHz, CDCl₃) δ 8.34 (s, 1H), 6.66 (s, 1H), 4.05 (s, 2H), 3.90 - 3.81 (m, 1H), 3.49 (dd, *J* = 10.4, 4.0 Hz, 2H), 2.78 - 2.63 (m, 3H), 2.47 (dd, *J* = 13.7, 8.7 Hz, 1H), 1.49 (s, 9H), 1.46 (s, 9H), 1.23 (d, *J* = 6.2 Hz, 3H).

### [2-(2,2-Dimethylpropanoyloxy)-2-[2-[2-[2-(2,5-dioxopyrrolidin-1-yl)oxycarbonyloxypropylthionyl] ethylamino]-2-oxo-ethyl]hydrazino] 2,2-dimethylpropanoate P' (X = O, R₁ = Boc, R₂ = ONO₂C₂H₄)

N,N-Disuccinimidyl carbonat (0.19 g, 0.72 mmol) wurden zu einer Lösung von BS3 (X = NBoc, R₁ = H) (0.25 g, 0.72 mmol) in 5 ml trockenem CH₂Cl₂ gegeben. Dann wurde trockenes Pyridin (0.06 ml, 0.73 mmol) unter Eiskühlung hinzugetropft. Die Reaktionslösung wurde 17 Stunden gerührt. Der Lösung wurde 50 ml DCM hinzugefügt. Die organische Phase wurde mit 10%iger Zitronensäure-Lösung gewaschen und mit MgSO₄ getrocknet. Die vereinigten organischen Phasen wurden am Rotationsverdampfer eingeengt und der Rückstand wurde mittels Säulenchromatographie (CH₂Cl₂/MeOH, 19:1) gereinigt. Ausbeute: 1.27 g (67%); Rf (CH₂Cl₂/MeOH, 9:1) 0.60; ¹H NMR (300 MHz, CDCl₃) δ 8.53 (d, *J* = 95.5 Hz, 1H), 7.07 (s, *J* = 9.0 Hz, 1H), 4.01 (s, 2H), 3.82 (ddd, *J* = 8.2, 6.1, 3.9 Hz, 1H), 3.41 (d, *J* = 6.1 Hz, 4H), 2.67 (dt, *J* = 13.2, 9.3 Hz, 4H), 2.46 (dd, *J* = 13.7, 8.2 Hz, 2H), 1.44 (s, 9H), 1.41 (s, 9H), 1.18 (t, *J* = 6.8 Hz, 3H).¹³C NMR (75 MHz, CDCl₃) δ 169.71, 162.47, 154.43, 77.16, 66.04, 50.53, 41.57, 39.13, 32.22, 28.18, 28.12, 25.57, 22.06, 18.88.

### [2-(2,2-Dimethylpropanoyloxy)-2-[2-[2-[2-(2,5-dioxopyrrolidin-1-yl)oxycarbonyloxypropylsulfonyl] ethylamino]-2-oxo-ethyl]hydrazino] 2,2-dimethylpropanoate (Formel (18)),

Zu einer Lösung von *P' (X* = *NBoc, R₁* = *H, R₂* = *ONO₂C₂H₄)* (0.10 g, 0.18 mmol) in 5 ml Dichlormethan wurde langsam bei Raumtemperatur m-CPBA (0.81 g, 0.36 mmol) gegeben. Nach 14-stündigem Rühren wurde das Reaktionsgemisch dreimal mit 5 % NaHCO₃ in einer gesättigten NaCl-Lösung (je 33 ml) gewaschen und die organische Phase danach mit MgSO₄ getrocknet. Das Lösungsmittel wurde im Vakuum entfernt und das Sulfon wurde als weißer amorpher Feststoff erhalten. Ausbeute: 0.08 g (76%); Rf (CH₂Cl₂/MeOH, 9:1) 0.45 ¹H NMR (300 MHz, CDCl₃) δ 8.48 (s, 1H), 6.95 (s, 1H), 4.39 (dt, *J* = 15.7, 7.8 Hz, 1H), 4.06 (s, 2H), 3.73 (d, *J* = 4.3 Hz, 2H), 3.48 - 3.17 (m, 4H), 3.41 (d, J= 6.1 Hz, 2H), 3.02 (d, *J* = 13.2 Hz, 1H), 2.85 - 2.68 (m, 3H), 1.46 (s, 9H), 1.43 (s, 9H), 1.30 (d, *J* = 6.4 Hz, 3H); ¹³C NMR (75 MHz, CDCl₃) δ 170.18, 167.99, 154.71, 133.30, 131.86, 130.13, 129.83, 128.20, 77.16, 62.88, 53.37, 33.53, 28.23, 28.16, 25.57, 23.25. ESI-MS: (berechnet MNa⁺: 603.19 g/mol, gefunden: 603.06 m/z).

### Synthesen zur Darstellung der Verbindung der Formel (15)

### N-(tert-Butoxycarbonyl)-aminooxyacetyl-1-((2-aminoethyl)thio)propan-2-ol Amid (BS3: X = O, R₁ = H)

Kommerziell erhältliches 2-(((tert-Butoxycarbonyl)amino)oxy)essigsäure (1.00 g, 4.73 mmol) wurde in trockenem CH₃CN (47 ml) gelöst. N-Hydroxysuccinimid (0.66 g, 5.68 mmol) und DCC (1.18 g, 5.68 mmol) wurden nacheinander zu der Lösung gegeben, und das resultierende Reaktionsgemisch wurde bei Raumtemperatur für 1 Stunde gerührt. Danach wurde eine Lösung von 1-((2-aminoethyl)thio)propan-2-ol (0.85 g, 5.68 mmol) in 3 ml trockenem CH₃CN zugegeben und die resultierende Reaktionsmischung bei Raumtemperatur für 18 h gerührt. Das CH₃CN wurde entfernt und das Konzentrat in 50 ml Ethylacetat aufgenommen. Es wurde mit 10%iger Zitronensäure-Lösung (50 ml) und gesättigter NaCl-Lösung gewaschen. Der erhaltene Rückstand wurde durch Chromatographie auf einer Kieselgelsäule mit einem Stufengradienten von MeOH (1-8%) in CH₂Cl₂ als mobile Phase gereinigt. Der gewünschte Baustein wurde als weißer Schaum erhalten. Ausbeute: 0.26 g (16%); Rf (CH₂Cl₂/MeOH, 9:1) 0.50; ¹H NMR (300 MHz, Acetone) δ 8.14 (s, 1H), 4.22 (s, 2H), 3.85 (t, *J=* 1.6 Hz, 1H), 3.53 - 3.38 (m, 2H), 2.72 - 2.66 (m, 2H), 2.62 - 2.58 (m, 2H), 1.46 (s, 9H), 1.19 (d, *J=* 6.1 Hz, 3H).

### [2-[2-[2-(4-Nitrophenoxy)carbonyloxypropylthionyl]ethylamino]-2-oxo-ethoxy]amino] 2,2-dimethylpropanoat P' (X = O, R₁ = H, R₂ = OC₆H₄pNO₂)

Bis(4-nitrophenyl)carbonat (0.276 g, 0.90 mmol) wurden zu einer Lösung von BS3 (X = O, R₁ = H) (0.24 g, 0.75 mmol) in 5 ml trockenem CH₂Cl₂ gegeben. Dann wurde trockenes Pyridin (0.07 ml, 0.75 mmol) unter Eiskühlung hinzugetropft. Die Reaktionslösung wurde 18 Stunden gerührt. Der Niederschlag wurde abfiltriert und es wurde mit 50 ml DCM nachgewaschen. Das Filtrat wurde mit gesättigter NH₄Cl-Lösung (50 ml) gewaschen und die wässrige Phase wurde mit 50 ml CHCl₃ extrahiert. Nach dem Trockenen mit MgSO₄ wurden die vereinigten organischen Phasen am Rotationsverdampfer eingeengt und der Rückstand wurde mittels Säulenchromatographie (Cyclohexan/EtOAc, 2:1) gereinigt. Ausbeute: 0.27 g (96%); Rf (Cyclohexan/EtOAc, 1:1) 0.34; ¹H NMR (300 MHz, CDCl₃) δ 8.35 (s, 1H), 8.27 (d, *J* = 9.1 Hz, 2H), 7.58 (s, 1H), 7.40 (d, *J* = 9.1 Hz, 2H), 5.06 - 4.94 (m, 1H), 4.32 (s, 2H), 3.54 *(d, J=* 6.3 Hz, 2H), 2.87 - 2.73 (m, 4H), 1.48 *(d, J=* 4.1 Hz, 3H), 1.47 (s, Hz, 9H).

### [2-[2-[2-(4-Nitrophenoxy)carbonyloxypropylsulfonyl]ethylamino]-2-oxo-ethoxy]amino] 2,2-dimethylpropanoate Formel (15)

Zu einer Lösung von *P' (X* = *O, R₁* = *Boc, R₂* = *OC₆H₄pNO₂)* (0.30 g, 0.59 mmol) in 5 ml Dichlormethan wurde langsam bei Raumtemperatur m-CPBA (0.263 g, 1.18 mmol) gegeben. Nach 12-stündigem Rühren wurde das Reaktionsgemisch zweimal mit einer gesättigten NaHCO₃-Lösung (15 ml) gewaschen und die organische Phase nach dem Trocknen mit MgSO₄ im Vakuum konzentriert. Das Sulfon wurde als weißer Schaum erhalten. Ausbeute: 0.250 g (84%); Rf (Cyclohexan/EtOAc, 1:1) 0.05; ¹H NMR (300 MHz, CDC13) δ 8.51 (s, 1H), 8.28 (d, *J* = 9.2 Hz, 2H), 7.64 (s, 1H), 7.41 (d, *J=* 9.2 Hz, 3H), 5.47 - 5.35 (m, 1H), 4.33 (s, 2H), 3.88 - 3.79 (m, 2H), 3.57 (dd, *J* = 14.9, 8.3 Hz, 1H), 3.41 - 3.34 (m, 2H), 3.28 (dd, *J* = 14.8, 3.8 Hz, 1H), 1.57 (d, *J* = 6.4 Hz, 3H), 1.48 (s, 9H); ¹³C NMR (75 MHz, CDCl₃) δ 168.58, 155.42, 151.58, 150.52, 145.67, 125.46, 122.04, 85.60, 77.16, 76.62, 70.63, 58.19, 53.67, 33.07, 28.16, 20.21; ESI-MS: (berechnet MH+: 528.16 g/mol, gefunden: 528.15 m/z).

### Synthesen zur Darstellung der Verbindung der Formel (16)

### Natrium 4-carboxy-2-nitrobenzenesulfonat

Es wurde 4-Sulfamylbenzoesäure in einer Mischung aus 5 ml rauchender HNO₃ und 10 ml H₂SO₄ (95%) gelöst. Die Reaktionslösung wurde bei 90°C über Nacht gerührt und anschließen mit 100 ml Wasser verdünnt. Bei 0 °C wurde die Säure durch Zugabe von Na₂CO₃ neutralisiert. Anschließend wurde durch Zugabe von HCl solange angesäuert bis der pH-Wert 2 betrug. Das Wasser wurde entfernt und der Rückstand wurde mit EtOH/iPrOH (1:1) extrahiert. Anschließend wurde die organischen Lösungsmittel entfernt wobei das Produkt als brauner Feststoff erhalten wurde. Ausbeute: 6.86 g (61%); Rf (CH₂Cl₂/MeOH/AcOH, 7:2:1) 0.05; ¹H NMR (300 MHz, MeOD) δ 8.23 (d, J= 1.6 Hz, 1H), 8.17 (d, *J* = 1.6 Hz, 1H), 8.16 (s, 1H); ESI-MS(neg.): (berechnet (M-Na)⁻: 245.97 g/mol, gefunden: 296.00 m/z).

### Natrium 4-(2-(tert-Butoxycarbonyl)hydrazin-1-earbonyl)-2-nitrobenzolsulfonat

Eine Lösung von *t*-Butyl-Carbazat (1.91 g, 14.27 mmol) und *Natrium 4-carboxy-2-nitrobenzolsulfonat* (3.84 g, 14.27 mmol) sowie 1-Ethyl-3-(3-dimethylaminopropyl)carbodiimid (2.76 g, 14.27 mmol) wurde in 60 ml Methanol/H₂O (1:1) über Nacht bei Raumtemperatur gerührt. Die Lösungsmittel wurden unter verminderten Druck entfernt und der Rückstand wurde mittels Säulenchromatographie (CH₂Cl₂/MeOH, 9:1) gereinigt. Eine Lösung von *t*-Butyl-Carbazat (1.91 g, 14.27 mmol) und *Natrium 4-carboxy-2-nitrobenzolsulfonat* (3.84 g, 14.27 mmol) sowie 1-Ethyl-3-(3-dimethylaminopropyl)carbodiimid (2.76 g, 14.27 mmol) wurde in 60 ml Methanol/H₂O (1:1) über Nacht bei Raumtemperatur gerührt. Die Lösungsmittel wurden unter verminderten Druck entfernt und der Rückstand wurde mittels Säulenchromatographie (CH₂Cl₂/MeOH, 9:1) gereinigt. Ausbeute: 6.86 g (61%); Rf (CH₂Cl₂/MeOH/AcOH, 7:2:1) 0.30; ¹H NMR (300 MHz, DMSO) δ 10.46 (s, 1H), 9.04 (s, 1H), 8.01 (d, *J* = 1.5 Hz, 1H), 7.99 (d, *J* = 1.5 Hz, 1H), 7.96 (s, 1H), 1.43 (s, 9H); ESI-MS(neg.): (berechnet (M-Na)⁻: 360.05 g/mol, gefunden: 360.00 m/z).

### tert-Butyl 2-(4-(chlorosulfonyl)-3-nitrobenzoyl)hydrazin-1-carboxylat

Zu einer Lösung von Natrium 4-(2-(tert-butoxycarbonyl)hydrazin-1-carbonyl)-2-nitrobenzolsulfonat (0.55 g, 1.42 mmol) und 18-Krone-6 Ether (0.02 g, 0.07 mmol) wurde Cyanurchlorid (0.26 g, 1.42 mmol) in trockenem Aceton gegeben. Die Lösung wurde für 18 h unter Rückfluss erhitzt. Nach dem Abkühlen wurde die Reaktionsmischung über Celite filtriert und mittels Säulenchromatographie gereinigt (CH₂Cl₂/MeOH, 9:1). Ausbeute: 0.23 g (43%); ¹H NMR (300 MHz, CDCl₃) δ 10.18 (s, 2H), 8.22 (d, *J* = 0.5 Hz, 1H), 8.22 (dd, *J* = 1.7, 0.5 Hz, 1H), 8.18 (d, *J* = 1.6 Hz, 1H), 8.15 (d, *J* = 0.6 Hz, 1H), 1.23 (s, 9H); ESI-MS(neg.): (berechnet (M)⁻: 379.02 g/mol, gefunden: 378.92 m/z).

### Synthesen zur Darstellung der Verbindung der Formel (20)

### 6-Azidoisobenzofuran-1(3H)-on

Eine klare Lösung von 6-Amino-phtalid (2.50 g, 15.92 mmol) in 1M HCl (28 ml) wurde auf 0°C gekühlt und mit 3 ml einer wässrigen Lösung aus NaNO₂ (1.66 g, 13.89 mmol) tropfenweise versetzt. Die so erhaltene Suspension wurde für 10 min bei 0°C gerührt und mit 10 ml einer Lösung aus NaN₃ (2.09 g, 31.85 mmol) tropfenweise bei 0°C versetzt (heftige HN₃-Gasentwicklung!, Schaumbildung). Die schaumige Suspension wurde eine Stunde bei 0°C gerührt. Der Niederschlag wurde abgesaugt und mit mehrfach mit insgesamt 300 ml Wasser gewaschen. Der braune Feststoff wurde zerstoßen und im Trockenschrank über Nacht getrocknet. Anschließend wurde er in 300 ml CH₂Cl₂ gelöst und abfiltriert. Das Filtrat wurde unter Vakuum vom Lösungsmittel befreit wobei ein hellbrauner Feststoff erhalten wurde. Ausbeute: 2.53 g (91%); Rf (CH₂Cl₂/MeOH/AcOH, 7:2:1) 0.30; ¹H NMR (300 MHz, CDCl₃) δ 7.58 (d, *J* = 1.9 Hz, 1H), 7.47 (dd, *J* = 8.2, 0.7 Hz, 1H), 7.31 (dd, *J* = 8.2, 2.1 Hz, 1H), 5.31 (s, 2H); ESI-MS: (berechnet (MH)⁺: 176.05 g/mol, gefunden: 176.23 m/z).

### 5-Azido-2-(hydroxymethyl)benzohydrazid

6-Azidoisobenzofuran-1(3H)-on (0.50 g, 2.83 mmol) wurden in 6 ml Dimethylformamid (DMF) gelöst und es wurde Hydrazinhydrat (0.71 ml, 14.13 mmol) hinzugegeben und die Lösung wurde bei 70 °C für 3 Stunden gerührt. Das DMF und das Hydrazinhydrat wurden unter Vakuum entfernt. Der Rückstand wurde mittels Säulenchromatographie (CH₂Cl₂/MeOH, 9:1) gereinigt, wobei ein hellgelbes Pulver erhalten wurde. Ausbeute: 0.10 g (17%). Rf (CH₂Cl₂/MeOH, 9:1) 0.53; ¹H NMR (300 MHz, DMSO) δ 9.62 (s, 1H), 7.57 (d, *J* = 8.3 Hz, 1H), 7.20 (dd, *J* = 8.3, 2.4 Hz, 1H), 7.09 (d, *J* = 2.4 Hz, 1H), 5.31 - 5.21 (m, 1H), 4.57 (d, *J* = 5.7 Hz, 2H), 4.50 (s, 2H), 3.33 (s, 2H), ESI-MS: (berechnet (MH)⁺: 208.08 g/mol, gefunden: 207.90 m/z), (berechnet (MNa)⁺: 230.07 g/mol, gefunden: 230.01 m/z).

### tert-Butyl 2-(5-azido-2-(hydrozymethyl)benzoyl)hydrazin-1-carbozylat

5-Azido-2-(hydroxymethyl)benzohydrazid (0.10 g, 0.48 mmol) wurden in Dioxan/EtOAc/*i*PrOH (1:1:1) 10 ml gelöst und mit Boc-Anhydrid (0.105 g, 0.48 mmol) und DIPEA (0.10 ml, 0.57 mmol) versetzt. Die Lösung wurde bei Raumtemperatur 12 Stunden gerührt und anschließend wurde das Lösungsmittel unter verminderten Druck entfernt. Der Rückstand wurde in 50 ml CH₂Cl₂ aufgenommen und zweimal mit einer 10%igen Zitronensäure Lösung gewaschen (je 50 ml). Nach dem Trocknen mit MgSO₄ und entfernen des organischen Lösungsmittel wurde ein gelbliches Öl erhalten. Ausbeute: 0.10 g (67%). Rf (CH₂Cl₂/MeOH, 9:1) 0.75; ¹H NMR (300 MHz, DMSO) δ 10.06 (s, 1H), 9.04 (d, *J* = 30.3 Hz, 1H), 7.69 - 7.58 (m, 1H), 7.25 (dd, *J* = 8.3, 2.4 Hz, 1H), 7.10 (d, *J* = 1.3 Hz, 1H), 5.28 (t*, J* = 5.7 Hz, 1H), 4.62 (d, *J* = 5.6 Hz, 2H), 1.43 (s, 9H); ESI-MS: (berechnet (MNa)⁺: 330.12 g/mol, gefunden: 330.29 m/z).

### tert-Butyl 2-(5-azido-2-(((((2,5-dioxopyrrolidin-1-yl)oxy)carbonyl)oxy)methyl)benzoyl)hydrazin-1-carboxylat

N,N-Disuccinimidyl carbonat (0.105 g, 0.41 mmol) wurden zu einer Lösung von tert-Butyl 2-(5-azido-2-(hydroxymethyl)benzoyl)hydrazin-1-carboxylat (0.105 g, 0.34 mmol) in 5 ml trockenem Dimehtylformamid gegeben. Dann wurde trockenes Pyridin (0.03 ml, 0.41 mmol) hinzugetropft. Die Reaktionslösung wurde 17 Stunden bei Raumtemperatur gerührt. Das Lösungsmittel wurde unter Vakuum entfernt. Der Lösung wurde 50 ml DCM hinzugefügt. Die organische Phase wurde mit 10%iger Zitronensäure-Lösung gewaschen (2x 50 ml) und mit MgSO₄ getrocknet. Die vereinigten organischen Phasen wurden am Rotationsverdampfer eingeengt und der Rückstand wurde mittels Säulenchromatographie (CH₂Cl₂/MeOH, 19:1) gereinigt. Ausbeute: 0.06 g (40%). Rf (CH₂Cl₂/MeOH, 19:1) 0.45; ¹H NMR (300 MHz, DMSO) δ 10.06 (s, *J* = 10.9 Hz, 1H), 8.99 (s, *J* = 8.4 Hz, 1H), 7.62 (d, *J* = 8.3 Hz, 1H), 7.53 (s, 1H), 7.25 (dd, *J* = 8.3, 2.4 Hz, 1H), 5.76 (s, 2H), 3.34 (s, 4H), 1.43 (s, 9H), ESI-MS: (berechnet (MNa)⁺: 471.12 g/mol, gefunden: 471.25 m/z).

### Synthesen zur Darstellung der Verbindung der Formel (21)

### 6-Methoxyisobenzofuran-1(3H)-on

Eine Mischung aus 3-Methoxybenzoesäure (10.00 g, 65.07 mmol) , 37% iger Formalin-Lösung (7.5 ml, 80 mmol), 37% iger HCl (8.00 ml) und 75 ml 100% iger Essigsäure wurde unter Rückfluss 18 Stunden erwärmt. Nach dem Abkühlen wurde die klare Lösung wird der Rührer abgeschaltet und 14 Stunden bei dieser Temperatur belassen. Die Essigsäure wurde bei 80 °C im Luftstrom entfernt. Der Rückstand wurde in 150 ml Toluol aufgenommen und auf 40 ml konzentriert. Die 80 °C heiße Lösung wurde mit 40 ml Portionen 20% iger Na₂CO₃-Lösung (3-mal) und mit 40 ml Wasser gewaschen. Nach Zugabe von 3 ml Morpholin wurde die organische Phase 2 h bei 80 °C gerührt und dann mit 50 ml Portionen 10prozentiger H₂SO₄ (3-mal) und Wasser gewaschen. Zur Kristallisation des Produkts wurde das Gemisch auf 25 ml konzentriert und das Gemisch wurde gerührt. Das Produkt wurde durch Abfiltrieren in Form von weißen Kristallen erhalten. Ausbeute: 3.52 g (33%). ¹H NMR (300 MHz, DMSO) δ 7.58 (dd, *J* = 8.3, 0.7 Hz, 1H), 7.35 (dd, *J* = 8.3, 2.4 Hz, 1H), 7.31 (d, *J* = 2.2 Hz, 1H), 5.34 (s, 2H), 3.84 (s, 3H).

### 6-Hydroxyisobenzofuran-1(3H)-on

Stickstoffatmosphäre wurde 6-Methoxyisobenzofuran-1(3H)-on (3.00 g, 18.09 mmol) in wasserfreiem Dichlormethan (100 ml) gelöst. Die resultierende Mischung wurde magnetisch gerührt und im Eisbad für 10 min gekühlt. Danach wurde BBr₃ (3.46 ml, 36.18 mmol) zugetropft. Dann wurde sich das Reaktionsgemisch auf Raumtemperatur erwärmen lassen und 12 Stunden gerührt. Anschließend wurde 5 ml Wasser zugegeben, und das Gemisch wurde in einen Scheidetrichter überführt und mit Ethylacetat (3 x 100 ml) extrahiert. Die vereinigten organischen Extrakte wurden über Magnesiumsulfat getrocknet, filtriert und unter reduziertem Druck konzentriert, wobei ein weißer Feststoff erhalten wurde. Ausbeute: 1.52 g (56%). ¹H NMR (300 MHz, DMSO) δ 10.08 (s, 1H), 7.47 (dd, *J=* 8.3, 0.6 Hz, 1H), 7.19 (d, *J=* 2.3 Hz, 1H), 7.16 (d, *J=* 2.3 Hz, 1H), 7.10 (d, *J=* 2.0 Hz, 1H), 5.28 (s, 2H); ESI-MS: (berechnet (MH)⁺: 151.04 g/mol, gefunden: 151.05 m/z).

### 5-Azido-2-(hydroxymethyl)benzohydrazid

6-Hydroxyisobenzofuran-1(3H)-on (0.43 g, 2.83 mmol) wurden in 6 ml Dimethylformamid (DMF) gelöst und es wurde Hydrazinhydrat (1.42 ml, 28.26 mmol) hinzugegeben und die Lösung wurde bei 100 °C für 3 Stunden gerührt. Das DMF und das Hydrazinhydrat wurden unter Vakuum entfernt. Der Rückstand wurde mittels Säulenchromatographie (CH₂Cl₂/MeOH, 9:1) gereinigt, wobei ein hellgelbes Pulver erhalten wurde. Ausbeute: 0.10 g (17%). Rf (CH₂Cl₂/MeOH, 9:1) 0.40; ¹H NMR (300 MHz, DMSO) δ 10.08 (s, 1H), 9.73 (s, 1H), 7.47 (d, *J=* 8.3 Hz, 1H), 7.18 (dd, *J* = 8.3, 2.3 Hz, 1H), 7.10 (d, *J* = 2.3 Hz, 1H), 4.57 (d, *J* = 5.7 Hz, 2H), 4.50 (s, 2H), 3.33 (s, 2H), ESI-MS: (berechnet (MH)⁺: 183.08 g/mol, gefunden: 183.15 m/z).

### Exemplarische Reinigung mit Fängermolekül (14)

Die Reinigung wurde mit einem weiteren Beispielpeptid 14 (AKADEVSLHKWYG; SEQ ID NO: 10) und einem Linker nach Formel (14) (Fängermolekül 14) auf kommerziell erhältlicher, Aldehydmodifizierter Agarose (High Density Glyoxal, 6BCT von ABT) durchgeführt.

### Festphasenpeptidsynthese der Peptide sowie Reinigung

Die automatisierte Festphasenpeptidsynthese erfolgte in 100 µmol Ansätzen, mit einem MultiPep RS-Peptidsyntheseautomaten der Firma Intavis AG. Die Synthese wurde an einem Wang-Harz (1.0-1.4 mmol/g) der Firma Carl Roth durchgeführt. Vor dem Beginn der Synthese wurde die entsprechende Menge an Peptid-Syntheseharz in 5 mL Spritzenreaktoren (PE-Reaktor der Firma Intavis) eingewogen und in DMF gequollen. Die Einwaage an Äquivalenten an Aminosäure bezog sich, sofern nicht anders erwähnt, auf die Erstbeladung des eingesetzten Harzes.

### Fmoc-Abspaltung:

Zur Abspaltung der temporären Fmoc-Schutzgruppen wurde das Harz einmal 5 min und ein weiteres Mal 8 min mit 1500 µL Piperidin/DMF (4:1) behandelt und nachfolgend siebenmal mit 10.2 mL DMF gewaschen und es wurde mit der Kupplung der Fmoc-Aminosäurederivate fortgefahren.

### Kupplung der Fmoc-Aminosäurederivate:

Eine Lösung von 5 eq. Aminosäure in DMF (0.3 M) wurde 1 min bei Raumtemperatur mit Lösungen von 4.5 eq. HCTU in DMF (0.3 M) und 10 eq. NMM in DMF (0.6 M) voraktiviert und anschließend zum Harz gegeben. Nach 30 min Reaktionszeit wurde das Harz dreimal mit 10.2 mL DMF gewaschen und es wurde mit dem Blockieren der Abbruchsequenzen fortgefahren.

### Blockieren der Abbruchsequenzen:

Das Harz wurde zweimal 5 min mit 1.5 mL einer Lösung von AC₂O/2,6-Lutidin/DMF (5:6:89) behandelt und nachfolgend siebenmal mit je 10.2 mL DMF gewaschen.

### Letzter Schritt Kopplung des Fängermoleküls:

Als letzter Schritt der Festphasenpeptidsynthese wurde das Fängermolekül (14) auf das gewünschte Zielpeptid (50 µmol) gekuppelt. Es wurde das Syntheseharz mit einer Lösung aus 4 eq. Fängermolekül (0.3 M), 6 eq. HOBt in DMF (0.4 M) und 4 eq. DIPEA in DMF (0,3 M) gemischt. Nach 60 min Reaktionszeit wurde zweimal mit je 2 mL DMF, zweimal mit 2 mL CH₂Cl₂ und anschließend wieder zweimal mit 2 mL DMF gewaschen.

### Alternativer letzter Schritt der Acetylierung des Vollängenpeptids:

Analog zur Vorschrift zur Blockierung der Abbruchsequenzen wurde das Vollängenpeptid als Kontrollprobe ebenfalls acetyliert (Peptid-Acetyliert).

### Freisetzung vom polymeren Träger:

Das Harz wurde mit 3 mL einer Lösung von 95% TFA, 2.5% Wasser und 2.5% Triisopropylsilan, behandelt. Das Syntheseharz wurde mit dieser Abspaltmischung versetzt und 3 h bei Raumtemperatur geschüttelt. Anschließend wurde die Abspaltlösung aufgefangen und das Harz zweimal mit je 1 mL TFA nachgewaschen. Die Abspaltlösung wurde mit den Waschlösungen vereinigt und durch Argonstrom eingeengt auf ca. 1 mL Volumen. Anschließend wurde mit 10 mL kaltem Diethylether gefällt und anschließend der Niederschlag abzentrifugiert. Der Überstand wurde verworfen. In Fig. 4h (oben) ist das Chromatogramm des Peptids ohne Linker vor der Reinigung mit einer Reinheit von 45% gezeigt.

### Reinigung

Der krude Niederschlag (etwa 5 µmol theoretische Ausbeute) wurde in Konjugationspuffer (0.1 M NH₄OAc, 0.1 M Anilin, pH = 3.8) aufgenommen. Wenn sich die Mischung nicht vollständig löste, wurde Acetonitril zugegeben. In einen 3 mL Spritzenreaktor mit einem 25 µm PE Vorfilter wurden 400 µL (~160 mg) Agarose gegeben. Das Reinigungsharz wurde dann durch 3-maliges Waschen mit Konjugationsspuffer (0.1 M NH₄OAc, 0.05 M Anilin, pH = 3.8) konditioniert. Die Peptidlösung wurde dann auf das Reinigungsharz gegeben. Die Immobilisierung wurde anschließend 60 min durchgeführt. Danach wurde dreimal mit Konjugationspuffer, dreimal mit einer 5 M Harnstoff Lösung, dreimal mit 70%igem Ethanol und schließlich fünfmal mit Wasser gewaschen. Das Gemisch wurde dann basisch mit 5 v% NH₄OH in Wasser behandelt, um konjugiertes Peptid vom Harz zu spalten. Lyophilisieren liefert das Peptid als weißen flockigen Feststoff.

### Nachweis Immobilisierung

Um die Immobilisierung eindeutig nachzuweisen, wurde in diesem Experiment acetyliertes Peptid, sowie Peptid mit gebundenem Linker sowohl auf modifizierter, als auch auf reiner Agarose (6% B Agarose Bead STANDARD, ABT) mit PEC gereinigt. Das Eluat nach der Linkerspaltung wurde jeweils mittels UPLC-UV vermessen. Im Ergebnis zeigte sich, dass nach der Spaltung lediglich bei Peptid mit gebundenem Linker auf modifiziertem Reinigungsharz ein signifikantes Signal mit der Produktmasse detektiert wird. Zusätzlich erkennt man, dass auch bei reiner Agarose etwa 2.3% Produkt im Vergleich zu modifizierter Agarose erhalten wird.

**Tabelle 3: Integrale über den Produktpeak für die Peptide nach Reinigung mit modifizierter und nicht modifizierter Agarose**

| | **Peptid-Linker Agarose modifizierte Agarose** | | **Peptid-Acetyliert Agarose modifizierte Agarose** | |
|---|---|---|---|---|
| Integral (µV^{∗}s) | 17759 | 763044 | 197 | 131 |
| Anteil | 2.31% | 100% | 0.03% | 0.02% |

### Regenerierung des Reinigungsharzes

Nach der Peptidreinigung mit dem Fängermolekül **(14)** bleibt die ursprüngliche Aldehydfunktion von 1 mit dem Hydroxyl-modifizierten Fängermolekül blockiert zurück. Um das Reinigungsharz wieder für einen neuen Reinigungszyklus bereit zu stellen, muss das Harz regeneriert und somit die Aldehydfunktion wiederhergestellt werden. Dies gelingt bei Verschieben des Gleichgewichtes durch Zugabe von Aldehyden oder Ketonen. Die Regenration für wiederholte Reinigungszyklen wurde wie folgt gezeigt:
Es wurden zwei Reinigungen von Peptid **14** simultan durchgeführt (Reinigung I). Anschließend wurde das Reinigungsharz für die Regeneration je viermal mit einer Mischung aus Wasser, Aceton und TFA **(Keton,** 49.95:49.95:0.1), bzw. Wasser, Acetaldehyd und TFA **(Aldehyd,** 89.95:9.95:0.1) und 5-mal mit Wasser gewaschen. Danach erfolgte die Reinigung inklusive der Konditionierung analog zum oben beschriebenen Vorgehen. Die Regeneration und Reinigung wurde insgesamt dreimal durchgeführt (Reinigung II - IV) und das lyophilisierte Produkt je in gleichen Volumina Wasser, Acetonitril und TFA (69.9:29.9: 1) aufgenommen und mittels UPLC-MS vermessen.

**Tabelle 4: Integrale über den Produktpeak und prozentualer Anteil für die Peptide nach Reinigung mit modifizierter Agarose (Reinigung I) und nach drei Regenrationszyklen (Reinigung II - IV).**

| | **Keton** | | | **Aldehyd** | | |
|---|---|---|---|---|---|---|
| **Reinigung** | **Integral (µV^{*}s)** | **Anteil** | **Reinheit** | **Integral (µV^{*}s)** | **Anteil** | **Reinheit** |
| I | 4100144 | 100% | 58,9% | 2138955 | 100% | 58,2% |
| II | 2044538 | 50% | 58,5% | 911365 | 43% | 58,7% |
| III | 1719147 | 42% | 57,8% | 567140 | 27% | 58,6% |
| IV | 381717 | 9% | 61,5% | 576371 | 27% | 59,8% |

Im Ergebnis zeigte sich, dass sich in beiden Fällen das Harz regenerieren lässt. Die Regenerierung mit einem Keton fällt erst im dritten Zyklus (Reinigung IV) deutlich ab. Im Unterschied dazu verbleibt die Reinigungskapazität bei der Aldehyd-Regenerierung bei etwa einem Viertel der Ausgangskapazität, fällt dafür aber schon nach dem zweiten Zyklus (Reinigung III) auf diesen Wert. Bei beiden Experimenten konnte eine Reinheit von etwa 60% erzielt werden, die konstant über die Regenerationszyklen erhalten bleibt (Beispielchromatogramm zu Reinigung I, Aldehyd, Fig. 4h (unten)).

## Patentansprüche

1. Verbindung der Formel
X₁-L-X₂ (1),
wobei
X₁ ausgewählt ist aus 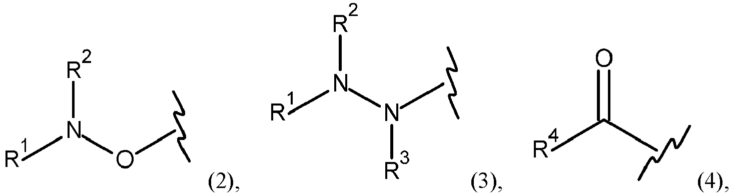
- wobei jedes R¹ und R² unabhängig voneinander ausgewählt ist aus H oder B, wobei wenigstens R¹ oder R² B ist, wobei
- R³ aus H oder B ausgewählt ist,
- wobei B eine säurelabile Amin-Schutzgruppe ist, wobei
- R⁴ ausgewählt ist aus H, C₁-C₁₂-Alkyl oder Aryl, wobei die Aldehyd- oder Ketogruppe durch eine säurelabile Schutzgruppe geschützt vorliegen kann,
L ausgewählt ist aus funktionellen Linkerverbindungen, die unter basischen Bedingungen von X₂ nukleophil abspaltbar sind, insbesondere weist L die Form -T-U- auf, wobei
- T ein Abstandshalter zwischen X₁ und U ist, wobei T insbesondere ausgewählt ist aus substituierte oder unsubstituierte -C₁-C₁₂-Alkyl-, insbesondere C₁-C₆-Alkyl, insbesondere C₁-C₃-Alkyl, -R⁵-C(=O)-NH-R⁶-, -R⁵-C(=O)-O-R⁶-, -R⁵-C(=O)-O-, -C(=O)-O-R⁶-, -C(=O)-NH-R⁶-, -C(=O),-C(=O)-O-, -R⁵-phenyl-R⁶-, -R⁵-phenyl-, -phenyl-R⁶-, -phenyl-,
- wobei R⁵ und R⁶ unabhängig voneinander ausgewählte substituierte oder unsubstituierte C₁-C₁₂-Alkyle, insbesondere C₁-C₆ Alkyle, besonders C₁-C₃ Alkyle sind, und wobei
- U der die Spaltung aktivierende Teil der funktionellen Linkerverbindung ist, wobei der aktivierende Teil ausgebildet ist ein bei einer basischen Abspaltung von X₂ entstehendes Anion zu stabilisieren,
X₂ die Form -Y-Z aufweist, wobei
- Y aus -O-C(=O)- oder -S(=O)₂- ausgewählt ist, und
- Z eine elektronenziehende Fluchtgruppe ist.

2. Verbindung nach Anspruch 1, wobei
- B ausgewählt ist aus Boc (-C=OOtBu), Trityl (-C(Ph)₃), Mmt (-C(Ph)₂C₆H₄OMe), DMT (-C(Ph)(C₆H₄OMe)2), Cbz (-C=OOCH₂Ph), Benzylideneamin (=CPh), Phtalimide (=(CO)₂C₆H₄), p-Toluenesulfonamide (-SO₂C₆H₄Me). Benzylamin (-CH₂Ph), Acetamide (-COMe), Trifluoracetamid (-COCF₃), Dde (1-(4,4-Dimethyl-2,6-dioxocyclohex-1-ylidene)-3-ethyl) und 1-(4,4-Dimethyl-2,6-dioxocyclohex-1-ylidene)-3-methylbutyl (ivDde), wobei B insbesondere Boc ist, und/oder die
- Acetal- oder Ketalschutzgruppen, ausgewählt sind aus 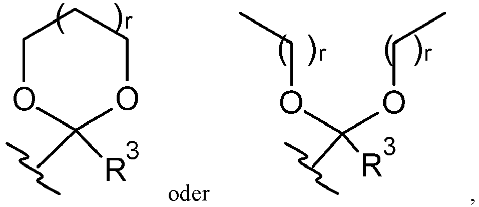 wobei r 0 bis 12, insbesondere 0, 1 oder 2, ist.

3. Verbindung nach Anspruch 1 oder 2, wobei T ausgewählt ist aus -C₁-C₁₂-Alkyl-, -R⁵-C(=O)-NH-R⁶-, -R⁵-C(=O)-O-R⁶-, -R⁵-C(=O)-O-, -C(=O)-O-R⁶-, -C(=O)-NH-R⁶-, -C(=O)-, -C(=O)-O-, wobei R⁵ und R⁶ unabhängig voneinander ausgewählte C₁-C₆-Alkyle, insbesondere C₁-C₂-Alkyle sind, insbesondere ist T ausgewählt aus -CH₂-, -CH₂-C(=O)-NH-(CH₂)₂-, -(CH₂)-C(=O)-O-(CH₂)₂-,-CH₂-C(=O)-O-, -C(=O)-O-, -C(=O)-O-(CH₂)₂-, -C(=O)-, insbesondere -CH₂-, -CH₂-C(=O)-NH-(CH₂)₂-, -C(=O)-O-(CH₂)₂-, -CH₂-C(=O)-O-, -C(=O)-.

4. Verbindung nach wenigstens einem der Ansprüche 1 bis 3, wobei U der Verbindung T-U-Y ausgewählt ist aus den Verbindungen nach den Formeln (5), (6), (7), (8), (9), (10) und (11), insbesondere aus (5), (6), (8), (9) und (10), 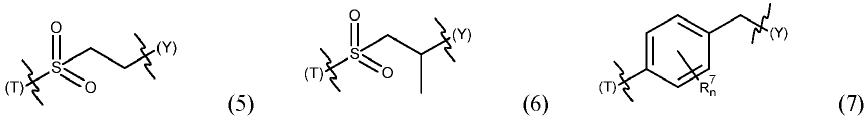 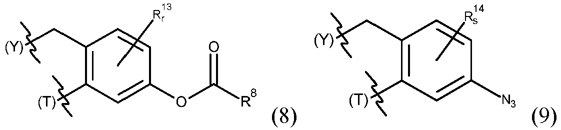 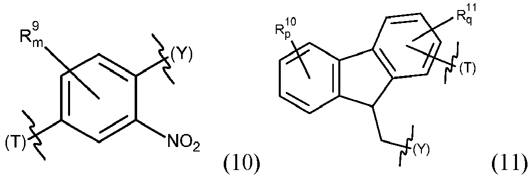
- wobei R⁸ ausgewählt ist aus C₁-C₆-Alkyl, CF₃, CH₂CF₃, 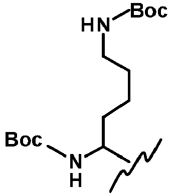 (Boc-Lys(Boc)-) 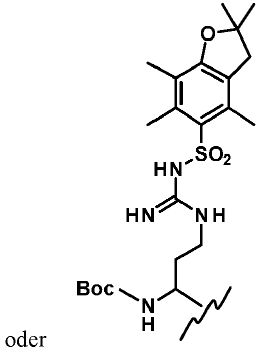 (Boc-Arg(pbf)), insbesondere aus Boc-Lys(Boc)-,
- wobei R⁷ₙ, R⁹ₘ, R¹⁰ₚ,R¹¹_{q}, R¹³ᵣ und R¹⁴ₛ ausgewählt ist aus C₁-C₆-Alkyl oder -I und/oder -M-Effekte erzeugenden Substituenten, insbesondere C₁-C₃-Alkyle, -F, -Cl, -Br, -I, -CN -NO₂, -N₃, -CF₃, -SO₃H, -CO₂H,
- wobei n gleich 0, 1, 2, 3 oder 4, insbesondere n ist 0 oder 1, insbesondere 0, ist
- wobei m gleich 0, 1, 2 oder 3, insbesondere m ist 0 oder 1, insbesondere 0, ist
- wobei p gleich 0, 1, 2, 3 oder 4, insbesondere p ist 0 oder 1, insbesondere 0, ist
- wobei q gleich 0, 1, 2 oder 3, insbesondere q ist 0 oder 1, insbesondere 0, ist
- wobei r gleich 0, 1, 2 oder 3, insbesondere r ist 0 oder 1, insbesondere 0, ist
- wobei s gleich 0, 1, 2 oder 3, insbesondere s ist 0 oder 1, insbesondere 0, ist.

5. Verbindung nach wenigstens einem der Ansprüche 1 bis 4, wobei Z ausgewählt ist aus der Gruppe -F, -Cl, -Br, -I, -N₃, -SR¹², -OCF₃, -OCH₂CF₃, -OSO₂CF₃, -SO₂C₆H₄CH₃, -SO₂CF₃, -SO₂CH₃ 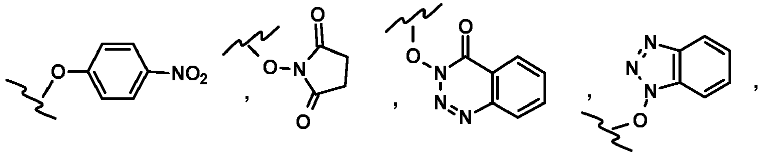 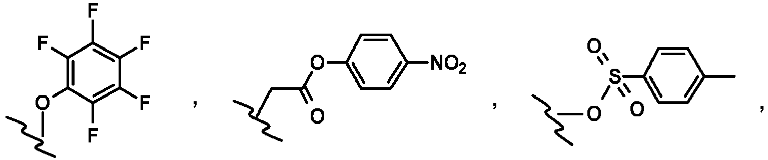 insbesondere -Cl, 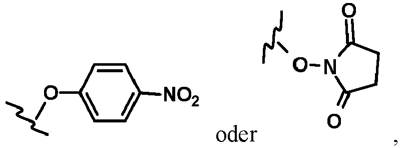 insbesondere 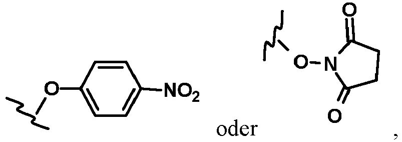 wobei R¹² ein C₁-C₆-Alkyl-, ein Aryl- oder ein Benzylrest ist.

6. Verbindung nach wenigstens einem der Ansprüche 1 bis 5, wobei X₁ eine Verbindung nach Formel (2) oder (3) ist, insbesondere Formel (3), wobei R³ H ist, R¹ und R² eine Boc-Schutzgruppe aufweisen oder R¹ H ist und R² eine Boc-Schutzgruppe ist, wobei insbesondere R¹ H ist und R² eine Boc-Schutzgruppe ist.

7. Verbindung nach wenigstens einem der Ansprüche 1 bis 6, wobei Y die Form -O-C(=O)- aufweist.

8. Verbindung nach wenigstens einem der Ansprüche 1 bis 7,
- wobei T die Form -(CH₂)-C(=O)-NH-(CH₂)₂-, -(CH₂)-C(=O)-O-(CH₂)₂-, -C(=O)-O-(CH₂)₂-aufweist, insbesondere -C(=O)-O-(CH₂)₂- und -(CH₂)-C(=O)-NH-(CH₂)₂-, insbesondere-(CH₂)-C(=O)-NH-(CH₂)₂-,
- wobei U eine Verbindung nach Formel (5) oder (6) ist, insbesondere nach Formel (6),

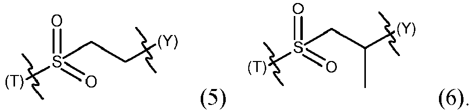

9. Verbindung nach wenigstens einem der Ansprüche 1 bis 7,
- wobei T die Form -CH₂-C(=O)-O-, -C(=O)-O- aufweist, insbesondere -CH₂-C(=O)-O-,
- wobei U eine Verbindung nach Formel (7) ist, 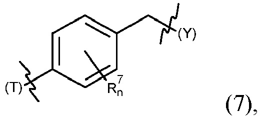 wobei R⁷ ausgewählt ist C₁-C₆-Alkyl oder -I und/oder -M-Effekt erzeugenden Substituenten, insbesondere C₁-C₃-Alkyl, -F, -Cl, -Br, -I, -CN -NO₂, -N₃, -CF₃, -SO₃H, -CO₂H wobei n gleich 0, 1, 2, 3 oder 4, insbesondere 0 oder 1, insbesondere 0, ist, und/oder
- wobei T die Form -CH₂- aufweist,
- wobei U eine Verbindung nach Formel (8) ist, 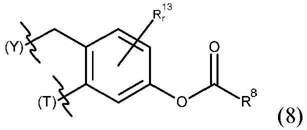
- wobei R⁸ Boc-Lys(Boc) und r gleich 0 ist, und/oder
- wobei T die Form -CH₂-, -(C=O)-, insbesondere -CH₂-, aufweist,
- wobei U eine Verbindung nach Formel (9) ist, wobei s gleich 0 ist, 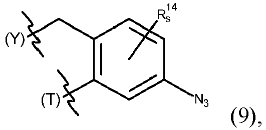 und/oder
- wobei T die Form -C(=O)- aufweist,
- wobei U eine Verbindung nach Formel (10) ist, wobei m gleich 0 ist, 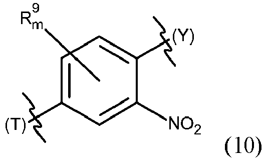
- wobei Y die Form -SO₂- aufweist, und
- wobei Z Cl ist.

10. Verwendung einer Verbindung nach wenigstens einem der Ansprüche 1 bis 9, als Verbindung zwischen der N-terminalen Aminogruppe eines Volllängenpeptids und einer festen Phase.

11. Eine Verbindung der Formel X₁-L-Y-PEP (12), wobei X₁, L und Y nach dem unabhängigen Anspruch 1 und den abhängigen Ansprüchen von Anspruch 1 definiert ist, und wobei PEP ein Volllängenpeptid umfasst, das über seinen N-Terminus mit Y verbunden ist.

12. Eine Verbindung der Formel D-X₁'-L-Y-PEP (13),
- wobei D ein oberflächenmodifizierter fester Träger ist, der **dadurch gekennzeichnet ist, dass** die Oberfläche durch synthetische oder natürliche Polymere modifiziert ist, insbesondere modifizierte Polysaccharide, insbesondere Aldehyd- oder Hydrazin-modifizierte Sepharose/Agarose oder Zellulose.
- wobei X₁' die Form -NH-O-, -NH-NH- oder -C(=O)- aufweist,
- wobei L, Y und PEP nach Anspruch 11 definiert sind.

13. Verfahren zur Reinigung von Peptiden, insbesondere mittels Festphasenpeptidsynthese (SPPS) hergestellten Peptiden, das die folgenden Schritte umfasst:
i. In-Kontakt-Bringen einer Zusammensetzung eines zu reinigenden Volllängenpeptids und mindestens einer Verunreinigung, insbesondere mindestens einer acetylierten Abbruchsequenz, mit einer Fängerverbindung nach einem der Ansprüche 1 bis 9, und anschließender Reaktion zu einer Verbindung der Formel (12),
ii. Abspaltung der säurelabilen Schutzgruppen durch Zugabe einer Säure,
iii. In-Kontakt-Bringen der Zusammensetzung aus ii. mit einem oberflächenmodifizierten festen Träger, wobei zwischen der Fängerverbindung und dem festen Träger eine kovalente Hydrazon oder Oxim-Bindung ausgebildet wird, und eine Verbindung der Formel (13) bereitgestellt wird,
iv. Abspaltung des Volllängenpeptids vom festen Träger.

14. Verfahren nach Anspruch 13, wobei der feste Träger an seiner Oberfläche die funktionalen Gruppen Aldehyd, insbesondere -O-CH₂-CHO, Keton, Hydroxylamin, insbesondere -ONH₂, und Hydrazin, insbesondere -N₂H₃, aufweist.

15. Verfahren nach Anspruch 13, wobei nach oder bei der Abspaltung des Volllängenpeptids vom festen Träger der feste Träger D von dem Rest X¹-L der Fängerverbindung abgespalten und der feste Träger regeneriert wird.

## Claims

1. Compound of the formula
X₁-L-X₂ (1),
wherein
X₁ is selected from 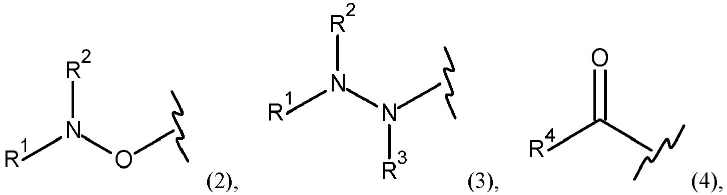
- wherein each R¹ and R² is independently from each other selected from H or B, wherein at least R¹ or R² is B, wherein
- R³ is selected from H or B,
- wherein B is an acid labile amine protecting group, wherein
- R⁴ is selected from H, C₁-C₁₂-alkyl or aryl, wherein the aldehyde or keto group may be protected by an acid labile protecting group,
L is selected from functional linker compounds, that are cleavable nucleophilically from X₂ under basic conditions, in particular L is of the form -T-U-, wherein
- T is a spacer between X₁ and U, wherein in particular T is selected from substituted or unsubstituted -C₁-C₁₂-alkyl-, in particular C₁-C₆-alkyl, in particular C₁-C₃-alkyl, -R⁵-C(=O)-NH-R⁶-, -R⁵-C(=O)-O-R⁶-, -R⁵-C(=O)-O-, -C(=O)-O-R⁶-, -C(=O)-NH-R⁶-, -C(=O), -C(=O)-O-, -R⁵-phenyl-R⁶-, -R⁵-phenyl-, -phenyl-R⁶-, -phenyl-,
- wherein R⁵ and R⁶ are independently from each other selected substituted or unsubstituted C₁-C₁₂-alkyls, in particular C₁-C₆ alkyls, particularly C₁-C₃ alkyls, and wherein
- U is the cleavage activating part of the functional linker compound, wherein the activating part is formed to stabilize an anion formed during an basic cleavage from X₂,
X₂ is of the form -Y-Z, wherein
- Y is selected from -O-C(=O)- or -S(=O)₂-, and
- Z is an electron-withdrawing leaving group.

2. Compound according to claim 1, wherein
- B is selected from Boc (-C=OOtBu), trityl (-C(Ph)₃), Mmt (-C(Ph)₂C₆H₄OMe), DMT (-C(Ph)(C₆H₄OMe)2), Cbz (-C=OOCH₂Ph), benzylideneamine (=CPh), phtalimides (=(CO)₂C₆H₄), p-toluenesulfonamides (-SO₂C₆H₄Me). benzylamine (-CH₂Ph), acetamides (-COMe), trifluoroacetamide (-COCF₃), Dde (1-(4,4-dimethyl-2,6-dioxocyclohex-1-ylidene)-3-ethyl) and 1-(4,4-dimethyl-2,6-dioxocyclohex-1-ylidene)-3-methylbutyl (ivDde), wherein particularly B is Boc, and/or the
- acetal- or ketal protecting groups are selected from 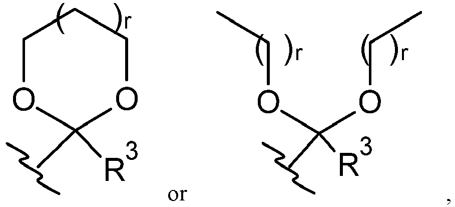 wherein r is 0 to 12, in particular 0, 1 or 2.

3. Compound according to claim 1 or 2, wherein T is selected from -C₁-C₁₂-alkyl-, -R⁵-C(=O)-NH-R⁶-, -R⁵-C(=O)-O-R⁶-, -R⁵-C(=O)-O-, -C(=O)-O-R⁶-, -C(=O)-NH-R⁶-, -C(=O)-, -C(=O)-O-, wherein R⁵ and R⁶ are independently from each other selected C₁-C₆-alkyls, in particular C₁-C₂-alkyls, in particular T is selected from -CH₂-, -CH₂-C(=O)-NH-(CH₂)₂-, -(CH₂)-C(=O)-O-(CH₂)₂-, -CH₂-C(=O)-O-, -C(=O)-O-, -C(=O)-O-(CH₂)₂-, -C(=O)-, in particular -CH₂-, -CH₂-C(=O)-NH-(CH₂)₂-, -C(=O)-O-(CH₂)₂-, -CH₂-C(=O)-O-, -C(=O)-.

4. Compound according to at least one of claims 1 to 3, wherein U of the moiety T-U-Y is selected from the moieties according to the formulas (5), (6), (7), (8), (9), (10) and (11), in particular from (5), (6), (8), (9) and (10), 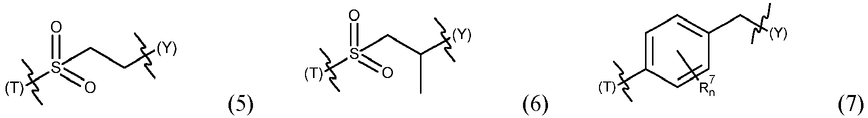 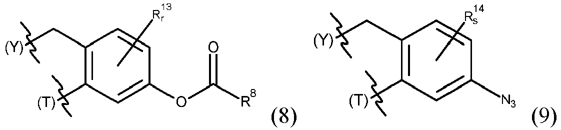 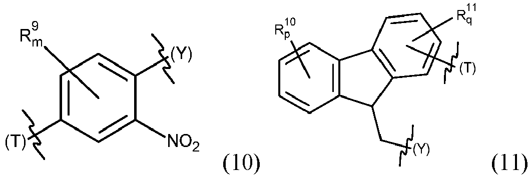
- wherein R⁸ is selected from C₁-C₆-alkyl, CF₃, CH₂CF₃, 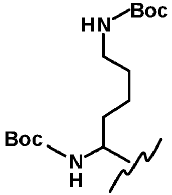 (Boc-Lys(Boc)-) or 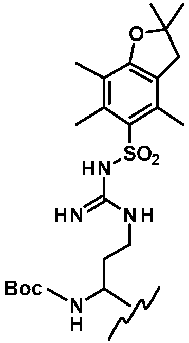 (Boc-Arg(pbf)), in particular from Boc-Lys(Boc)-,
- wherein R⁷ₙ, R⁹ₘ, R¹⁰ₚ,R¹¹_{q}, R¹³ᵣ and R¹⁴ₛ is selected from C₁-C₆-alkyl or -I and/or -M-effects generating substituents, in particular C₁-C₃-alkyls, -F, -Cl, -Br, -I, -CN -NO₂,-N₃, -CF₃, -SO₃H, -CO₂H,
- wherein n equals 0, 1, 2, 3 or 4, in particular n is 0 or 1, in particular 0,
- wherein m equals 0, 1, 2 or 3, in particular m is 0 or 1, in particular 0,
- wherein p equals 0, 1, 2, 3 or 4, in particular p is 0 or 1, in particular 0,
- wherein q equals 0, 1, 2 or 3, in particular q is 0 or 1, in particular 0,
- wherein r equals 0, 1, 2 or 3, in particular r is 0 or 1, in particular 0,
- wherein s equals 0, 1, 2 or 3, in particular s is 0 or 1, in particular 0.

5. Compound according to at least one of claims 1 to 4, wherein Z is selected from the group -F, -Cl, -Br, -I, -N₃, -SR¹², -OCF₃, -OCH₂CF₃, -OSO₂CF₃, -SO₂C₆H₄CH₃, -SO₂CF₃, -SO₂CH₃ 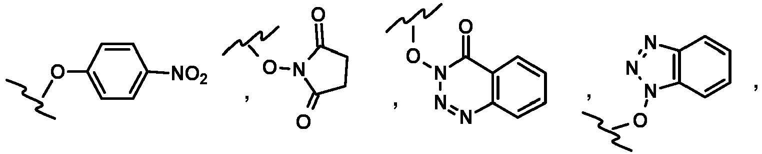 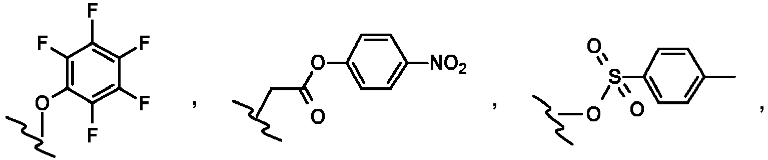 in particular -Cl 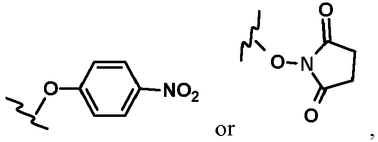 in particular 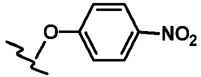 or 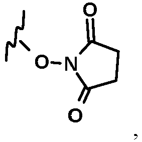 wherein R¹² is a C₁-C₆-alkyl-, an aryl- or a benzyl residue.

6. Compound according to at least one of claims 1 to 5, wherein X₁ is a moiety of formula (2) or (3), in particular of formula (3), wherein R³ is H, R¹ and R² comprise a Boc protecting group or R¹ is H and R² is a Boc protecting group, wherein in particular R¹ is H and R² is a Boc protecting group.

7. Compound according to at least one of claims 1 to 6, wherein Y is of the form -O-C(=O)-.

8. Compound according to at least one of claims 1 to 7,
- wherein T is of the form -(CH₂)-C(=O)-NH-(CH₂)₂-, -(CH₂)-C(=O)-O-(CH₂)₂-, -C(=O)-O-(CH₂)₂-, in particular -C(=O)-O-(CH₂)₂- and -(CH₂)-C(=O)-NH-(CH₂)₂-, in particular -(CH₂)-C(=O)-NH-(CH₂)₂-,
- wherein U is a moiety of formula (5) or (6), in particular of formula (6),

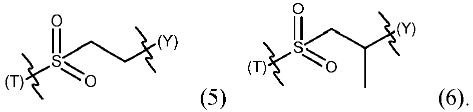

9. Compound according to at least one of claims 1 to 7,
- wherein T is of the form -CH₂-C(=O)-O-, -C(=O)-O-, in particular -CH₂-C(=O)-O-,
- wherein U is a moiety of formula (7), 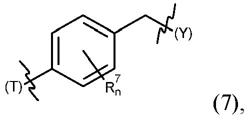 wherein R⁷ is selected from C₁-C₆-alkyl or -I and/or -M-effect generating substituents, in particular C₁-C₃-alkyl, -F, -Cl,-Br, -I, -CN -NO₂, -N₃, -CF₃, -SO₃H, -CO₂H wherein n equals 0, 1, 2, 3 or 4, in particular 0 or 1, in particular 0 and/or
- wherein T is of the form -CH₂-,
- wherein U is a moiety of formula (8), 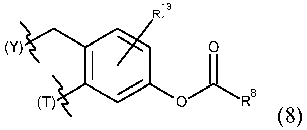
- wherein R⁸ is Boc-Lys(Boc) and r equals 0 and/or
- wherein T is of the form -CH₂-, -(C=O)-, in particular -CH₂-,
- wherein U is a moiety of formula (9), wherein s equals 0, 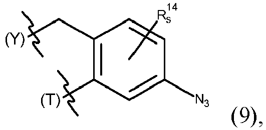 and/or
- wherein T is of the form -C(=O)-,
- wherein U is a moiety of formula (10), wherein m equals 0, 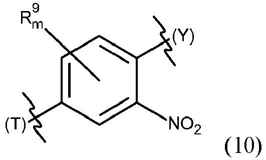
- wherein Y is of the form -SO₂-, and
- wherein Z is Cl.

10. Use of a compound according to at least one of claims 1 to 9 as a connection between the N-terminal amino group of a full-length peptide and a solid phase.

11. A compound of formula X₁-L-Y-PEP (12), wherein X₁, L and Y is defined according to the independent claim 1 and the dependent claims of claim 1, and wherein PEP comprises a full-length peptide bound to Y via its N-terminus.

12. A compound of formula D-X₁'-L-Y-PEP (13),
- wherein D is a surface-modified solid support, which is **characterized in that** the surface is modified by synthetic or natural polymers, in particular modified polysaccharides, in particular aldehyde- or hydrazine-modified sepharose/agarose or cellulose,
- wherein X₁' is of the form -NH-O-, -NH-NH- or -C(=O)-,
- wherein L, Y and PEP are defined according to claim 11.

13. Method for the purification of peptides, in particular of peptides prepared by solid phase peptide synthesis (SPPS), comprising the following steps:
i. contacting a composition of a full-length peptide to be purified and at least one impurity, in particular at least one acetylated truncated sequence, with a capture compound according to any one of claims 1 to 9, and subsequent reaction to a compound of formula (12),
ii. cleavage of the acid labile protecting groups by addition of an acid,
iii. contacting the composition of ii. with a surface-modified solid support, wherein a covalent hydrazone or oxime bond is formed between the capture compound and the solid support, and a compound of formula (13) is provided,
iv. cleavage of the full-length peptide from the solid support.

14. Method according to claim 13, wherein the solid support comprises on its surface the functional groups aldehyde, in particular -O-CH₂-CHO, ketone, hydroxylamine, in particular -ONH₂, and hydrazine, in particular -N₂H₃.

15. Method according to claim 13, wherein after or during cleavage of the full-length peptide from the solid support, the solid support D is cleaved from the residue X¹-L of the capture compound and the solid support is regenerated.

## Revendications

1. Composé de la formule
X₁-L-X₂ (1),
dans laquelle
X₁ est sélectionné parmi 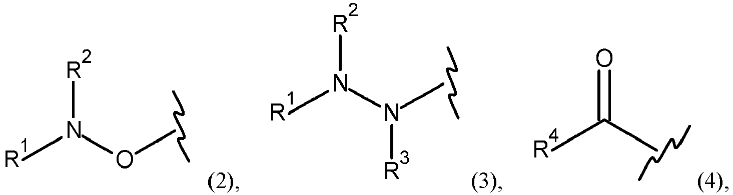
- dans lesquelles chaque R¹ et R² est sélectionné indépendamment l'un de l'autre parmi H ou B, dans lesquelles au moins R¹ ou R² est B, dans lesquelles
- R³ est sélectionné parmi H ou B,
- dans laquelle B est un groupe protecteur d'amine labile aux acides, dans lesquelles
- R⁴ est sélectionné parmi H, alkyle en C₁-C₁₂ ou aryle, dans lesquelles le groupe aldéhyde ou cétone peut être présent de manière protégée par un groupe protecteur labile aux acides,
L est sélectionné parmi des composés coupleurs fonctionnels qui peuvent être séparés de X₂ par voie nucléophile dans des conditions basiques, notamment L présente la forme -T-U-, dans laquelle
- T est un écarteur entre X₁ et U, dans laquelle T est notamment sélectionné parmi -C₁-C₁₂-alkyle-substitué ou non substitué, notamment alkyle en C₁-C₆, notamment alkyle en C₁-C₃, -R⁵-C(=O)-NH-R⁶-, -R⁵-C(=O)-O-R⁶-, -R⁵-C(=O)-O-, -C(=O)-O-R⁶-, -C(=O)-NH-R⁶-, -C(=O), -C(=O)-O-, -R⁵-phényle-R⁶-, -R⁵-phényle-, -phényle-R⁶-, -phényle-,
- dans laquelle R⁵ et R⁶ sont indépendamment l'un de l'autre des alkyles en C₁-C₁₂ substitués ou non substitués, notamment alkyles en C₁-C₆, particulièrement alkyles en C₁-C₃, et dans laquelle
- U est la partie activant la séparation du composé coupleur fonctionnel, dans laquelle la partie d'activation est réalisée pour stabiliser un anion apparaissant lors d'une séparation basique de X₂,
X₂ présente la forme -Y-Z, dans laquelle
- Y est sélectionné parmi -O-C(=O)- ou -S(=O)₂-, et
- Z est un groupe de départ attirant des électrons.

2. Composé selon la revendication 1, dans lequel
- B est sélectionné parmi Boc (-C=OOtBu), trityle (-C(Ph)₃), Mmt (-C(Ph)₂C₆H₄OMe), DMT (-C(Ph)(C₆H₄OMe)₂), Cbz (-C=OOCH₂Ph), benzylidèneamine (=CPh), phtalimide (=(CO)₂C₆H₄), p-toluènesulfonamide (-SO₂C₆H₄Me). benzylamine (-CH₂Ph), acétamide (-COMe), trifluoracétamide (-COCF₃), Dde (1-(4,4-diméthyl-2,6-dioxocyclohex-1-ylidène)-3-éthyle) et 1-(4,4-diméthyl-2,6-dioxocyclohex-1-ylidène)-3-méthylbutyle (ivDde), dans lequel B est notamment Boc, et/ou les
- groupes protecteurs d'acétal ou cétal sont sélectionnés parmi 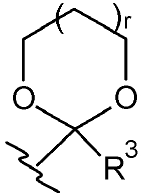 ou 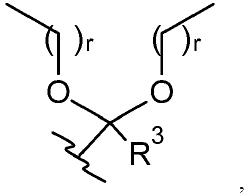 dans lesquelles r est 0 à 12, notamment 0, 1 ou 2.

3. Composé selon la revendication 1 ou 2, dans lequel T est sélectionné parmi -C₁-C₁₂-alkyle-, -R⁵-C(=O)-NH-R⁶-, -R⁵-C(=O)-O-R⁶-, -R⁵-C(=O)-O-, -C(=O)-O-R⁶-, -C(=O)-NH-R⁶-, -C(=O)-,-C(=O)-O-, dans lesquelles R⁵ et R⁶ sont indépendamment l'un de l'autre des alkyles en C₁-C₆ sélectionnés, notamment des alkyles en C₁-C₂, notamment T est sélectionné parmi -CH₂-, -CH₂-C(=O)-NH-(CH₂)₂-, -(CH₂)-C(=O)-O-(CH₂)₂-, -CH₂-C(=O)-O-, -C(=O)-O-, -C(=O)-O-(CH₂)₂-,-C(=O)-, notamment -CH₂-, -CH₂-C(=O)-NH-(CH₂)₂-, -C(=O)-O-(CH₂)₂-, -CH₂-C(=O)-O-, -C(=O)-.

4. Composé selon au moins une des revendications 1 à 3, dans lequel U du composé T-U-Y est sélectionné parmi les composés selon les formules (5), (6), (7), (8), (9), (10) et (11), notamment parmi (5), (6), (8), (9) et (10), 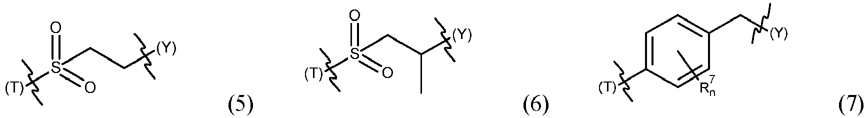 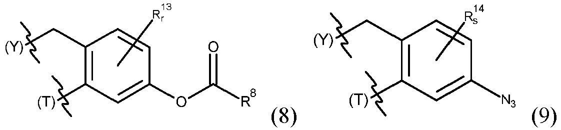 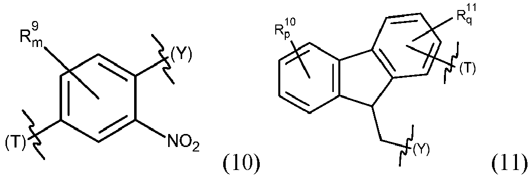
- dans lesquelles R⁸ est sélectionné parmi alkyle en C₁-C₆, CF₃, CH₂CF₃, 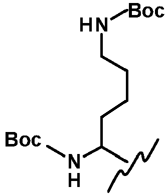 (Boc-Lys(Boc)-) ou 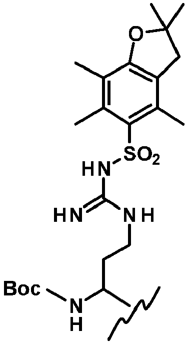 (Boc-Arg(pbf)), notamment parmi Boc-Lys(Boc)-,
- dans lesquelles R⁷ₙ, R⁹ₘ, R¹⁰ₚ, R¹¹_{q}, R¹³ᵣ et R¹⁴ₛ sont sélectionnés parmi alkyle en C₁-C₆ ou des substituants générant des effets -I et/ou -M, notamment alkyles en C₁-C₃, -F, -Cl, -Br, -I, -CN -NO₂, -N₃, -CF₃, -SO₃H, -CO₂H,
- dans lesquelles n est égal à 0, 1, 2, 3 ou 4, notamment n est 0 ou 1, notamment 0
- dans lesquelles m est égal à 0, 1, 2 ou 3, notamment m est 0 ou 1, notamment 0
- dans lesquelles p est égal à 0, 1, 2, 3 ou 4, notamment p est 0 ou 1, notamment 0
- dans lesquelles q est égal à 0, 1, 2 ou 3, notamment q est 0 ou 1, notamment 0
- dans lesquelles r est égal à 0, 1, 2 ou 3, notamment r est 0 ou 1, notamment 0
- dans lesquelles s est égal à 0, 1, 2 ou 3, notamment s est 0 ou 1, notamment 0.

5. Composé selon au moins une des revendications 1 à 4, dans lequel Z est sélectionné parmi le groupe -F, -Cl, -Br, -I, -N₃, -SR¹², -OCF₃, -OCH₂CF₃, -OSO₂CF₃, -SO₂C₆H₄CH₃, -SO₂CF₃, -SO₂CH₃ 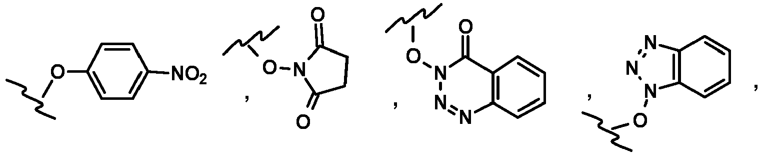 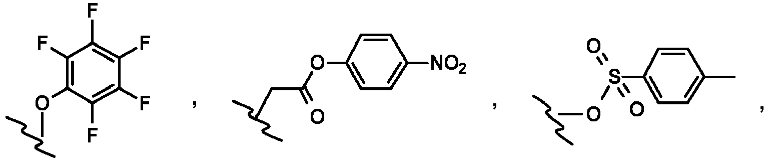 notamment -Cl, 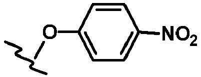 ou 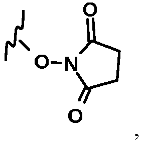 notamment 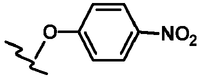 ou 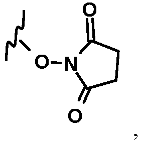 dans lesquelles R¹² est un alkyle en C₁-C₆, un radical aryle ou benzyle.

6. Composé selon au moins une des revendications 1 à 5, dans lequel X₁ est un composé selon la formule (2) ou (3), notamment formule (3), dans laquelle R³ est H, R¹ et R² présentent un groupe protecteur Boc ou R¹ est H et R² est un groupe protecteur Boc, dans laquelle notamment R¹ est H et R² est un groupe protecteur Boc.

7. Composé selon au moins une des revendications 1 à 6, dans lequel Y présente la forme -O-C(=O)-.

8. Composé selon au moins une des revendications 1 à 7,
- dans lequel T présente la forme -(CH₂)-C(=O)-NH-(CH₂)₂-, -(CH₂)-C(=O)-O-(CH₂)₂-,-C(=O)-O-(CH₂)₂-, notamment -C(=O)-O-(CH₂)₂- et -(CH₂)-C(=O)-NH-(CH₂)₂-, notamment-(CH₂)-C(=O)-NH-(CH₂)₂-,
- dans lequel U est un composé selon la formule (5) ou (6), notamment selon la formule (6),

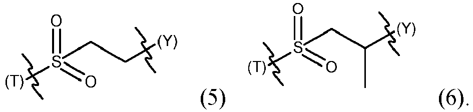

9. Composé selon au moins une des revendications 1 à 7,
- dans lequel T présente la forme -CH₂-C(=O)-O-, -C(=O)-O-, notamment -CH₂-C(=O)-O-,
- dans lequel U est un composé selon la formule (7), 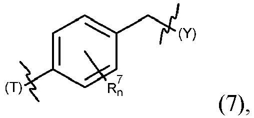 dans laquelle R⁷ est sélectionné parmi alkyle en C₁-C₆ ou des substituants générant un effet -I et/ou -M, notamment alkyle en C₁-C₃, -F, -Cl, -Br, -I, -CN -NO₂, -N₃, -CF₃, -SO₃H, -CO₂H, dans laquelle n est égal à 0, 1, 2, 3 ou 4, notamment 0 ou 1, notamment 0, et/ou
- dans lequel T présente la forme -CH₂-,
- dans lequel U est un composé selon la formule (8), 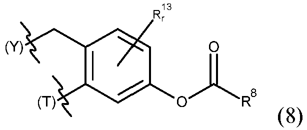
- dans laquelle R⁸ est Boc-Lys(Boc) et r est égal à 0, et/ou
- dans lequel T présente la forme -CH₂-, -(C=O)-, notamment -CH₂-,
- dans lequel U est un composé selon la formule (9), dans laquelle s est égal à 0, 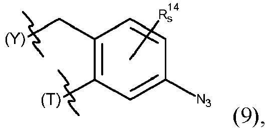 et/ou
- dans lequel T présente la forme -C(=O)-,
- dans lequel U est un composé selon la formule (10), dans laquelle m est égal à 0, 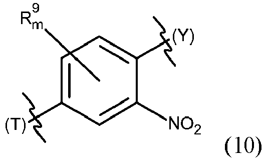
- dans lequel Y présente la forme -SO₂-, et
- dans lequel Z est Cl.

10. Utilisation d'un composé selon au moins une des revendications 1 à 9, en tant que composé entre le groupe amino N-terminal d'un peptide de longueur entière et une phase solide.

11. Composé de la formule X₁-L-Y-PEP (12), dans laquelle X₁, L et Y sont définis selon la revendication indépendante 1 et les revendications dépendantes de la revendication 1, et dans laquelle PEP comprend un peptide de longueur entière qui est relié à Y par le biais de sa terminaison N.

12. Composé de la formule D-X₁'-L-Y-PEP (13),
- dans laquelle D est un support solide à surface modifiée qui est **caractérisé en ce que** la surface est modifiée par des polymères synthétiques ou naturels, notamment des polysaccharides modifiés, notamment de la sépharose/agarose ou cellulose modifiée par un aldéhyde ou une hydrazine,
- dans laquelle X₁' présente la forme -NH-O-, -NH-NH- ou -C(=O)-,
- dans laquelle L, Y et PEP sont définis selon la revendication 11.

13. Procédé de purification de peptides, notamment de peptides fabriqués au moyen de la synthèse peptidique en phase solide (SPPS), qui comprend les étapes suivantes :
i. mise en contact d'une composition d'un peptide de longueur entière à purifier et d'au moins une impureté, notamment d'au moins une séquence tronquée acétylée, avec un composé capteur selon une des revendications 1 à 9, et réaction subséquente à un composé de la formule (12),
ii. séparation des groupes protecteurs labiles aux acides par addition d'un acide,
iii. mise en contact de la composition de ii. avec un support solide à surface modifiée, dans lequel une liaison hydrazone ou oxime covalente est réalisée entre le composé capteur et le support solide, et un composé de la formule (13) est mis à disposition,
iv. séparation du peptide de longueur entière du support solide.

14. Procédé selon la revendication 13, dans lequel le support solide présente à sa surface les groupes fonctionnels aldéhyde, notamment -O-CH₂-CHO, cétone, hydroxylamine, notamment -ONH₂, et hydrazine, notamment -N₂H₃.

15. Procédé selon la revendication 13, dans lequel le support solide D est séparé du radical X¹-L du composé capteur après ou lors de la séparation du peptide de longueur entière du support solide et le support solide est régénéré.
